(19)

Europäisches Patentamt
European Patent Office
Office européen des brevets

(11)    **EP 3 091 079 A1**

(12)    **EUROPEAN PATENT APPLICATION**

(43) Date of publication:
09.11.2016   Bulletin 2016/45

(51) Int Cl.:
*C12N 15/82* (2006.01)     *C07K 14/415* (2006.01)

(21) Application number: **16168936.9**

(22) Date of filing: **27.02.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.02.2011   US 201161447151 P**
**28.02.2011   EP 11156182**
**25.03.2011   US 201161467423 P**
**25.03.2011   EP 11159809**
**06.05.2011   US 201161483115 P**
**06.05.2011   EP 11165187**

(62) Document number(s) of the earlier application(s) in accordance with Art. 76 EPC:
**12752254.8 / 2 681 325**

(71) Applicant: **BASF Plant Science Company GmbH**
**67056 Ludwigshafen (DE)**

(72) Inventors:
• **Hatzfeld, Yves**
**59000 Lille (FR)**
• **Mironov, Vladimir**
**7052 Trondheim (NO)**

(74) Representative: **BASF IP Association**
**BASF SE**
**ZRX-C6**
**67056 Ludwigshafen (DE)**

Remarks:
•The complete document including Reference Tables and the Sequence Listing can be downloaded from the EPO website
•This application was filed on 10.5.2016 as a divisional application to the application mentioned under INID code 62.

(54)    **PLANTS HAVING ENHANCED YIELD-RELATED TRAITS AND PRODUCING METHODS THEREOF**

(57)    The present invention relates generally to the field of molecular biology and concerns a method for enhancing various economically important yield-related traits in plants. The present invention concerns a method for increasing yield in plants by introducing and expressing in said plant a nucleic acid molecule encoding a CDKB-RKA polypeptide having reduced, and preferably lacking catalytic kinase activity. This alternative invention also concerns plants showing increased yield relative to control plants, and comprising a CDKB-RKA polypeptide. This alternative invention also provides constructs comprising a B-type CDK polypeptide having reduced, and preferably lacking catalytic kinase activity, that are useful in performing the methods of the invention.

FIGURE 3

EP 3 091 079 A1

**Description**

**Background**

The present invention relates generally to the field of molecular biology and concerns a method for enhancing various economically important yield-related traits in plants. More specifically, the present invention concerns a method for enhancing yield-related traits in plants by modulating expression in a plant of a nucleic acid encoding a CDKB-RKA polypeptide wherein said CDKB-RKA is a modified CDKB polypeptide which CDKB-RKA polypeptide has modified, preferably reduced, more preferably lacks catalytic kinase activity in comparison to a full length or unmodified CDKB polypeptide. More in particularly, the present invention concerns a method for enhancing yield-related traits in plants by introducing and expressing in said plant of a nucleic acid encoding a CDKB-RKA polypeptide as defined herein. The present invention also concerns plants having modulated expression of a nucleic acid encoding said CDKB-RKA polypeptide, which plants have enhanced yield-related traits relative to control plants. The invention also provides constructs comprising the CDKB-RKA polypeptide as defined herein that are useful in performing the methods of the invention.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is a particularly important trait, since the seeds of many plants are important for human and animal nutrition. Crops such as corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Another important trait for many crops is early vigour. Improving early vigour is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. The ability to engineer early vigour into plants would be of great importance in agriculture. For example, poor early vigour has been a limitation to the introduction of maize (Zea mays L.) hybrids based on Corn Belt germplasm in the European Atlantic.

A further important trait is that of improved abiotic stress tolerance. Abiotic stress is a primary cause of crop loss worldwide, reducing average yields for most major crop plants by more than 50% (Wang et al., Planta 218, 1-14, 2003). Abiotic stresses may be caused by drought, salinity, extremes of temperature, chemical toxicity and oxidative stress. The ability to improve plant tolerance to abiotic stress would be of great economic advantage to farmers worldwide and would allow for the cultivation of crops during adverse conditions and in territories where cultivation of crops may not otherwise be possible.

Crop yield may therefore be increased by optimising one of the above-mentioned factors.

The inherent growth mechanisms of a plant reside in a highly ordered sequence of events collectively known as the 'cell cycle'. Progression through the cell cycle is fundamental to the growth and development of all multicellular organisms and is crucial to cell proliferation. The major components of the cell cycle are highly conserved in yeast, mammals, and plants. The cell cycle is typically divided into the following sequential phases: G0 - G1 - S - G2 - M. DNA replication or synthesis generally takes place during the S phase ("S" is for DNA synthesis) and mitotic segregation of the chromosomes occurs during the M phase (the "M" is for mitosis), with intervening gap phases, G1 (during which cells grow before DNA replication) and G2 (a period after DNA replication during which the cell prepares for division). Cell division is completed after cytokinesis, the last step of the M phase. Cells that have exited the cell cycle and that have become quiescent are said to be in the G0 phase. Cells in this phase can be stimulated to renter the cell cycle at the G1 phase. The "G" in G1, G2 and G0 stands for "gap". Completion of the cell cycle process allows each daughter cell during cell division to receive a full copy of the parental genome.

Cell division is controlled by two principal cell cycle events, namely initiation of DNA synthesis and initiation of mitosis. Each transition to each of these key events is controlled by a checkpoint represented by specific protein complexes (involved in DNA replication and division). The expression of genes necessary for DNA synthesis at the G1/S boundary is regulated by the E2F family of transcription factors in mammals and plant cells (La Thangue, 1994 (Curr. Opin. Cell Biol. 6 (3), 443 - 450); Muller et al., 2001 (Genes Dev. 15 (3), 267 - 285); De Veylder et al., 2002 (EMBO J. 21 (6), 1360 - 1368)). Entry into the cell cycle is regulated/triggered by an E2F/Rb complex that integrates signals and allows activation of transcription of cell cycle genes. The transition between the different phases of the cell cycle, and therefore progression through the cell cycle, is driven by the formation and activation of different heterodimeric serine/threonine protein kinases, generally referred to as cyclin-dependent kinases (CDKs). A prerequisite for activity of these kinases is the physical association with a specific cyclin, the timing of activation being largely dependent upon cyclin expression. Cyclin binding induces conformational changes in the N-terminal lobe of the associating CDK and contributes to the localisation and substrate specificity of the complex. Monomeric CDKs are activated when they are associated with cyclins and thus have kinase activity. Cyclin protein levels fluctuate in the cell cycle and therefore represent a major factor in determining timing of CDK activation. The periodic activation of these complexes containing cyclins and CDK during cell cycle mediates the temporal regulation of cell-cycle transitions (checkpoints). Other factors regulating CDK activity include CDK inhibitors (CKIs or ICKs, KIPs, CIPs, INKs), CDK activating kinases (CAKs), a CDK phosphatase (Cdc25) and a CDK subunit (CKS) (Mironov et al. 1999 (Plant Cell 11 (4), 509 - 522); Reed 1996 (Progression Cell cycle Research 2, 15 - 27)).

In plants, two major classes of CDKs, known as A-type and B-type CDKs, have been studied to date. The A-type CDKs regulate both the G1-to-S and G2-to-M transitions, whereas the B-type CDKs seem to control the G2-to-M checkpoint only (Hemerly et al., 1995 (EMBO J. 14 (16), 3925 - 3936); Magyar et al., 1997 (Plant Cell 9 (2), 223 - 235); Porceddu et al., 2001 (J. Biol. Chem. 276 (39) 36354 - 36360)). In addition, the presence of C-type CDKs and CDK-activating kinases (CAKs) has been reported (Magyar et al., 1997 (Plant Cell 9 (2), 223 - 235); Umeda et al., 1998 (Proc Natl Acad Sci U S A. 95 (9), 5021 - 5026.; Joubès et al., 2001 (Plant Physiol. 126 (4), 1403 - 1415)), as has the presence of D-type, E-type and F-type CDKs (Vandepoele et al. 2002 (Plant Cell 14 (4), 903 - 916)).

Use of CDKB genes and CDKB polypeptides for increasing yield in plants has been disclosed in the prior art. For instance, US application US20070214517 discloses the use of various genes, including for instance a CDKB gene for increasing yield in plants. In another example, WO2005/024029 describes methods for improving growth characteristics of plants which comprise introducing and expressing in a plant genes encoding A or B-type cyclin-dependent kinases or variants thereof. B-type CDK nucleic acids or variants thereof are defined in WO 2005/024029 as nucleic acid/genes encoding a B-type CDK protein having: (i) a PPTALRE motif with no mismatches or with a mismatch at position 2 and/or 4 from left to right; (ii) a catalytic kinase domain; and (iii) a T-loop activation kinase domain (Magyar et al., 1997 (Plant Cell 9 (2), 223 - 235)).

Depending on the end use, the modification of certain yield traits may be favoured over others. For example for applications such as forage or wood production, or bio-fuel resource, an increase in the vegetative parts of a plant may be desirable, and for applications such as flour, starch or oil production, an increase in seed parameters may be particularly desirable. Even amongst the seed parameters, some may be favoured over others, depending on the application. Various mechanisms may contribute to increasing seed yield, whether that is in the form of increased seed size or increased seed number.

It has now been found that various yield-related traits may be improved in plants by modulating expression in a plant of a nucleic acid encoding a CDKB-RKA polypeptide wherein said CDKB-RKA is a modified CDKB polypeptide which CDKB-RKA polypeptide has modified, preferably reduced, catalytic kinase activity in comparison to a full length or unmodified CDKB polypeptide.

**Detailed description of the invention**

The present invention is directed to the use of nucleic acid sequences encoding CDKB-RKA polypeptides as defined herein, for increasing yield, and preferably for increasing seed yield and/or for increasing biomass such as vegetative biomass in plants relative to control plants. The present invention is based on the use of modified forms of B-type cyclin-dependent kinases -herein also named "CDKB-RKA polypeptides" or "CDKB-RKA"- that have reduced kinase activity and preferably that lack kinase activity as compared to wild-type B-type cyclin-dependent kinases (CDKB) and on the use of genes encoding such CDKB-RKA polypeptides.

Surprisingly, it has now been found that modulating expression in a plant of a nucleic acid encoding a CDKB-RKA as defined herein, preferably by introducing and expressing in said plant of a nucleic acid encoding a CDKB-RKA as defined herein, gives plants having enhanced yield-related traits relative to control plants, in particular increased yield, and more particularly (i) increased vegetative biomass such as increased leaf and/or root biomass, and/or (ii) increased seed yield relative to control plants.

In particular embodiments, a CDKB-RKA as defined herein does not comprise or consist of a full length CDKB polypeptide as described in WO 2005/024029 or as given in Example 1, Table A2, and in particular does not comprise or consist of a full length CDKB polypeptide as represented by SEQ ID NO: 320.

According to a first aspect, the present invention provides a method for enhancing yield-related traits in plants relative to control plants, comprising modulating expression in a plant of a nucleic acid encoding a CDKB-RKA as defined herein. A preferred method for modulating, preferably increasing expression of a nucleic acid encoding a CDKB-RKA polypeptide as defined herein is by introducing and expressing a nucleic acid encoding such polypeptide in a plant. Hence, a method for increasing yield in a plant relative to a control plant is provided herein, wherein said method comprises introducing and expressing in said plant a nucleic acid molecule encoding a CDKB-RKA polypeptide as defined herein.

In another aspect, the present invention provides a method for producing plants having enhanced yield-related traits relative to control plants, wherein said method comprises the steps of modulating expression in said plant of a nucleic acid encoding a CDKB-RKA polypeptide as defined herein and optionally selecting for plants having enhanced yield-related traits. In a preferred embodiment, the present invention provides a method for producing plants having enhanced yield-related traits relative to control plants, wherein said method comprises the steps of introducing and expressing in said plant a nucleic acid molecule encoding a CDKB-RKA polypeptide as defined herein and optionally selecting for plants having enhanced yield-related traits.

Any reference hereinafter to a "protein useful in the methods of the invention" is taken to mean a CDKB-RKA polypeptide as defined herein. Any reference hereinafter to a "nucleic acid useful in the methods of the invention" is taken to mean a nucleic acid capable of encoding a CDKB-RKA as defined herein. It shall further be noted that the terms "CDKB-RKA" and "CDKB-RKA polypeptide" and "CDKB-RKA protein" are used herein as synonyms.

The term "CDKB-RKA" as used herein encompasses modified forms of B-type cyclin-dependent kinases (CDKB), wherein said modified forms have at least reduced kinase activity compared to wild-type CDKB proteins. Preferably said CDKB-RKA lacks kinase activity. CDKB polypeptides are well known in the art and have for instance been described in Van Leene et al., 2010, Mol Syst Biol 6, 397; Francis 2007 New Phytol 174(2):261-78; Doonan & Kitsios 2009 Mol Biotechnol 42(1):14-29. Examples of CDKB polypeptides are also shown in Example 1, Table A2.

More in particular, the term "CDKB-RKA" as used herein encompasses B-type cyclin-dependent kinases (CDKB) that are mutated, which mutants have at least reduced kinase activity compared to wild-type proteins, and preferably lack kinase activity. In an example, term "CDKB-RKA" as used encompasses B-type cyclin-dependent kinases (CDKB) that are mutated in the kinase domain. The term "reduced catalytic kinase activity" as used herein refers to a polypeptide which has reduced catalytic kinase activity as compared to a wild type polypeptide. The term "lacking catalytic kinase activity" as used herein refers to a polypeptide which has no or no detectable catalytic kinase activity as compared to a wild type polypeptide. The terms "catalytic kinase activity" and "kinase activity" are used herein as synonyms. Tools and techniques for detecting or determining catalytic kinase activity are well known in the art. Catalytic kinase activity can for instance be determined by means of a kinase assay, such as the one described in Example 4 of the present specification.

The term "mutated" in the context of the present invention intends to refer to one or more non-silent mutations, i.e. mutations that modify the amino sequence of the polypeptide as compared to a corresponding unmodified polypeptide. Non-limitative examples of such mutations may for instance include: insertions; deletions; amino acid substitutions; frameshift mutations caused by insertion or deletion of a number of nucleotides in a DNA acid sequence that is not evenly divisible by three, point mutations, nonsense mutations resulting for instance in a premature stop codon or a nonsense codon in the transcribed mRNA, and possibly resulting in a truncated protein product; missense mutations or nonsynonymous mutations which is a type of point mutations where a single nucleotide is changed to cause substitution of a different amino acid; etc. Techniques for inducing mutations such as insertions, substitutions, truncations, deletions, etc. are well known in the art, and will therefore not be described in detail herein.

The term "CDKB-RKA" as used herein also encompasses truncated forms of CDKB, wherein the truncations are preferably located in the kinase domain. The term "CDKB-RKA" as used herein also encompasses truncated forms of CDKB polypeptides in which no active kinase domain is present. An example thereof is represented by *Arabidopsis* sequence SEQ ID NO: 318, which is a truncated version of SEQ ID NO: 320. Preferably, a CDKB-RKA polypeptide useful in the methods of the present invention is a truncated form of a CDKB polypeptide with a deletion in the C-terminal half of the polypeptide that reduces, preferably substantially inactivates that kinase activity of the polypeptide. More preferably, a CDKB-RKA polypeptide useful in the methods of the present invention is a truncated form of a CDKB polypeptide with a deletion in the C-terminal half of the polypeptide that comprises or consists of substantially the complete kinase domain of that CDKB polypeptide or a part thereof.

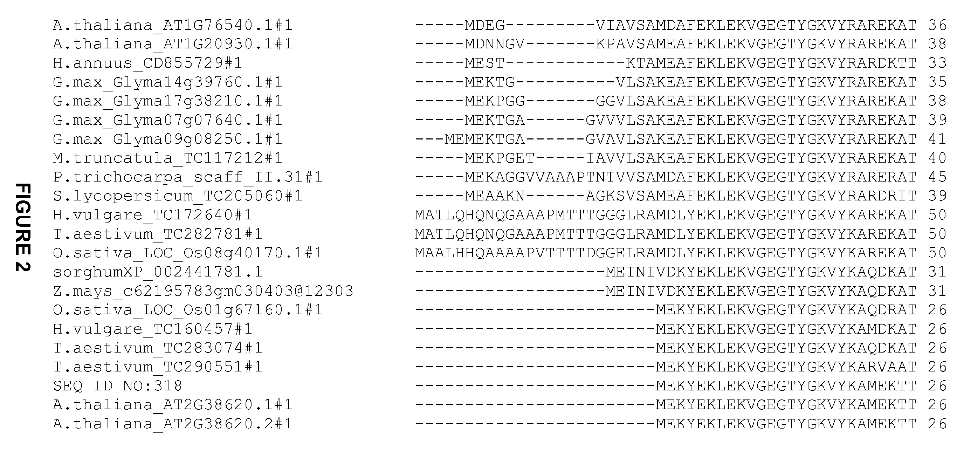

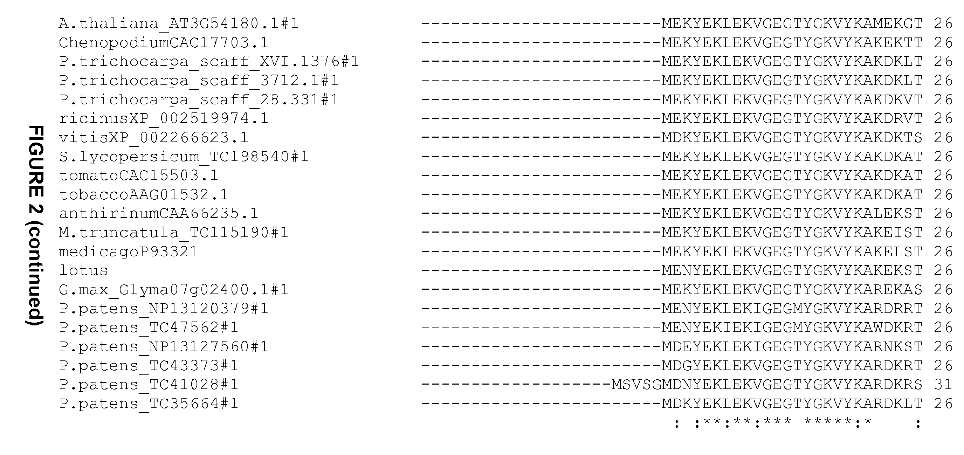

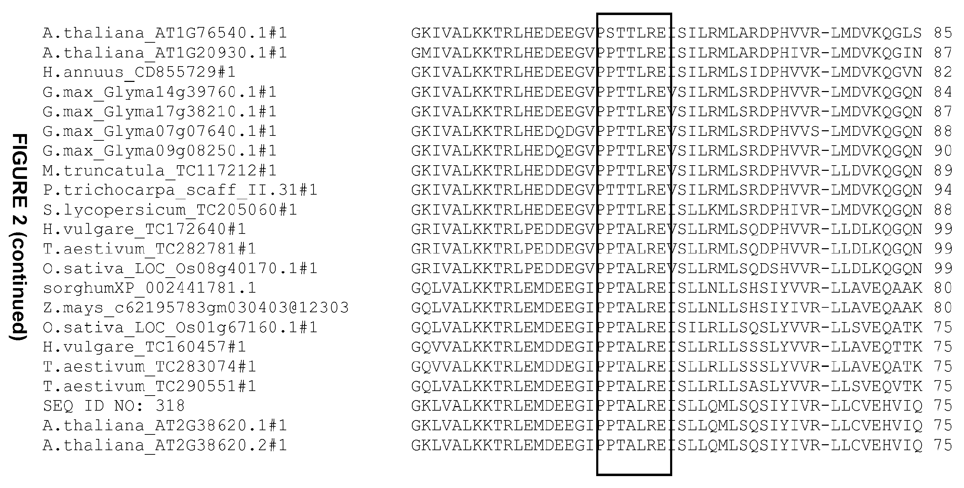

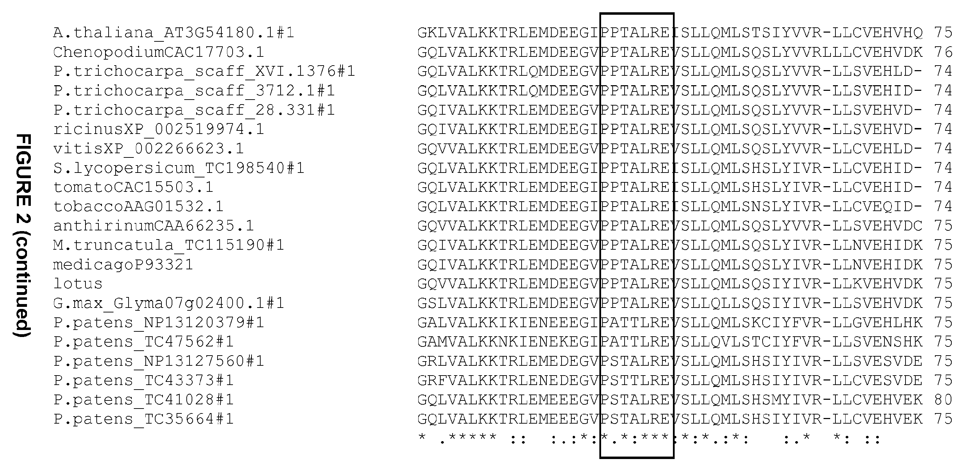

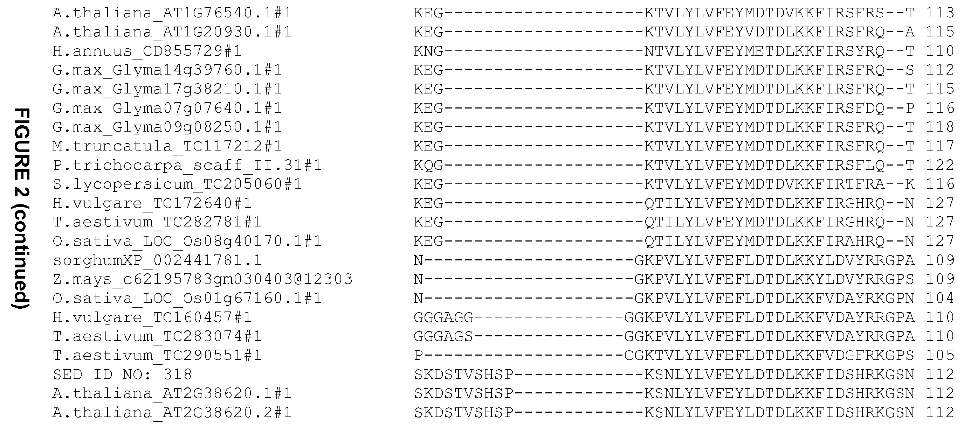

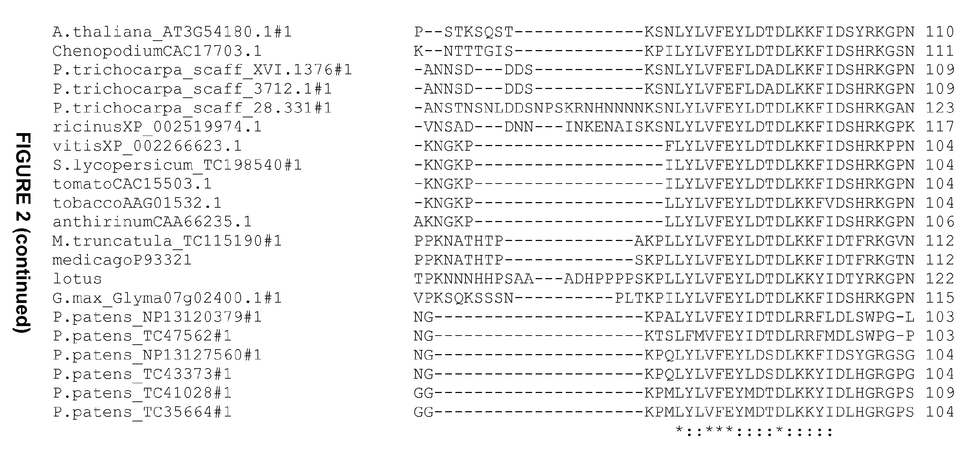

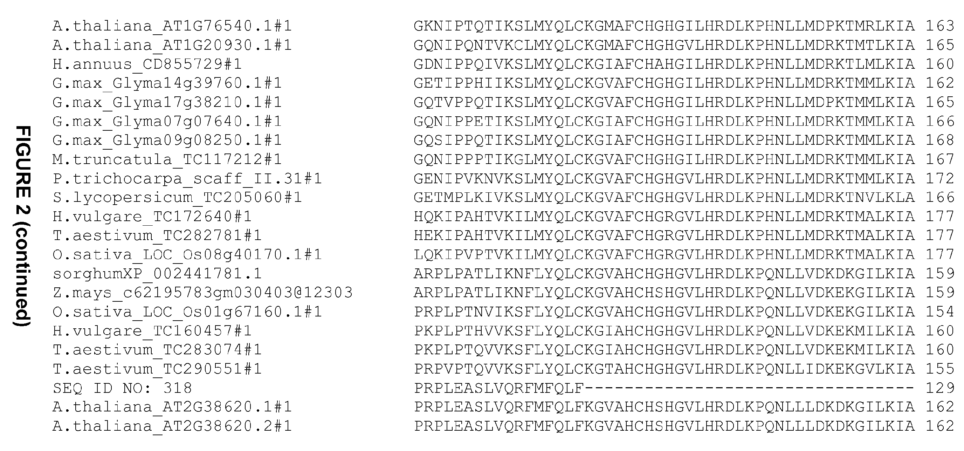

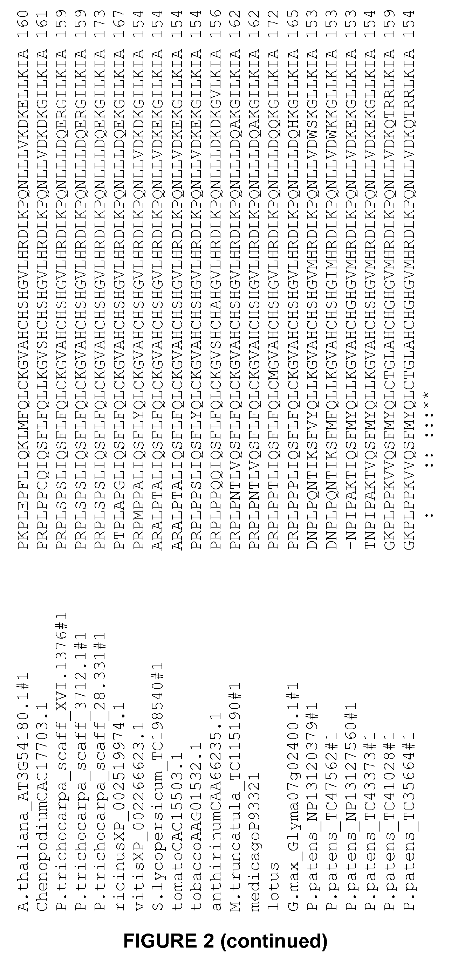

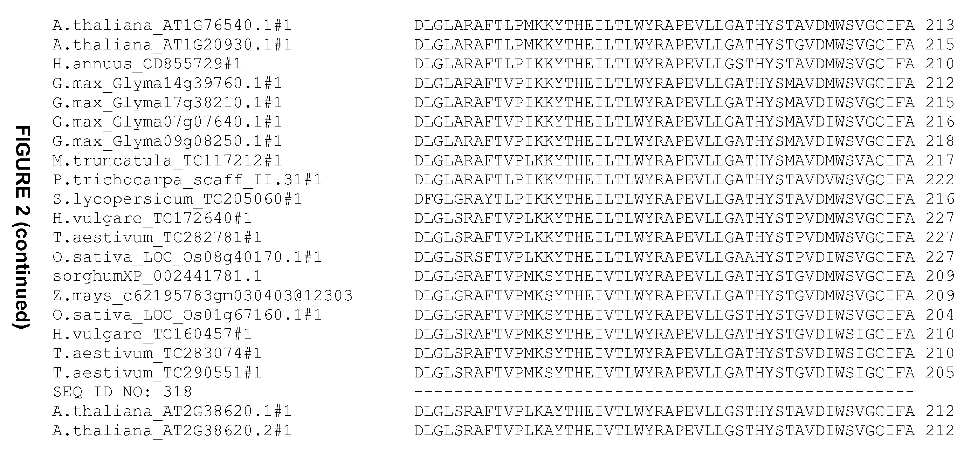

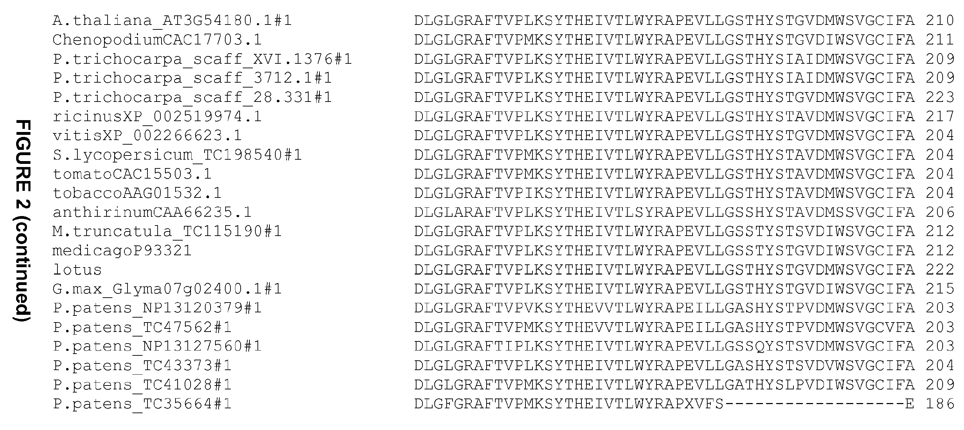

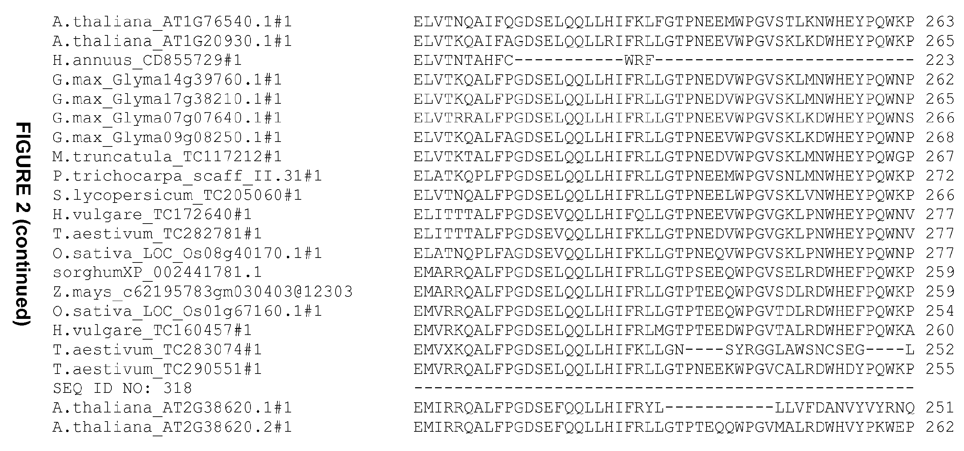

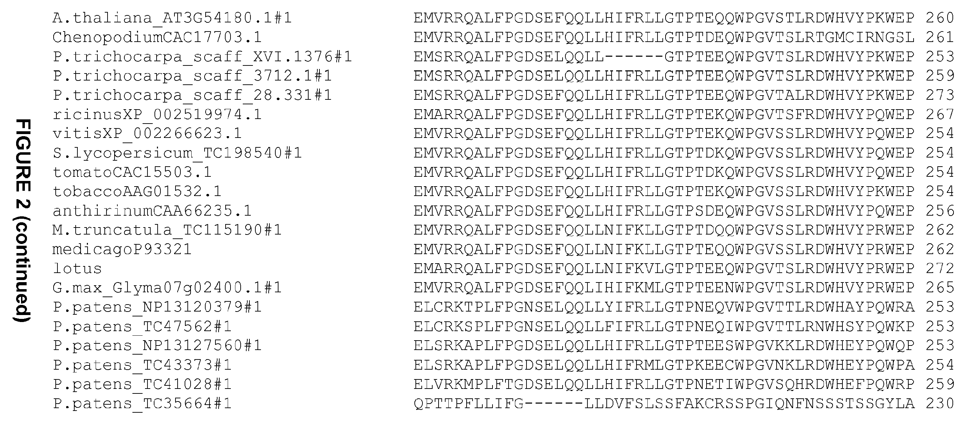

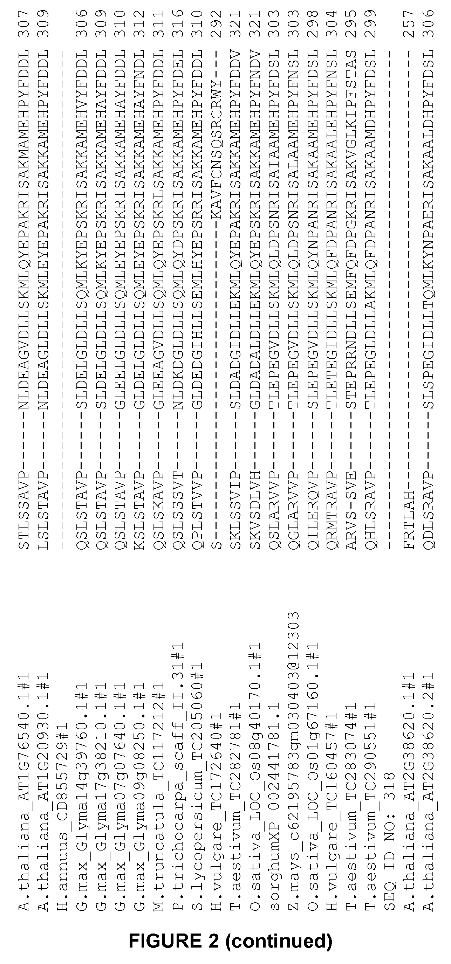

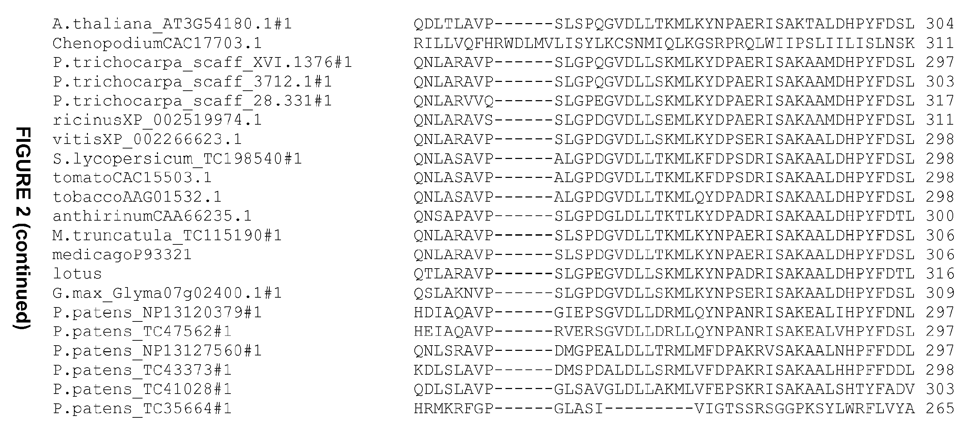

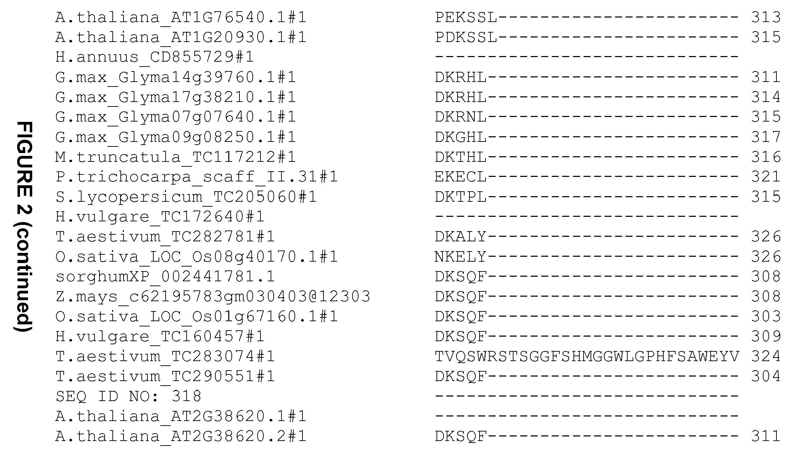

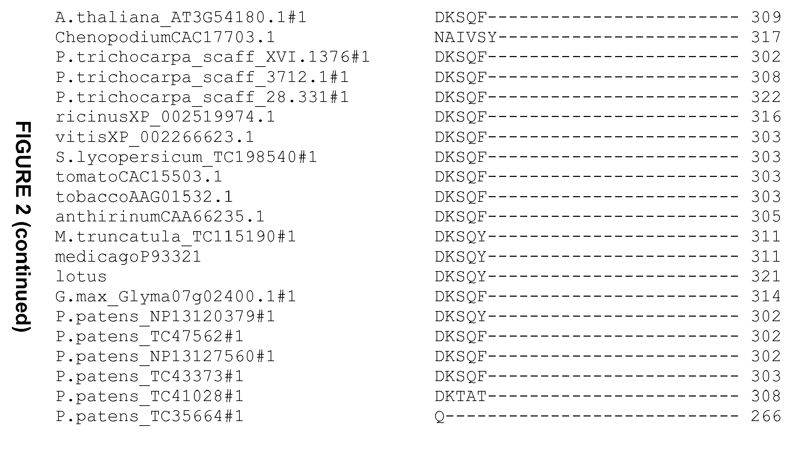

In a preferred embodiment, a CDKB-RKA polypeptide as provided herein comprise a cyclin binding domain represented by a PPTALRE motif with no mismatches or with a mismatch at position 2 and/or 4 from left to right. Preferably a CDKB-RKA polypeptide as provided herein comprise a cyclin binding domain represented by SEQ ID NO: 403, and more preferably represented by SEQ ID NO: 404. In other words, the present invention relates to the use of a nucleic acid sequence encoding a CDKB-RKA polypeptide that is capable of binding cyclins and hence that has cyclin-binding activity. The PPTALRE motifs with no mismatches or with a mismatch at position 2 and/or 4 from left to right, of a representative number of B-type CDK polypeptides can be seen on Figure 2.

Alternatively or additionally, the present invention relates to the use of a nucleic acid sequence encoding a CDKB-RKA polypeptide that

(i) has cyclin-binding activity, and
(ii) has reduced kinase activity and/or lacks kinase activity as defined herein.

In another embodiment, the present invention relates to the use of a nucleic acid sequence encoding a CDKB-RKA polypeptide that

(i) has a cyclin-binding domain represented by a PPTALRE motif with no mismatches or with a mismatch at position 2 and/or 4 from left to right, and preferably represented by SEQ ID NO: 403, and more preferably represented by SEQ ID NO: 404, and
(ii) has a mutated or truncated kinase domain or lacks a kinase domain.

In yet another embodiment, the present invention relates to the use of a nucleic acid sequence encoding a CDKB-RKA polypeptide that

(i) has a cyclin-binding domain represented by a PPTALRE motif with no mismatches or with a mismatch at position 2 and/or 4 from left to right, and preferably represented by SEQ ID NO: 403, and more preferably represented by SEQ ID NO: 404, and
(ii) has a mutated or truncated kinase domain or that lacks a kinase domain, and
(iii) has at least about 30-40%, preferably at least about 41-50%, more preferably at least about 51-60%, 61-70%, 71-80%, 81-85%, 86-90%, 91-95%, 96-99% or more than 99% overall sequence identity to SEQ ID NO: 318.

In another embodiment, a CDKB-RKA polypeptide as provided herein comprises a protein kinase inhibitory domain. In a preferred embodiment, said protein kinase inhibitory domain has a protein kinase inhibitory function, and it is able to be phosphorylated by a kinase, such as by a Wee1 Kinase, to inhibit catalytic kinase activity.

In yet another embodiment, a CDKB-RKA polypeptide as provided herein lacks a T-loop activation kinase domain (as defined by Magyar et al. 1997; plant cell 9(2) 223-235).

Nucleic acids encoding a CDKB-RKA polypeptide as defined herein can be derived from any one of the CDKB nucleic acid sequences as given in Table A2 of the Examples section herein. Nucleic acids derived from nucleic acid sequences as given in Table A2 of the Examples section herein are useful in performing the methods of the invention. The amino acid sequences given in Table A2 of the Examples section are example sequences of orthologues and paralogues of a CDKB polypeptide represented by SEQ ID NO: 320, the terms "orthologues" and "paralogues" being as defined herein. Further orthologues and paralogues may readily be identified by performing a so-called reciprocal blast search as described in the definitions section; where the query sequence is SEQ ID NO: 319 or SEQ ID NO: 320, the second BLAST (back-BLAST) would be against *Arabidopsis* sequences. In addition, in another embodiment, a nucleic acid sequence encoding a CDKB polypeptide as defined herein can also be derived from an orthologue or paralogue of any of the polypeptides given in Table A2. Other examples of nucleic acids encoding CDKB polypeptides from which nucleic acid sequences encoding CDKB-RKA polypeptides as defined herein can be derived are also disclosed for instance in WO 2005/024029 or US20070214517, which are incorporated herein by reference.

In a preferred example, a CDKB-RKA polypeptide useful in the methods of the present invention is a modified form such as a mutated form and/or a truncated form, of any of the CDKB polypeptides as represented in Table A2, and preferably as represented by SEQ ID NO: 320.

In another preferred example, a CDKB-RKA polypeptide useful in the methods of the present invention is a truncated form of any of the CDKB polypeptides as represented in Table A2, and preferably as represented by SEQ ID NO: 320, with a deletion in the C-terminal half of said polypeptide that reduces, preferably substantially inactivates that kinase activity of the polypeptide.

In yet another preferred example a CDKB-RKA polypeptide useful in the methods of the present invention is a truncated form of any of the CDKB polypeptides as represented in Table A2, and preferably as represented by SEQ ID NO: 320, with a deletion that consists of a part of or substantially the complete kinase domain of any of said polypeptides.

For instance, a CDKB-RKA polypeptide useful in the methods of the present invention is a truncated form of the CDKB polypeptide as represented by SEQ ID NO: 320, with a deletion of substantially the complete kinase domain of said polypeptide, for instance as represented by the amino acid coordinates 140 to 152 on SEQ ID NO: 4 (IPR008271), or of only a part thereof, wherein said part may comprise 1, 2, 3, 4, 5, 6, 7, 8, 9, 10, 11, or 12 amino acids.

In a particularly preferred embodiment, a CDKB-RKA polypeptide useful in the methods of the present invention corresponds to an amino acid sequence represented by SEQ ID NO: 318 or a homologous, orthologous or paralogous sequence thereof.

The term "homologue of SEQ ID NO: 318" as defined herein refers to an amino acid sequence that has in increasing order of preference at least 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to SEQ ID NO: 318, and for instance has at least about 30-40%, preferably at least about 41-50%, more preferably at least about 51-60%, 61-70%, 71-80%, 81-85%, 86-90%, 91-95%, 96-99% or more than 99% overall sequence identity to SEQ ID NO: 318, provided that the amino acid sequence has

(i) reduced catalytic kinase activity, and preferably lacks catalytic kinase activity, as defined above, and
(ii) optionally a cyclin binding domain represented by a PPTALRE motif with no mismatches or with a mismatch at position 2 and/or 4 from left to right, and preferably represented by SEQ ID NO: 403, and more preferably represented by SEQ ID NO: 404.

The overall sequence identity can be determined using a global alignment algorithm, such as the Needleman Wunsch algorithm in the program GAP (GCG Wisconsin Package, Accelrys), preferably with default parameters and preferably with sequences of mature proteins (i.e. without taking into account secretion signals or transit peptides). In one embodiment the sequence identity level is determined by comparison of the polypeptide sequences over the entire length of the sequence of SEQ ID NO: 318.

In one embodiment of the present invention the function of the nucleic acid sequences according to the invention is to confer information for synthesis of the CDKB-RKA polypeptides having reduced, and preferably lacking catalytic kinase activity and that increase yield or yield-related traits, when such a nucleic acid sequence of the invention is transcribed and translated in a living plant cell.

Nucleic acids encoding CDKB-RKA polypeptides as defined herein may be derived from any natural or artificial source. The nucleic acid may be modified from its native form in composition and/or genomic environment through deliberate human manipulation. Preferably the CDKB-RKA polypeptide-encoding nucleic acid is from a plant, further preferably from a dicotyledonous plant, more preferably from the family *Brassicaceae,* most preferably the nucleic acid is from *Arabidopsis thaliana.*

In addition, CDKB-RKA polypeptides as defined herein, when expressed in rice according to the methods of the present invention as outlined in Examples 8 and 9, give plants having increased yield related traits, in particular any one or more aboveground biomass, root biomass, and seed yield including total weight of seeds and number of seeds.

The present invention is illustrated by transforming plants with the nucleic acid sequence represented by SEQ ID NO: 317, encoding the polypeptide sequence of SEQ ID NO: 318. However, performance of the invention is not restricted to these sequences; the methods of the invention may advantageously be performed using any CDKB-RKA-encoding nucleic acid or CDKB-RKA polypeptide or variants thereof as defined herein.

Nucleic acid variants may also be useful in practicing the methods of the invention. Examples of such nucleic acid variants include nucleic acids encoding homologues and derivatives of any one of the amino acid sequences given in Table A2 of the Examples section or of SEQ ID NO: 318, the terms "homologue" and "derivative" being as defined herein, provided that said homologues and derivatives have reduced, and preferably lack catalytic kinase activity. Also useful in the methods of the invention are nucleic acids encoding homologues and derivatives of orthologues or paralogues of any one of the amino acid sequences given in Table A2 of the Examples section or of SEQ ID NO: 318, provided that said homologues and derivatives of orthologues or paralogues have reduced, and preferably lack catalytic kinase activity as defined above. Further variants useful in practicing the methods of the invention are variants in which codon usage is optimised or in which miRNA target sites are removed.

Further nucleic acid variants useful in practicing the methods of the invention include portions of nucleic acids encoding CDKB-RKA polypeptides, nucleic acids hybridising to nucleic acids encoding CDKB-RKA polypeptides, splice variants of nucleic acids encoding CDKB-RKA polypeptides, allelic variants of nucleic acids encoding CDKB-RKA polypeptides and variants of nucleic acids encoding CDKB-RKA polypeptides obtained by gene shuffling. The terms hybridising sequence, splice variant, allelic variant and gene shuffling are as described herein. These further nucleic acid variants according to the invention are all characterized in that they encode polypeptides that have reduced, and preferably lack catalytic kinase activity.

According to the present invention, there is provided a method for enhancing yield-related traits in plants, comprising introducing and expressing in a plant a portion of any one of the nucleic acid sequences given in Table A2 of the Examples section or of SEQ ID NO: 317, or a portion of a nucleic acid encoding an orthologue, paralogue or homologue of any of the amino acid sequences given in Table A2 of the Examples section or of SEQ ID NO: 317.

A portion of a nucleic acid may be prepared, for example, by making one or more deletions to the nucleic acid. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

Portions useful in the methods of the invention, encode a CDKB-RKA polypeptide as defined herein. Preferably, the portion is a portion of any one of the nucleic acids given in Table A2 of the Examples section or of SEQ ID NO: 317, or is a portion of a nucleic acid encoding an orthologue or paralogue of any one of the amino acid sequences given in Table A2 of the Examples section or of SEQ ID NO: 318. Preferably the portion is at least 50, 60, 70, 80, 90, 100, 110, 120, 150, 200, 250, 300, 350, 400, 450, 500, 550, 600, 650, 700 consecutive nucleotides in length, the consecutive nucleotides being of any one of the nucleic acid sequences given in Table A2 of the Examples section or of SEQ ID NO: 317, or of a nucleic acid encoding an orthologue or paralogue of any one of the amino acid sequences given in Table A2 of the Examples section or of SEQ ID NO: 317. Preferably, the portion encodes a fragment of an amino acid sequence which has reduced, and preferably lacks kinase activity, and which (i) comprises a cyclin binding domain and/or (ii) has at least about 30-40%, preferably at least about 41-50%, more preferably at least about 51-60%, 61-70%, 71-80%, 81-85%, 86-90%, 91-95%, 96-99% or more than 99% overall sequence identity to SEQ ID NO: 318.

Another nucleic acid variant useful in the methods of the invention is a nucleic acid capable of hybridising, under reduced stringency conditions, preferably under stringent conditions, with a nucleic acid encoding a CDKB-RKA polypeptide as defined herein, or with a portion as defined herein.

According to the present invention, there is provided a method for enhancing yield-related traits in plants, comprising introducing and expressing in a plant a nucleic acid capable of hybridizing to any one of the nucleic acids given in Table A2 of the Examples section or to SEQ ID NO: 317 to a portion as defined herein, or comprising introducing and expressing in a plant a nucleic acid capable of hybridising to a nucleic acid encoding an orthologue, paralogue or homologue of any of the nucleic acid sequences given in Table A2 of the Examples section or to SEQ ID NO: 317 or to a portion as defined herein.

Hybridising sequences useful in the methods of the invention encode a CDKB-RKA polypeptide as defined herein. Preferably, the hybridising sequence is capable of hybridising to the complement of any one of the nucleic acids given in Table A2 of the Examples section, or to SEQ ID NO: 317, or to a portion of any of these sequences, a portion being as defined above, or the hybridising sequence is capable of hybridising to the complement of a nucleic acid encoding an orthologue or paralogue of any one of the amino acid sequences given in Table A2 of the Examples section or of SEQ ID NO: 317.

Most preferably, the hybridising sequence is capable of hybridising to the complement of a nucleic acid as represented by SEQ ID NO: 317 or to a portion thereof. In one embodiment, the hybridization conditions are of medium stringency, preferably of high stringency, as defined above.

Preferably, the hybridising sequence encodes a polypeptide with an amino acid sequence which has reduced, and preferably lacks kinase activity, and which (i) comprises a cyclin binding domain and/or (ii) has at least about 30-40%, preferably at least about 41-50%, more preferably at least about 51-60%, 61-70%, 71-80%, 81-85%, 86-90%, 91-95%, 96-99% or more than 99% overall sequence identity to SEQ ID NO: 318.

Another nucleic acid variant useful in the methods of the invention is a splice variant encoding a CDKB-RKA polypeptide as defined hereinabove, a splice variant being as defined herein.

According to the present invention, there is provided a method for enhancing yield-related traits in plants, comprising introducing and expressing in a plant a splice variant of any one of the nucleic acid sequences given in Table A2 of the Examples section or of SEQ ID NO: 317, or a splice variant of a nucleic acid encoding an orthologue, paralogue or homologue of any of the amino acid sequences given in Table A2 of the Examples section or of SEQ ID NO: 317. Preferred splice variants are splice variants of a nucleic acid represented by SEQ ID NO: 317, or a splice variant of a nucleic acid encoding an orthologue or paralogue of SEQ ID NO: 318. Preferably, the amino acid sequence encoded by the splice variant has reduced, and preferably lacks kinase activity, and (i) comprises a cyclin binding domain and/or (ii) has at least about 30-40%, preferably at least about 41-50%, more preferably at least about 51-60%, 61-70%, 71-80%, 81-85%, 86-90%, 91-95%, 96-99% or more than 99% overall sequence identity to SEQ ID NO: 318.

Another nucleic acid variant useful in performing the methods of the invention is an allelic variant of a nucleic acid encoding a CDKB-RKA polypeptide as defined hereinabove, an allelic variant being as defined herein.

According to the present invention, there is provided a method for enhancing yield-related traits in plants, comprising introducing and expressing in a plant an allelic variant of any one of the nucleic acids given in Table A2 of the Examples section or of SEQ ID NO: 317, or comprising introducing and expressing in a plant an allelic variant of a nucleic acid encoding an orthologue, paralogue or homologue of any of the amino acid sequences given in Table A2 of the Examples section or of SEQ ID NO: 317.

Allelic variants exist in nature, and encompassed within the methods of the present invention is the use of these natural alleles. Preferably, the allelic variant is an allelic variant of SEQ ID NO: 317 or an allelic variant of a nucleic acid encoding an orthologue or paralogue of SEQ ID NO: 318. Preferably, the amino acid sequence encoded by the allelic variant, has reduced, and preferably lacks kinase activity, and (i) comprises a cyclin binding domain and/or (ii) has at least about 30-40%, preferably at least about 41-50%, more preferably at least about 51-60%, 61-70%, 71-80%, 81-85%, 86-90%, 91-95%, 96-99% or more than 99% overall sequence identity to SEQ ID NO: 318.

Gene shuffling or directed evolution may also be used to generate variants of nucleic acids encoding CDKB-RKA polypeptides as defined above; the term "gene shuffling" being as defined herein.

According to the present invention, there is provided a method for enhancing yield-related traits in plants, comprising introducing and expressing in a plant a variant of any one of the nucleic acid sequences given in Table A2 of the Examples section or of SEQ ID NO: 317, or comprising introducing and expressing in a plant a variant of a nucleic acid encoding an orthologue, paralogue or homologue of any of the amino acid sequences given in Table A2 of the Examples section or of SEQ ID NO: 317, which variant nucleic acid is obtained by gene shuffling.

Preferably, the amino acid sequence encoded by the variant nucleic acid obtained by gene shuffling, has reduced, and preferably lacks kinase activity, and (i) comprises a cyclin binding domain and/or (ii) has at least about 30-40%, preferably at least about 41-50%, more preferably at least about 51-60%, 61-70%, 71-80%, 81-85%, 86-90%, 91-95%, 96-99% or more than 99% overall sequence identity to SEQ ID NO: 318.

Furthermore, nucleic acid variants may also be obtained by site-directed mutagenesis. Several methods are available to achieve site-directed mutagenesis, the most common being PCR based methods (Current Protocols in Molecular Biology. Wiley Eds.). CDKB-RKA polypeptides differing from the sequence of SEQ ID NO: 318 by one or several amino acids (substitution(s), insertion(s) and/or deletion(s) as defined above) may equally be useful to increase the yield of plants in the methods and constructs and plants of the invention.

In another embodiment the present invention extends to recombinant chromosomal DNA comprising a nucleic acid sequence useful in the methods of the invention, wherein said nucleic acid is present in the chromosomal DNA as a result of recombinant methods, i.e. said nucleic acid is not in the chromosomal DNA in its natural genetic environment. In a further embodiment the recombinant chromosomal DNA of the invention is comprised in a plant cell.

Performance of the methods of the invention gives plants having enhanced yield-related traits. In particular performance of the methods of the invention gives plants having increased yield, especially increased seed yield relative to control plants. The terms "yield" and "seed yield" are described in more detail in the "definitions" section herein. In another embodiment, performance of the methods of the invention gives plants having increased yield, especially increased vegetative biomass, such as increased leaf and/or root biomass relative to control plants. The terms "vegetative biomass" is described in more detail in the "definitions" section herein.

Reference herein to enhanced yield-related traits is taken to mean an increase in early vigour and/or in biomass (weight) of one or more parts of a plant, which may include (i) aboveground parts and preferably aboveground harvestable parts and/or (ii) parts below ground and preferably harvestable below ground. In particular, such harvestable parts are seeds, and performance of the methods of the invention results in plants having increased seed yield relative to the seed yield of control plants.

The present invention provides a method for increasing yield-related traits, and preferably yield, and more preferably seed yield and biomass, especially increased vegetative biomass, such as increased leaf and/or root biomass, relative to control plants, which method comprises modulating expression in a plant of a nucleic acid encoding a CDKB-RKA polypeptide as defined herein.

According to a preferred feature of the present invention, performance of the methods of the invention gives plants having an increased growth rate relative to control plants. Therefore, according to the present invention, there is provided a method for increasing the growth rate of plants, which method comprises modulating expression in a plant of a nucleic acid encoding a CDKB-RKA polypeptide as defined herein.

Performance of the methods of the invention gives plants grown under non-stress conditions or under mild drought conditions increased yield relative to control plants grown under comparable conditions. Therefore, according to the present invention, there is provided a method for increasing yield in plants grown under non-stress conditions or under mild drought conditions, which method comprises modulating expression in a plant of a nucleic acid encoding a CDKB-RKA polypeptide as defined herein.

Performance of the methods of the invention gives plants grown under conditions of drought, increased yield relative to control plants grown under comparable conditions. Therefore, according to the present invention, there is provided a method for increasing yield in plants grown under conditions of drought which method comprises modulating expression in a plant of a nucleic acid encoding a CDKB-RKA polypeptide as defined herein.

Performance of the methods of the invention gives plants grown under conditions of nutrient deficiency, particularly under conditions of nitrogen deficiency, increased yield relative to control plants grown under comparable conditions. Therefore, according to the present invention, there is provided a method for increasing yield in plants grown under conditions of nutrient deficiency, which method comprises modulating expression in a plant of a nucleic acid encoding a CDKB-RKA polypeptide as defined herein.

Performance of the methods of the invention gives plants grown under conditions of salt stress, increased yield relative to control plants grown under comparable conditions. Therefore, according to the present invention, there is provided a method for increasing yield in plants grown under conditions of salt stress, which method comprises modulating expression in a plant of a nucleic acid encoding a CDKB-RKA polypeptide as defined herein.

The invention also provides genetic constructs and vectors to facilitate introduction and/or expression in plants of nucleic acids encoding CDKB-RKA polypeptides as defined herein. The gene constructs may be inserted into vectors, which may be commercially available, suitable for transforming into plants and suitable for expression of the gene of interest in the transformed cells. The invention also provides use of a gene construct as defined herein in the methods of the invention.

More specifically, the present invention provides a construct comprising:

(a) a nucleic acid encoding a CDKB-RKA polypeptide as defined above;
(b) one or more control sequences capable of driving expression of the nucleic acid sequence of (a); and optionally
(c) a transcription termination sequence.

Preferably, the nucleic acid encoding a CDKB-RKA polypeptide is as defined above. The term "control sequence" and "termination sequence" are as defined herein.

The genetic construct of the invention may be comprised in a host cell, plant cell, seed, agricultural product or plant. The invention furthermore provides plants transformed with a construct as described above. In particular, the invention provides plants transformed with a construct as described above, which plants have increased yield-related traits as described herein.

Plants are transformed with a genetic construct such as a vector or an expression cassette comprising any of the nucleic acids described above. The skilled artisan is well aware of the genetic elements that must be present on the genetic construct in order to successfully transform, select and propagate host cells containing the sequence of interest. The sequence of interest is operably linked to one or more control sequences (at least to a promoter).

In one embodiment the genetic construct of the invention confers increased yield or yield related traits(s) to a living plant cell when it has been introduced into said plant cell and express the nucleic acid encoding the CDKB-RKA polypeptide, comprised in the genetic construct.

It should be clear that the applicability of the present invention is not restricted to the CDKB-RKA polypeptide-encoding nucleic acid represented by SEQ ID NO: 317.

Advantageously, any type of promoter, whether natural or synthetic, may be used to drive expression of the nucleic acid sequence, but preferably the promoter is of plant origin. A constitutive promoter is particularly useful in the methods. Preferably the constitutive promoter is a medium strength promoter. In another embodiment said constitutive promoter is an ubiquitous constitutive promoter of medium strength. See the "Definitions" section herein for definitions of the various promoter types.

Optionally, one or more terminator sequences may be used in the construct introduced into a plant. Preferably, the construct comprises an expression cassette comprising a medium strength constitutive promoter, operably linked to the nucleic acid encoding the CDKB-RKA polypeptide as defined herein. Furthermore, one or more sequences encoding selectable markers may be present on the construct introduced into a plant.

According to a preferred feature of the invention, the modulated expression is increased expression. Methods for increasing expression of nucleic acids or genes, or gene products, are well documented in the art and examples are provided in the definitions section.

As mentioned above, a preferred method for modulating expression of a nucleic acid encoding a CDKB-RKA polypeptide as defined herein is by introducing and expressing in a plant a nucleic acid encoding a CDKB-RKA polypeptide; however the effects of performing the method, i.e. enhancing yield-related traits may also be achieved using other well known techniques, including but not limited to T-DNA activation tagging, TILLING, homologous recombination. A description of these techniques is provided in the definitions section.

The invention also provides a method for the production of transgenic plants having enhanced yield-related traits relative to control plants, comprising introduction and expression in a plant of any nucleic acid encoding a CDKB-RKA polypeptide as defined herein.

More specifically, the present invention provides a method for the production of transgenic plants having enhanced yield-related traits, particularly increased yield, more particularly increased seed yield and/or increased biomass such as increased vegetative biomass, which method comprises:

(i) introducing and expressing in a plant or plant cell a CDKB-RKA polypeptide-encoding nucleic acid as defined herein or a genetic construct comprising a CDKB-RKA polypeptide-encoding nucleic acid as defined herein; and

(ii) cultivating the plant cell under conditions promoting plant growth and development.

Cultivating the plant cell under conditions promoting plant growth and development, may or may not include regeneration and or growth to maturity.

The nucleic acid of (i) may be any of the nucleic acids capable of encoding a CDKB-RKA polypeptide as defined herein.

The nucleic acid may be introduced directly into a plant cell or into the plant itself (including introduction into a tissue, organ or any other part of a plant). According to a preferred feature of the present invention, the nucleic acid is preferably introduced into a plant by transformation. The term "transformation" is described in more detail in the "definitions" section herein.

In one embodiment the present invention extends to any plant cell or plant produced by any of the methods described herein, and to all plant parts and propagules thereof. The present invention encompasses plants or parts thereof (including seeds) obtainable by the methods according to the present invention. The plants or plant parts or plant cells comprise a nucleic acid transgene encoding a CDKB-RKA polypeptide as defined above, preferably in a genetic construct such as an expression cassette. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

In a further embodiment invention extends to seeds comprising the expression cassettes of the invention, the genetic constructs of the invention, the nucleic acids encoding the CDKB-RKA and/or the CDKB-RKA encoded by the nucleic acids as described above.

In a particular embodiment the plant cells of the invention are non-propagative cells, i.e. cells that are not capable to regenerate into a plant using cell culture techniques known in the art. While plants cells generally have the characteristic of totipotency, some plant cells can not be used to regenerate or propagate intact plants from said cells. In one embodiment of the invention the plant cells of the invention are such cells.

In another embodiment the plant cells of the invention are plant cells that do not sustain themselves in an autotrophic way, such plant cells are not deemed to represent a plant variety. In a further embodiment the plant cells of the invention are non-plant variety and non-propagative.

The invention also includes host cells containing an isolated nucleic acid encoding a CDKB-RKA polypeptide as defined hereinabove. In one embodiment host cells according to the invention are plant cells, yeasts, bacteria or fungi. Host plants for the nucleic acids or the vector used in the method according to the invention, the expression cassette or construct or vector are, in principle, advantageously all plants, which are capable of synthesizing the polypeptides used in the inventive method. In a particular embodiment the plant cells of the invention overexpress the nucleic acid molecule of the invention.

The invention also includes methods for the production of a product comprising a) growing the plants of the invention and b) producing said product from or by the plants of the invention or parts, including seeds, of these plants. In a further embodiment the methods comprises the steps of a) growing the plants of the invention, b) removing the harvestable parts as defined above from the plants and c) producing said product from, or with the harvestable parts of the invention.

Advantageously the methods of the invention are more efficient than the known methods, because the plants of the invention have increased yield and/or stress tolerance to an environmental stress compared to a control plant used in comparable methods. In one embodiment the products produced by the methods of the invention are plant products such as, but not limited to, a foodstuff, feedstuff, a food supplement, feed supplement, fiber, cosmetic or pharmaceutical. In another embodiment the inventive methods for the production are used to make agricultural products such as, but not limited to, plant extracts, proteins, amino acids, carbohydrates, fats, oils, polymers, vitamins, and the like.

In yet another embodiment the polynucleotide sequences or the polypeptide sequences of the invention are comprised in an agricultural product. In a particular embodiment the nucleic acid sequences and protein sequences of the invention may be used as product markers, for example where an agricultural product was produced by the methods of the invention. Such a marker can be used to identify a product to have been produced by an advantageous process resulting not only in a greater efficiency of the process but also improved quality of the product due to increased quality of the plant material and harvestable parts used in the process. Such markers can be detected by a variety of methods known in the art, for example but not limited to PCR based methods for nucleic acid detection or antibody based methods for protein detection.

The methods of the invention are advantageously applicable to any plant, in particular to any plant as defined herein. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to an embodiment of the present invention, the plant is a crop plant. Examples of crop plants include but are not limited to chicory, carrot, cassava, trefoil, soybean, beet, sugar beet, sunflower, canola, alfalfa, rapeseed, linseed, cotton, tomato, potato and tobacco. According to another embodiment of the present invention, the plant is a monocotyledonous plant. Examples of monocotyledonous plants include sugarcane. According to another embodiment of the present invention, the plant is a cereal. Examples of cereals include rice, maize, wheat, barley, millet, rye, triticale, sorghum, emmer, spelt, einkorn, teff, milo and oats. In a particular embodiment the plants used in the methods of the invention are selected from the group consisting of maize, wheat, rice, soybean, cotton, oilseed rape including canola, sugarcane, sugar beet and alfalfa.

The invention also extends to harvestable parts of a plant such as, but not limited to seeds, leaves, fruits, flowers, stems, roots, rhizomes, tubers and bulbs, which harvestable parts comprise a recombinant nucleic acid encoding a CDKB-RKA polypeptide. The invention furthermore relates to products derived or produced, preferably directly derived or produced, from a harvestable part of such a plant, such as dry pellets or powders, oil, fat and fatty acids, starch or proteins.

The present invention also encompasses use of nucleic acids encoding CDKB-RKA polypeptides as described herein and use of these CDKB-RKA polypeptides in enhancing any of the aforementioned yield-related traits in plants. For example, nucleic acids encoding CDKB-RKA polypeptide described herein, or the CDKB-RKA polypeptides themselves, may find use in breeding programs in which a DNA marker is identified which may be genetically linked to a CDKB-RKA polypeptide-encoding gene. The nucleic acids/genes, or the CDKB-RKA polypeptides themselves may be used to define a molecular marker. This DNA or protein marker may then be used in breeding programs to select plants having enhanced yield-related traits as defined hereinabove in the methods of the invention. Furthermore, allelic variants of a CDKB-RKA polypeptide-encoding nucleic acid/gene may find use in marker-assisted breeding programs. Nucleic acids encoding CDKB-RKA polypeptides may also be used as probes for genetically and physically mapping the genes that they are a part of, and as markers for traits linked to those genes. Such information may be useful in plant breeding in order to develop lines with desired phenotypes.

Embodiments

The present invention is characterized by one or more of the following embodiments.

1. A method for increasing yield in a plant relative to a control plant, comprising introducing and expressing in said plant a nucleic acid molecule encoding a CDKB-RKA polypeptide, wherein said CDKB-RKA polypeptide is a modified CDKB polypeptide, and wherein said CDKB-RKA polypeptide has reduced catalytic kinase activity, as compared to said CDKB polypeptide.

2. Method according to embodiment 1, wherein said CDKB-RKA polypeptide lacks catalytic kinase activity.

3. Method according to embodiment 1 or 2, wherein said CDKB-RKA polypeptide is a mutated CDKB polypeptide.

4. Method according to embodiment 1, 2 or 3, wherein said CDKB-RKA polypeptide is a CDKB polypeptide comprising a mutated kinase domain.

5. Method according to any of embodiments 1 to 4, wherein said CDKB-RKA polypeptide is a truncated CDKB polypeptide.

6. Method according to any of embodiments 1 to 5, wherein said CDKB-RKA polypeptide is a truncated CDKB polypeptide comprising a deletion in the C-terminal half of said polypeptide that reduces, and preferably substantially inactivates, that kinase activity of said CDKB.

7. Method according to any of embodiments 1 to 6 wherein said CDKB-RKA polypeptide is a truncated CDKB polypeptide comprising a deletion of the kinase domain of said CDKB polypeptide.

8. Method according to any of embodiments 1 to 6 wherein said CDKB-RKA polypeptide is a truncated CDKB polypeptide comprising a deletion of a part of the kinase domain of said CDKB polypeptide, wherein said part preferably comprises at least 1, 2, 3, 4, 5, 6, 7, 8, 9, 10, 11 or 12 amino acids.

9. Method according to any of embodiments 1 to 8, wherein said kinase domain of said CDKB polypeptide is represented by a conserved domain from amino acid 140 to 152 in SEQ ID NO: 320.

10. Method according to any of embodiments 1 to 9 wherein said CDKB polypeptide has cyclin-binding activity.

11. Method according to any of embodiments 1 to 10, wherein said CDKB-RKA polypeptide comprises a cyclin binding domain, preferably represented by a PPTALRE motif with no mismatches or with a mismatch at position 2 and/or 4 from left to right, and more preferably represented by SEQ ID NO: 403, and more preferably represented by SEQ ID NO: 404.

12. Method according to any of embodiments 1 to 11, wherein said CDKB-RKA polypeptide comprises a protein kinase inhibitory domain.

13. Method according to any of embodiments 1 to 12, wherein said CDKB-RKA polypeptide lacks a T-loop activation kinase domain.

14. Method according to any one of embodiments 1 to 13, wherein said nucleic acid encoding an CDKB-RKA is selected from any one of

- a nucleic acid derived from a nucleic acid encoding any one of the polypeptides listed in Table A2;
- a nucleic acid derived from an orthologue or paralogue of any of the polypeptides given in Table A2;
- a portion of a nucleic acid encoding any one of the polypeptides listed in Table A2 or of an orthologue or paralogue of any of the polypeptides given in Table A2;
- a nucleic acid capable of hybridising with a nucleic acid encoding any one of the polypeptides listed in Table A2 or of an orthologue or paralogue of any of the polypeptides given in Table A2.

15. Method according to any one of embodiments 1 to 14, wherein said nucleic acid encodes a polypeptide represented by SEQ ID NO: 318 or a homologue, orthologue or paralogue thereof, or a portion of said nucleic acid, or a nucleic acid capable of hybridising with said nucleic acid.

16. Method according to any one of embodiments 1 to 15, wherein said nucleic acid encoding said CDKB polypeptide is of plant origin, preferably from a dicotyledonous plant, further preferably from the family *Brassicaceae,* more preferably from the genus *Arabidopsis,* most preferably from *Arabidopsis thaliana.*

17. Method according to any one of embodiments 1 to 16, wherein said nucleic acid encodes a polypeptide having has at least about 30-40%, preferably at least about 41-50%, more preferably at least about 51-60%, 61-70%, 71-80%, 81-85%, 86-90%, 91-95%, 96-99% or more than 99% overall sequence identity to SEQ ID NO: 318, and preferably corresponding to SEQ ID NO: 318.

18. Method according to any of embodiments 1 to 17, wherein said increased yield relative to control plants comprises (i) increased seed yield and/or (ii) increased vegetative biomass, such as increased leaf biomass and/or increased root biomass.

19. Method according to any one of embodiments 1 to 18, wherein said enhanced yield-related traits are obtained under non-stress conditions.

20. Method according to any one of embodiments 1 to 19 wherein said nucleic acid is operably linked to a constitutive promoter, preferably to a constitutive plant promoter, more preferably to a medium strength constitutive plant promoter.

21. Transgenic plant, transgenic plant part thereof, including seeds, or transgenic plant cell, obtainable by a method according to any one of embodiments 1 to 20, wherein said plant, plant part or plant cell comprises a recombinant nucleic acid encoding a CDKB-RKA polypeptide as defined in any of embodiments 1 and 17.

22. Construct comprising:

(i) nucleic acid encoding a CDKB-RKA polypeptide as defined in any of embodiments 1 to 17
(ii) one or more control sequences capable of driving expression of the nucleic acid sequence of (i); and optionally
(iii) a transcription termination sequence.

23. Construct according to embodiment 22, wherein one of said control sequences is a constitutive promoter, preferably to a constitutive plant promoter, more preferably to a medium strength constitutive plant promoter.

24. Use of a construct according to embodiment 22 or 23 in a method for making plants having enhanced yield-related traits, preferably increased yield relative to control plants, and more preferably increased seed yield and/or increased biomass relative to control plants.

25. Transgenic plant, transgenic plant part or transgenic plant cell transformed with a construct according to embodiment 22 or 23.

26. Method for the production of a transgenic plant having enhanced yield-related traits relative to control plants, preferably increased yield relative to control plants, and more preferably increased seed yield and/or increased biomass relative to control plants, comprising:

(a) introducing and expressing in a plant cell or plant a nucleic acid encoding a CDKB-RKA polypeptide as defined in any of embodiments 1 to 17; and
(b) cultivating said plant cell or plant under conditions promoting plant growth and development.

27. Transgenic plant having enhanced yield-related traits relative to control plants, preferably increased yield relative to control plants, and more preferably increased seed yield and/or increased biomass, resulting from the introduction and expression therein of a nucleic acid encoding a CDKB-RKA polypeptide as defined in any of embodiments 1 to 17, or a transgenic plant cell derived from said transgenic plant.

28. Transgenic plant according to embodiment 21, 25 or 27, or a transgenic plant cell derived therefrom, wherein said plant is a crop plant, such as beet, sugarbeet or alfalfa; or a monocotyledonous plant such as sugarcane; or a cereal, such as rice, maize, wheat, barley, millet, rye, triticale, sorghum, emmer, spelt, einkorn, teff, milo or oats.

29. Harvestable parts of a plant according to any of embodiments 21, 25, 27 or 28 wherein said harvestable parts preferably are seeds.

# EP 3 091 079 A1

30. Products derived from or produced from a plant according to embodiment 29 and/or from harvestable parts of a plant according to any of embodiments 21, 25, 27 or 28.

31. Use of a nucleic acid encoding a CDKB-RKA polypeptide as defined in any of embodiments 1 to 17 for enhancing yield-related traits in plants relative to control plants, preferably for increasing yield, and more preferably for increasing seed yield and/or for increasing biomass in plants relative to control plants.

32. A method for the production of a product comprising the steps of growing the plants according to any of embodiments 21, 25, 27 or 28 and producing said product from or by

(a) said plants; or
(b) parts, including seeds, of said plants.

33. Construct according to embodiment 22 or 23 comprised in a plant cell.

34. Recombinant chromosomal DNA comprising the construct according to embodiment 22 or 23.

35. Use of a nucleic acid encoding a CDKB-RKA polypeptide as defined in any of embodiments 1 to 17 as molecular marker for selecting for enhanced yield-related traits in plants relative to control plants, preferably for selecting for increased yield, and more preferably selecting for increasing seed yield and/or selecting for increasing biomass in plants relative to control plants.

In other embodiments, the invention is characterized by one or more of the following embodiments I to XV:

I. A method for increasing yield in a plant relative to a control plant, comprising introducing and expressing in said plant a nucleic acid molecule encoding a CDKB-RKA polypeptide, wherein said CDKB-RKA polypeptide is a modified CDKB polypeptide.

II. Method according to embodiment I, wherein said CDKB-RKA polypeptide lacks catalytic kinase activity.

III. Method according to embodiment I or II, wherein said CDKB-RKA polypeptide is a mutated and/or a truncated CDKB polypeptide.

IV. Method according to any of embodiments I to III, wherein said CDKB-RKA polypeptide is a truncated CDKB polypeptide comprising a deletion in the C-terminal half of said polypeptide that reduces, and preferably substantially inactivates, that kinase activity of said CDKB polypeptide.

V. Method according to any of embodiments I to IV, wherein said CDKB-RKA polypeptide is a truncated CDKB polypeptide comprising a deletion of the kinase domain or a part thereof of said CDKB polypeptide.

VI. Method according to any of embodiments I to V, wherein said CDKB-RKA polypeptide comprises a cyclin binding domain represented by a PPTALRE motif with no mismatches or with a mismatch at position 2 and/or 4 from left to right, preferably represented by SEQ ID NO: 403, and more preferably represented by SEQ ID NO: 404.

VII. Method according to any one of embodiments I to VI, wherein said nucleic acid encodes a polypeptide represented by SEQ ID NO: 318 or a homologue, orthologue or paralogue thereof, or a portion of said nucleic acid, or a nucleic acid capable of hybridising with said nucleic acid.

VIII. Method according to any of embodiments I to VII, wherein said increased yield relative to control plants comprises (i) increased seed yield and/or (ii) increased vegetative biomass, such as increased leaf biomass and/or increased root biomass.

IX. Method according to any one of embodiments I to VIII, wherein said nucleic acid is operably linked to a constitutive promoter, preferably to a constitutive plant promoter, more preferably to a medium strength constitutive plant promoter.

X. Plant, plant part thereof, including seeds, or plant cell, obtainable by a method according to any one of embodiments I to IX, wherein said plant, plant part or plant cell comprises a recombinant nucleic acid encoding a CDKB-RKA polypeptide as defined in any of embodiments I to VII.

XI. Construct comprising:

(i) nucleic acid encoding a CDKB-RKA polypeptide as defined in any of embodiments I to VII;
(ii) one or more control sequences capable of driving expression of the nucleic acid sequence of (i); wherein one of said control sequences is a constitutive promoter, preferably to a constitutive plant promoter, more preferably to a medium strength constitutive plant promoter and optionally
(iii) a transcription termination sequence.

XII. Plant, plant part or plant cell transformed with a construct according to embodiment XI. XIII. Method for the production of a transgenic plant having increased yield relative to control plants, and preferably increased seed yield and/or increased biomass such as increased vegetative biomass relative to control plants, comprising:

(i) introducing and expressing in a plant cell or plant a nucleic acid encoding a CDKB-RKA polypeptide as defined in any of embodiments I to VII; and
(ii) cultivating said plant cell or plant under conditions promoting plant growth and development.

XIV. Transgenic plant having increased yield relative to control plants, and preferably increased seed yield and/or increased biomass such as increased vegetative biomass, resulting from the introduction and expression therein of a nucleic acid encoding a CDKB-RKA polypeptide as defined in any of embodiments I to VII or a transgenic plant cell derived from said transgenic plant.

XV. Use of a nucleic acid encoding a CDKB-RKA polypeptide as defined in any of embodiments I to VII for enhancing yield-related traits in plants relative to control plants, preferably for increasing yield, and more preferably for increasing seed yield and/or for increasing biomass biomass in plants relative to control plants and/or use of a construct according to embodiment XI in a method for making plants having enhanced yield-related traits, preferably increased yield relative to control plants, and more preferably increased seed yield and/or increased biomass relative to control plants.

**Definitions**

The following definitions will be used throughout the present application. The section captions and headings in this application are for convenience and reference purpose only and should not affect in any way the meaning or interpretation of this application. The technical terms and expressions used within the scope of this application are generally to be given the meaning commonly applied to them in the pertinent art of plant biology, molecular biology, bioinformatics and plant breeding. All of the following term definitions apply to the complete content of this application. The term "essentially", "about", "approximately" and the like in connection with an attribute or a value, particularly also define exactly the attribute or exactly the value, respectively. The term "about" in the context of a given numeric value or range relates in particular to a value or range that is within 20%, within 10%, or within 5% of the value or range given. As used herein, the term "comprising" also encompasses the term "consisting of".

Peptide(s)/Protein(s)

The terms "peptides", "oligopeptides", "polypeptide" and "protein" are used interchangeably herein and refer to amino acids in a polymeric form of any length, linked together by peptide bonds, unless mentioned herein otherwise.

Polynucleotide(s)/Nucleic acid(s)/Nucleic acid sequence(s)/nucleotide sequence(s)

The terms "polynucleotide(s)", "nucleic acid sequence(s)", "nucleotide sequence(s)", "nucleic acid(s)", "nucleic acid molecule" are used interchangeably herein and refer to nucleotides, either ribonucleotides or deoxyribonucleotides or a combination of both, in a polymeric unbranched form of any length.

Homologue(s)

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

Orthologues and paralogues are two different forms of homologues and encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

A "deletion" refers to removal of one or more amino acids from a protein.

An "insertion" refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag·100 epitope, c-myc epitope, FLAG®-epitope, lacZ, CMP (calmodulin-binding peptide), HA epitope, protein C epitope and VSV epitope.

A "substitution" refers to replacement of amino acids of the protein with other amino acids having similar properties (such as similar hydrophobicity, hydrophilicity, antigenicity, propensity to form or break $\alpha$-helical structures or $\beta$-sheet structures). Amino acid substitutions are typically of single residues, but may be clustered depending upon functional constraints placed upon the polypeptide and may range from 1 to 10 amino acids. The amino acid substitutions are preferably conservative amino acid substitutions. Conservative substitution tables are well known in the art (see for example Creighton (1984) Proteins. W.H. Freeman and Company (Eds) and Table 1 below).

**Table 1:** Examples of conserved amino acid substitutions

| Residue | Conservative Substitutions | Residue | Conservative Substitutions |
|---------|----------------------------|---------|----------------------------|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

Amino acid substitutions, deletions and/or insertions may readily be made using peptide synthetic techniques known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols (see Current Protocols in Molecular Biology, John Wiley & Sons, N.Y. (1989 and yearly updates)).

Derivatives

"Derivatives" include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

Domain, Motif/Consensus sequence/Signature

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.
The term "motif" or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).
Specialist databases exist for the identification of domains, for example, SMART (Schultz et al. (1998) Proc.

Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32:D134-D137, (2004)), or Pfam (Bateman et al., Nucleic Acids Research 30(1): 276-280 (2002)). A set of tools for *in silico* analysis of protein sequences is available on the ExPASy proteomics server (Swiss Institute of Bioinformatics (Gasteiger et al., ExPASy: the proteomics server for in-depth protein knowledge and analysis, Nucleic Acids Res. 31:3784-3788(2003)). Domains or motifs may also be identified using routine techniques, such as by sequence alignment.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid or amino acid sequence or over selected domains or conserved motif(s), using the programs mentioned above using the default parameters. For local alignments, the Smith-Waterman algorithm is particularly useful (Smith TF, Waterman MS (1981) J. Mol. Biol 147(1);195-7).

Reciprocal BLAST

Typically, this involves a first BLAST involving BLASTing a query sequence (for example using any of the sequences listed in Tables A of the Examples section) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived. The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

Hybridisation

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitro-cellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting point ($T_m$) for the specific sequence at a defined ionic strength and pH. Medium stringency conditions are when the temperature is 20°C below $T_m$, and high stringency conditions are when the temperature is 10°C below $T_m$. High stringency hybridisation conditions are typically used for isolating hybridising sequences that have high sequence similarity to the target nucleic acid sequence. However, nucleic acids may deviate in sequence and still encode a substantially identical polypeptide, due to the degeneracy of the genetic code. Therefore medium stringency hybridisation conditions may sometimes be needed to identify such nucleic acid molecules.

The $T_m$ is the temperature under defined ionic strength and pH, at which 50% of the target sequence hybridises to a perfectly matched probe. The $T_m$ is dependent upon the solution conditions and the base composition and length of the probe. For example, longer sequences hybridise specifically at higher temperatures. The maximum rate of hybridisation is obtained from about 16°C up to 32°C below $T_m$. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tm decreases about 1°C per % base mismatch. The $T_m$ may be calculated using the following equations, depending on the types of hybrids:

1) DNA-DNA hybrids (Meinkoth and Wahl, Anal. Biochem., 138: 267-284, 1984):

$$T_m = 81.5°C + 16.6 \times \log_{10}[Na^+]^a + 0.41 \times \%[G/C^b] - 500 \times [L^c]^{-1} - 0.61 \times \% \text{ formamide}$$

2) DNA-RNA or RNA-RNA hybrids:

$$T_m = 79.8°C + 18.5 (\log_{10}[Na^+]^a) + 0.58 (\%G/C^b) + 11.8 (\%G/C^b)^2 - 820/L^c$$

3) oligo-DNA or oligo-RNA[d] hybrids:

For <20 nucleotides: $T_m = 2 (l_n)$
For 20-35 nucleotides: $T_m = 22 + 1.46 (l_n)$

[a] or for other monovalent cation, but only accurate in the 0.01-0.4 M range.
[b] only accurate for %GC in the 30% to 75% range.
[c] L = length of duplex in base pairs.

[d] oligo, oligonucleotide; $l_n$, = effective length of primer = $2 \times$(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected. The skilled artisan is aware of various parameters which may be altered during washing and which will either maintain or change the stringency conditions.

For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acids of known sequence are hybridised, the hybrid length may be determined by aligning the sequences and identifying the conserved regions described herein. $1\times$SSC is 0.15M NaCl and 15mM sodium citrate; the hybridisation solution and wash solutions may additionally include 5x Denhardt's reagent, 0.5-1.0% SDS, 100 $\mu$g/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition, Cold Spring Harbor Laboratory Press, CSH, New York or to Current Protocols in Molecular Biology, John Wiley & Sons, N.Y. (1989 and yearly updates).

Splice variant

The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex (2005) BMC Bioinformatics 6: 25).

Allelic variant

"Alleles" or "allelic variants" are alternative forms of a given gene, located at the same chromosomal position. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

Endogenous gene

Reference herein to an "endogenous" gene not only refers to the gene in question as found in a plant in its natural form (i.e., without there being any human intervention), but also refers to that same gene (or a substantially homologous nucleic acid/gene) in an isolated form subsequently (re)introduced into a plant (a transgene). For example, a transgenic plant containing such a transgene may encounter a substantial reduction of the transgene expression and/or substantial reduction of expression of the endogenous gene. The isolated gene may be isolated from an organism or may be manmade, for example by chemical synthesis.

Gene shuffling/Directed evolution

"Gene shuffling" or "directed evolution" consists of iterations of DNA shuffling followed by appropriate screening and/or selection to generate variants of nucleic acids or portions thereof encoding proteins having a modified biological activity (Castle et al., (2004) Science 304(5674): 1151-4; US patents 5,811,238 and 6,395,547).

Construct

Artificial DNA (such as but, not limited to plasmids or viral DNA) capable of replication in a host cell and used for introduction of a DNA sequence of interest into a host cell or host organism. Host cells of the invention may be any cell selected from bacterial cells, such as Escherichia coli or Agrobacterium species cells, yeast cells, fungal, algal or cyanobacterial cells or plant cells. The skilled artisan is well aware of the genetic elements that must be present on the genetic construct in order to successfully transform, select and propagate host cells containing the sequence of interest. The sequence of interest is operably linked to one or more control sequences (at least to a promoter) as described herein. Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

Regulatory element/Control sequence/Promoter

The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or-10 box transcriptional regulatory sequences. The term "regulatory element" also encompasses a synthetic fusion molecule or derivative that confers, activates or enhances expression of a nucleic acid molecule in a cell, tissue or organ.

A "plant promoter" comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule must, as described above, be linked operably to or comprise a suitable promoter which expresses the gene at the right point in time and with the required spatial expression pattern.

For the identification of functionally equivalent promoters, the promoter strength and/or expression pattern of a candidate promoter may be analysed for example by operably linking the promoter to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell. Generally, by "medium strength promoter" is intended a promoter that drives expression of a coding sequence at a lower level than a strong promoter, in particular at a level that is in all instances below that obtained when under the control of a 35S CaMV promoter.

Operably linked

The term "operably linked" as used herein refers to a functional linkage between the promoter sequence and the gene of interest, such that the promoter sequence is able to initiate transcription of the gene of interest.

<u>Constitutive promoter</u>

A "constitutive promoter" refers to a promoter that is transcriptionally active during most, but not necessarily all, phases of growth and development and under most environmental conditions, in at least one cell, tissue or organ. Table 2a below gives examples of constitutive promoters.

Table 2a: Examples of constitutive promoters

| Gene Source | Reference |
|---|---|
| Actin | McElroy et al, Plant Cell, 2: 163-171, 1990 |
| HMGP | WO 2004/070039 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| 34S FMV | Sanger et al., Plant. Mol. Biol., 14, 1990: 433-443 |
| Rubisco small subunit | US 4,962,028 |
| OCS | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

<u>Ubiquitous promoter</u>

A "ubiquitous promoter" is active in substantially all tissues or cells of an organism.

<u>Developmentally-regulated promoter</u>

A "developmentally-regulated promoter" is active during certain developmental stages or in parts of the plant that undergo developmental changes.

<u>Inducible promoter</u>

An "inducible promoter" has induced or increased transcription initiation in response to a chemical (for a review see Gatz 1997, Annu. Rev. Plant Physiol. Plant Mol. Biol., 48:89-108), environmental or physical stimulus, or may be "stress-inducible", i.e. activated when a plant is exposed to various stress conditions, or a "pathogen-inducible" i.e. activated when a plant is exposed to exposure to various pathogens.

<u>Organ-specific/Tissue-specific promoter</u>

An "organ-specific" or "tissue-specific promoter" is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

Examples of root-specific promoters are listed in Table 2b below:

**Table 2b:** Examples of root-specific promoters

| Gene Source | Reference |
|---|---|
| RCc3 | Plant Mol Biol. 1995 Jan;27(2):237-48 |
| Arabidopsis PHT1 | Koyama et al. J Biosci Bioeng. 2005 Jan;99(1):38-42.; Mudge et al. (2002, Plant J. 31:341) |
| Medicago phosphate transporter | Xiao et al., 2006, Plant Biol (Stuttg). 2006 Jul;8(4):439-49 |
| Arabidopsis Pyk10 | Nitz et al. (2001) Plant Sci 161(2): 337-346 |
| root-expressible genes | Tingey et al., EMBO J. 6: 1, 1987. |
| tobacco auxin-inducible gene | Van der Zaal et al., Plant Mol. Biol. 16, 983, 1991. |
| β-tubulin | Oppenheimer, et al., Gene 63: 87, 1988. |
| tobacco root-specific genes | Conkling, et al., Plant Physiol. 93: 1203, 1990. |
| B. napus G1-3b gene | United States Patent No. 5, 401, 836 |
| SbPRP1 | Suzuki et al., Plant Mol. Biol. 21: 109-119, 1993. |
| LRX1 | Baumberger et al. 2001, Genes & Dev. 15:1128 |
| BTG-26 Brassica napus | US 20050044585 |
| LeAMT1 (tomato) | Lauter et al. (1996, PNAS 3:8139) |
| The LeNRT1-1 (tomato) | Lauter et al. (1996, PNAS 3:8139) |
| class I patatin gene (potato) | Liu et al., Plant Mol. Biol. 17 (6): 1139-1154 |
| KDC1 (Daucus carota) | Downey et al. (2000, J. Biol. Chem. 275:39420) |
| TobRB7 gene | W Song (1997) PhD Thesis, North Carolina State University, Raleigh, NC USA |
| OsRAB5a (rice) | Wang et al. 2002, Plant Sci. 163:273 |
| ALF5 (Arabidopsis) | Diener et al. (2001, Plant Cell 13:1625) |
| NRT2;1Np (N. plumbaginifolia) | Quesada et al. (1997, Plant Mol. Biol. 34:265) |

A "seed-specific promoter" is transcriptionally active predominantly in seed tissue, but not necessarily exclusively in seed tissue (in cases of leaky expression). The seed-specific promoter may be active during seed development and/or during germination. The seed specific promoter may be endosperm/aleurone/embryo specific. Examples of seed-specific promoters (endosperm/aleurone/embryo specific) are shown in Table 2c to Table 2f below. Further examples of seed-specific promoters are given in Qing Qu and Takaiwa (Plant Biotechnol. J. 2, 113-125, 2004), which disclosure is incorporated by reference herein as if fully set forth.

**Table 2c:** Examples of seed-specific promoters

| Gene source | Reference |
|---|---|
| seed-specific genes | Simon et al., Plant Mol. Biol. 5: 191, 1985; |
| | Scofield et al., J. Biol. Chem. 262: 12202, 1987.; |
| | Baszczynski et al., Plant Mol. Biol. 14: 633, 1990. |
| Brazil Nut albumin | Pearson et al., Plant Mol. Biol. 18: 235-245, 1992. |
| legumin | Ellis et al., Plant Mol. Biol. 10: 203-214, 1988. |
| glutelin (rice) | Takaiwa et al., Mol. Gen. Genet. 208: 15-22, 1986; |

(continued)

| Gene source | Reference |
|---|---|
| | Takaiwa et al., FEBS Letts. 221: 43-47, 1987. |
| zein | Matzke et al Plant Mol Biol, 14(3):323-32 1990 |
| napA | Stalberg et al, Planta 199: 515-519, 1996. |
| wheat LMW and HMW glutenin-1 | Mol Gen Genet 216:81-90, 1989; NAR 17:461-2, 1989 |
| wheat SPA | Albani et al, Plant Cell, 9: 171-184, 1997 |
| wheat $\alpha$, $\beta$, Y-gliadins | EMBO J. 3:1409-15, 1984 |
| barley Itr1 promoter | Diaz et al. (1995) Mol Gen Genet 248(5):592-8 |
| barley B1, C, D, hordein | Theor Appl Gen 98:1253-62, 1999; Plant J 4:343-55, 1993; Mol Gen Genet 250:750-60, 1996 |
| barley DOF | Mena et al, The Plant Journal, 116(1): 53-62, 1998 |
| blz2 | EP99106056.7 |
| synthetic promoter | Vicente-Carbajosa et al., Plant J. 13: 629-640, 1998. |
| rice prolamin NRP33 | Wu et al, Plant Cell Physiology 39(8) 885-889, 1998 |
| rice $\alpha$-globulin Glb-1 | Wu et al, Plant Cell Physiology 39(8) 885-889, 1998 |
| rice OSH1 | Sato et al, Proc. Natl. Acad. Sci. USA, 93: 8117-8122, 1996 |
| rice $\alpha$-globulin REB/OHP-1 | Nakase et al. Plant Mol. Biol. 33: 513-522, 1997 |
| rice ADP-glucose pyrophosphorylase | Trans Res 6:157-68, 1997 |
| maize ESR gene family | Plant J 12:235-46, 1997 |
| sorghum $\alpha$-kafirin | DeRose et al., Plant Mol. Biol 32:1029-35, 1996 |
| KNOX | Postma-Haarsma et al, Plant Mol. Biol. 39:257-71, 1999 |
| rice oleosin | Wu et al, J. Biochem. 123:386, 1998 |
| sunflower oleosin | Cummins et al., Plant Mol. Biol. 19: 873-876, 1992 |
| PRO0117, putative rice 40S ribosomal protein | WO 2004/070039 |
| PRO0136, rice alanine aminotransferase | unpublished |
| PRO0147, trypsin inhibitor ITR1 (barley) | unpublished |
| PRO0151, rice WSI18 | WO 2004/070039 |
| PRO0175, rice RAB21 | WO 2004/070039 |
| PRO005 | WO 2004/070039 |
| PRO0095 | WO 2004/070039 |
| $\alpha$-amylase (Amy32b) | Lanahan et al, Plant Cell 4:203-211, 1992; Skriver et al, Proc Natl Acad Sci USA 88:7266-7270, 1991 |
| cathepsin $\beta$-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

**Table 2d:** examples of endosperm-specific promoters

| Gene source | Reference |
| --- | --- |
| glutelin (rice) | Takaiwa et al. (1986) Mol Gen Genet 208:15-22; Takaiwa et al. (1987) FEBS Letts. 221:43-47 |
| zein | Matzke et al., (1990) Plant Mol Biol 14(3): 323-32 |
| wheat LMW and HMW glutenin-1 | Colot et al. (1989) Mol Gen Genet 216:81-90, Anderson et al. (1989) NAR 17:461-2 |
| wheat SPA | Albani et al. (1997) Plant Cell 9:171-184 |
| wheat gliadins | Rafalski et al. (1984) EMBO 3:1409-15 |
| barley Itr1 promoter | Diaz et al. (1995) Mol Gen Genet 248(5):592-8 |
| barley B1, C, D, hordein | Cho et al. (1999) Theor Appl Genet 98:1253-62; Muller et al. (1993) Plant J 4:343-55; Sorenson et al. (1996) Mol Gen Genet 250:750-60 |
| barley DOF | Mena et al, (1998) Plant J 116(1): 53-62 |
| blz2 | Onate et al. (1999) J Biol Chem 274(14):9175-82 |
| synthetic promoter | Vicente-Carbajosa et al. (1998) Plant J 13:629-640 |
| rice prolamin NRP33 | Wu et al, (1998) Plant Cell Physiol 39(8) 885-889 |
| rice globulin Glb-1 | Wu et al. (1998) Plant Cell Physiol 39(8) 885-889 |
| rice globulin REB/OHP-1 | Nakase et al. (1997) Plant Molec Biol 33: 513-522 |
| rice ADP-glucose pyrophosphorylase | Russell et al. (1997) Trans Res 6:157-68 |
| maize ESR gene family | Opsahl-Ferstad et al. (1997) Plant J 12:235-46 |
| sorghum kafirin | DeRose et al. (1996) Plant Mol Biol 32:1029-35 |

**Table 2e:** Examples of embryo specific promoters:

| Gene source | Reference |
| --- | --- |
| rice OSH1 | Sato et al, Proc. Natl. Acad. Sci. USA, 93: 8117-8122, 1996 |
| KNOX | Postma-Haarsma et al, Plant Mol. Biol. 39:257-71, 1999 |
| PRO0151 | WO 2004/070039 |
| PRO0175 | WO 2004/070039 |
| PRO005 | WO 2004/070039 |
| PRO0095 | WO 2004/070039 |

**Table 2f:** Examples of aleurone-specific promoters:

| Gene source | Reference |
| --- | --- |
| $\alpha$-amylase (Amy32b) | Lanahan et al, Plant Cell 4:203-211, 1992; Skriver et al, Proc Natl Acad Sci USA 88:7266-7270, 1991 |
| cathepsin $\beta$-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

A "green tissue-specific promoter" as defined herein is a promoter that is transcriptionally active predominantly in green tissue, substantially to the exclusion of any other parts of a plant, whilst still allowing for any leaky expression in these other plant parts.

Examples of green tissue-specific promoters which may be used to perform the methods of the invention are shown in Table 2g below.

**Table 2g:** Examples of green tissue-specific promoters

| Gene | Expression | Reference |
|------|-----------|-----------|
| Maize Orthophosphate dikinase | Leaf specific | Fukavama et al., Plant Physiol. 2001 Nov;127(3):1136-46 |
| Maize Phosphoenolpyruvate carboxylase | Leaf specific | Kausch et al., Plant Mol Biol. 2001 Jan;45(1):1-15 |
| Rice Phosphoenolpyruvate carboxylase | Leaf specific | Lin et al., 2004 DNA Seq. 2004 Aug;15(4):269-76 |
| Rice small subunit Rubisco | Leaf specific | Nomura et al., Plant Mol Biol. 2000 Sep;44(1):99-106 |
| rice beta expansin EXBP9 | Shoot specific | WO 2004/070039 |
| Pigeonpea small subunit Rubisco | Leaf specific | Panguluri et al., Indian J Exp Biol. 2005 Apr;43(4):369-72 |
| Pea RBCS3A | Leaf specific | |

Another example of a tissue-specific promoter is a meristem-specific promoter, which is transcriptionally active predominantly in meristematic tissue, substantially to the exclusion of any other parts of a plant, whilst still allowing for any leaky expression in these other plant parts. Examples of green meristem-specific promoters which may be used to perform the methods of the invention are shown in Table 2h below.

**Table 2h:** Examples of meristem-specific promoters

| Gene source | Expression pattern | Reference |
|-------------|-------------------|-----------|
| rice OSH1 | Shoot apical meristem, from embryo globular stage | Sato et al. (1996) Proc. Natl. Acad. Sci. USA, 93: 8117-8122 |
| | to seedling stage | |
| Rice metallothionein | Meristem specific | BAD87835.1 |
| WAK1 & WAK 2 | Shoot and root apical meristems, and in expanding leaves and sepals | Wagner & Kohorn (2001) Plant Cell 13(2): 303-318 |

Terminator

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

Selectable marker (gene)/Reporter gene

"Selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptII that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta®; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the loxP sequences. If the marker gene is integrated between the loxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

Transgenic/Transgene/Recombinant

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either

(a) the nucleic acid sequences encoding proteins useful in the methods of the invention, or
(b) genetic control sequence(s) which is operably linked with the nucleic acid sequence according to the invention, for example a promoter, or
(c) a) and b)

are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

A transgenic plant for the purposes of the invention is thus understood as meaning, as above, that the nucleic acids used in the method of the invention are not present in, or originating from, the genome of said plant, or are present in the genome of said plant but not at their natural locus in the genome of said plant, it being possible for the nucleic acids to be expressed homologously or heterologously. However, as mentioned, transgenic also means that, while the nucleic acids according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, preferably, heterologous expression of the nucleic acids takes place. Preferred transgenic plants are mentioned herein.

It shall further be noted that in the context of the present invention, the term "isolated nucleic acid" or "isolated polypeptide" may in some instances be considered as a synonym for a "recombinant nucleic acid" or a "recombinant polypeptide", respectively and refers to a nucleic acid or polypeptide that is not located in its natural genetic environment and/or that has been modified by recombinant methods.

Modulation

The term "modulation" means in relation to expression or gene expression, a process in which the expression level is changed by said gene expression in comparison to the control plant, the expression level may be increased or decreased. The original, unmodulated expression may be of any kind of expression of a structural RNA (rRNA, tRNA) or mRNA with subsequent translation. For the purposes of this invention, the original unmodulated expression may also be absence of any expression. The term "modulating the activity" shall mean any change of the expression of the inventive nucleic acid sequences or encoded proteins, which leads to increased yield and/or increased growth of the plants. The expression can increase from zero (absence of, or immeasurable expression) to a certain amount, or can decrease from a certain amount to immeasurable small amounts or zero.

Expression

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

Increased expression/overexpression

The term "increased expression" or "overexpression" as used herein means any form of expression that is additional to the original wild-type expression level. For the purposes of this invention, the original wild-type expression level might also be zero, i.e. absence of expression or immeasurable expression.

Methods for increasing expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest. For example, endogenous promoters may be altered in vivo by mutation, deletion, and/or substitution (see, Kmiec, US 5,565,350; Zarling et al., WO9322443), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. For general information see: The Maize Handbook, Chapter 116, Freeling and Walbot, Eds., Springer, N.Y. (1994).

Decreased expression

Reference herein to "decreased expression" or "reduction or substantial elimination" of expression is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants.
For the reduction or substantial elimination of expression an endogenous gene in a plant, a sufficient length of substantially contiguous nucleotides of a nucleic acid sequence is required. In order to perform gene silencing, this may be as little as 20, 19, 18, 17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from the nucleic acid encoding the protein of interest (target gene), or from any nucleic acid capable of encoding an orthologue, paralogue or homologue of the protein of interest. Preferably, the stretch of substantially contiguous nucleotides is capable of forming hydrogen bonds with the target gene (either sense or antisense strand), more preferably, the stretch of substantially contiguous nucleotides has, in increasing order of preference, 50%, 60%, 70%, 80%, 85%, 90%, 95%, 96%, 97%, 98%, 99%, 100% sequence identity to the target gene (either sense or antisense strand). A nucleic acid sequence encoding a (functional) polypeptide is not a requirement for the various methods discussed herein for the reduction or substantial elimination of expression of an endogenous gene.
This reduction or substantial elimination of expression may be achieved using routine tools and techniques. A preferred method for the reduction or substantial elimination of endogenous gene expression is by introducing and expressing in a plant a genetic construct into which the nucleic acid (in this case a stretch of substantially contiguous nucleotides derived from the gene of interest, or from any nucleic acid capable of encoding an orthologue, paralogue or homologue of any one of the protein of interest) is cloned as an inverted repeat (in part or completely), separated by a spacer (non-coding DNA).
In such a preferred method, expression of the endogenous gene is reduced or substantially eliminated through RNA-mediated silencing using an inverted repeat of a nucleic acid or a part thereof (in this case a stretch of substantially contiguous nucleotides derived from the gene of interest, or from any nucleic acid capable of encoding an orthologue, paralogue or homologue of the protein of interest), preferably capable of forming a hairpin structure. The inverted repeat is cloned in an expression vector comprising control sequences. A non-coding DNA nucleic acid sequence (a spacer, for example a matrix attachment region fragment (MAR), an intron, a polylinker, etc.) is located between the two inverted nucleic acids forming the inverted repeat. After transcription of the inverted repeat, a chimeric RNA with a self-complementary structure is formed (partial or complete). This double-stranded RNA structure is referred to as the hairpin RNA (hpRNA). The hpRNA is processed by the plant into siRNAs that are incorporated into an RNA-induced silencing complex (RISC). The RISC further cleaves the mRNA transcripts, thereby substantially reducing the number of mRNA transcripts to be translated into polypeptides. For further general details see for example, Grierson et al. (1998) WO 98/53083; Waterhouse et al. (1999) WO 99/53050).
Performance of the methods of the invention does not rely on introducing and expressing in a plant a genetic construct into which the nucleic acid is cloned as an inverted repeat, but any one or more of several well-known "gene silencing" methods may be used to achieve the same effects.
One such method for the reduction of endogenous gene expression is RNA-mediated silencing of gene expression (downregulation). Silencing in this case is triggered in a plant by a double stranded RNA sequence (dsRNA) that is substantially similar to the target endogenous gene. This dsRNA is further processed by the plant into about 20 to about 26 nucleotides called short interfering RNAs (siRNAs). The siRNAs are incorporated into an RNA-induced silencing complex (RISC) that cleaves the mRNA transcript of the endogenous target gene, thereby substantially reducing the number of mRNA transcripts to be translated into a polypeptide. Preferably, the double stranded RNA sequence corresponds to a target gene.
Another example of an RNA silencing method involves the introduction of nucleic acid sequences or parts thereof (in this case a stretch of substantially contiguous nucleotides derived from the gene of interest, or from any nucleic acid capable of encoding an orthologue, paralogue or homologue of the protein of interest) in a sense orientation into a plant. "Sense orientation" refers to a DNA sequence that is homologous to an mRNA transcript thereof. Introduced into a plant would therefore be at least one copy of the nucleic acid sequence. The additional nucleic acid sequence will reduce expression of the endogenous gene, giving rise to a phenomenon known as co-suppression. The reduction of gene expression will be more pronounced if several additional copies of a nucleic acid sequence are introduced into the plant, as there is a positive correlation between high transcript levels and the triggering of co-suppression.

Another example of an RNA silencing method involves the use of antisense nucleic acid sequences. An "antisense" nucleic acid sequence comprises a nucleotide sequence that is complementary to a "sense" nucleic acid sequence encoding a protein, i.e. complementary to the coding strand of a double-stranded cDNA molecule or complementary to an mRNA transcript sequence. The antisense nucleic acid sequence is preferably complementary to the endogenous gene to be silenced. The complementarity may be located in the "coding region" and/or in the "non-coding region" of a gene. The term "coding region" refers to a region of the nucleotide sequence comprising codons that are translated into amino acid residues. The term "non-coding region" refers to 5' and 3' sequences that flank the coding region that are transcribed but not translated into amino acids (also referred to as 5' and 3' untranslated regions).

Antisense nucleic acid sequences can be designed according to the rules of Watson and Crick base pairing. The antisense nucleic acid sequence may be complementary to the entire nucleic acid sequence (in this case a stretch of substantially contiguous nucleotides derived from the gene of interest, or from any nucleic acid capable of encoding an orthologue, paralogue or homologue of the protein of interest), but may also be an oligonucleotide that is antisense to only a part of the nucleic acid sequence (including the mRNA 5' and 3' UTR). For example, the antisense oligonucleotide sequence may be complementary to the region surrounding the translation start site of an mRNA transcript encoding a polypeptide. The length of a suitable antisense oligonucleotide sequence is known in the art and may start from about 50, 45, 40, 35, 30, 25, 20, 15 or 10 nucleotides in length or less. An antisense nucleic acid sequence according to the invention may be constructed using chemical synthesis and enzymatic ligation reactions using methods known in the art. For example, an antisense nucleic acid sequence (e.g., an antisense oligonucleotide sequence) may be chemically synthesized using naturally occurring nucleotides or variously modified nucleotides designed to increase the biological stability of the molecules or to increase the physical stability of the duplex formed between the antisense and sense nucleic acid sequences, e.g., phosphorothioate derivatives and acridine substituted nucleotides may be used. Examples of modified nucleotides that may be used to generate the antisense nucleic acid sequences are well known in the art. Known nucleotide modifications include methylation, cyclization and 'caps' and substitution of one or more of the naturally occurring nucleotides with an analogue such as inosine. Other modifications of nucleotides are well known in the art.

The antisense nucleic acid sequence can be produced biologically using an expression vector into which a nucleic acid sequence has been subcloned in an antisense orientation (i.e., RNA transcribed from the inserted nucleic acid will be of an antisense orientation to a target nucleic acid of interest). Preferably, production of antisense nucleic acid sequences in plants occurs by means of a stably integrated nucleic acid construct comprising a promoter, an operably linked antisense oligonucleotide, and a terminator.

The nucleic acid molecules used for silencing in the methods of the invention (whether introduced into a plant or generated in situ) hybridize with or bind to mRNA transcripts and/or genomic DNA encoding a polypeptide to thereby inhibit expression of the protein, e.g., by inhibiting transcription and/or translation. The hybridization can be by conventional nucleotide complementarity to form a stable duplex, or, for example, in the case of an antisense nucleic acid sequence which binds to DNA duplexes, through specific interactions in the major groove of the double helix. Antisense nucleic acid sequences may be introduced into a plant by transformation or direct injection at a specific tissue site. Alternatively, antisense nucleic acid sequences can be modified to target selected cells and then administered systemically. For example, for systemic administration, antisense nucleic acid sequences can be modified such that they specifically bind to receptors or antigens expressed on a selected cell surface, e.g., by linking the antisense nucleic acid sequence to peptides or antibodies which bind to cell surface receptors or antigens. The antisense nucleic acid sequences can also be delivered to cells using the vectors described herein.

According to a further aspect, the antisense nucleic acid sequence is an $\alpha$-anomeric nucleic acid sequence. An $\alpha$-anomeric nucleic acid sequence forms specific double-stranded hybrids with complementary RNA in which, contrary to the usual b-units, the strands run parallel to each other (Gaultier et al. (1987) Nucl Ac Res 15: 6625-6641). The antisense nucleic acid sequence may also comprise a 2'-o-methylribonucleotide (Inoue et al. (1987) Nucl Ac Res 15, 6131-6148) or a chimeric RNA-DNA analogue (Inoue et al. (1987) FEBS Lett. 215, 327-330).

The reduction or substantial elimination of endogenous gene expression may also be performed using ribozymes. Ribozymes are catalytic RNA molecules with ribonuclease activity that are capable of cleaving a single-stranded nucleic acid sequence, such as an mRNA, to which they have a complementary region. Thus, ribozymes (e.g., hammerhead ribozymes (described in Haselhoff and Gerlach (1988) Nature 334, 585-591) can be used to catalytically cleave mRNA transcripts encoding a polypeptide, thereby substantially reducing the number of mRNA transcripts to be translated into a polypeptide. A ribozyme having specificity for a nucleic acid sequence can be designed (see for example: Cech et al. U.S. Patent No. 4,987,071; and Cech et al. U.S. Patent No. 5,116,742). Alternatively, mRNA transcripts corresponding to a nucleic acid sequence can be used to select a catalytic RNA having a specific ribonuclease activity from a pool of RNA molecules (Bartel and Szostak (1993) Science 261, 1411-1418). The use of ribozymes for gene silencing in plants is known in the art (e.g., Atkins et al. (1994) WO 94/00012; Lenne et al. (1995) WO 95/03404; Lutziger et al. (2000) WO 00/00619; Prinsen et al. (1997) WO 97/13865 and Scott et al. (1997) WO 97/38116).

Gene silencing may also be achieved by insertion mutagenesis (for example, T-DNA insertion or transposon insertion) or by strategies as described by, among others, Angell and Baulcombe ((1999) Plant J 20(3): 357-62), (Amplicon VIGS WO 98/36083), or Baulcombe (WO 99/15682).

Gene silencing may also occur if there is a mutation on an endogenous gene and/or a mutation on an isolated gene/nucleic acid subsequently introduced into a plant. The reduction or substantial elimination may be caused by a non-functional polypeptide. For example, the polypeptide may bind to various interacting proteins; one or more mutation(s) and/or truncation(s) may therefore provide for a polypeptide that is still able to bind interacting proteins (such as receptor proteins) but that cannot exhibit its normal function (such as signalling ligand).

A further approach to gene silencing is by targeting nucleic acid sequences complementary to the regulatory region of the gene (e.g., the promoter and/or enhancers) to form triple helical structures that prevent transcription of the gene in target cells. See Helene, C., Anticancer Drug Res. 6, 569-84, 1991; Helene et al., Ann. N.Y. Acad. Sci. 660, 27-36 1992; and Maher, L.J. Bioassays 14, 807-15, 1992.

Other methods, such as the use of antibodies directed to an endogenous polypeptide for inhibiting its function in planta, or interference in the signalling pathway in which a polypeptide is involved, will be well known to the skilled man. In particular, it can be envisaged that manmade molecules may be useful for inhibiting the biological function of a target polypeptide, or for interfering with the signalling pathway in which the target polypeptide is involved.

Alternatively, a screening program may be set up to identify in a plant population natural variants of a gene, which variants encode polypeptides with reduced activity. Such natural variants may also be used for example, to perform homologous recombination.

Artificial and/or natural microRNAs (miRNAs) may be used to knock out gene expression and/or mRNA translation. Endogenous miRNAs are single stranded small RNAs of typically 19-24 nucleotides long. They function primarily to regulate gene expression and/ or mRNA translation. Most plant microRNAs (miRNAs) have perfect or near-perfect complementarity with their target sequences. However, there are natural targets with up to five mismatches. They are processed from longer non-coding RNAs with characteristic fold-back structures by double-strand specific RNases of the Dicer family. Upon processing, they are incorporated in the RNA-induced silencing complex (RISC) by binding to its main component, an Argonaute protein. MiRNAs serve as the specificity components of RISC, since they base-pair to target nucleic acids, mostly mRNAs, in the cytoplasm. Subsequent regulatory events include target mRNA cleavage and destruction and/or translational inhibition. Effects of miRNA overexpression are thus often reflected in decreased mRNA levels of target genes.

Artificial microRNAs (amiRNAs), which are typically 21 nucleotides in length, can be genetically engineered specifically to negatively regulate gene expression of single or multiple genes of interest. Determinants of plant microRNA target selection are well known in the art. Empirical parameters for target recognition have been defined and can be used to aid in the design of specific amiRNAs, (Schwab et al., Dev. Cell 8, 517-527, 2005). Convenient tools for design and generation of amiRNAs and their precursors are also available to the public (Schwab et al., Plant Cell 18, 1121-1133, 2006).

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid to be introduced.

Described above are examples of various methods for the reduction or substantial elimination of expression in a plant of an endogenous gene. A person skilled in the art would readily be able to adapt the aforementioned methods for silencing so as to achieve reduction of expression of an endogenous gene in a whole plant or in parts thereof through the use of an appropriate promoter, for example.

Transformation

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art. Alternatively, a plant cell that cannot be regenerated into a plant may be chosen as host cell, i.e. the resulting transformed plant cell does not have the capacity to regenerate into a (whole) plant.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via *Agrobacterium*-mediated transformation. An advantageous transformation method is the transformation *in planta.* To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for *Agrobacterium*-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth. Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming *Agrobacterium tumefaciens,* for example pBin19 (Bevan et al., Nucl. Acids Res. 12 (1984) 8711). Agrobacteria transformed by such a vector can then be used in known manner for the transformation of plants, such as plants used as a model, like *Arabidopsis (Arabidopsis thaliana* is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of *Agrobacterium tumefaciens* is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of *Arabidopsis* are treated with agrobacteria and seeds are obtained from the developing plants of which a certain proportion is transformed and thus transgenic [Feldman, KA and Marks MD (1987). Mol Gen Genet 208:1-9; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of *Arabidopsis,* intact plants under reduced pressure are treated with an agrobacterial suspension [Bechthold, N (1993). C R Acad Sci Paris Life Sci, 316: 1194-1199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

The genetically modified plant cells can be regenerated via all methods with which the skilled worker is familiar. Suitable methods can be found in the abovementioned publications by S.D. Kung and R. Wu, Potrykus or Höfgen and Willmitzer. Alternatively, the genetically modified plant cells are non-regenerable into a whole plant.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

### T-DNA activation tagging

"T-DNA activation" tagging (Hayashi et al. Science (1992) 1350-1353), involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to modified expression of genes near the inserted T-DNA. The resulting transgenic plants show dominant phenotypes due to modified expression of genes close to the introduced promoter.

### TILLING

The term "TILLING" is an abbreviation of "Targeted Induced Local Lesions In Genomes" and refers to a mutagenesis technology useful to generate and/or identify nucleic acids encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

Homologous recombination

"Homologous recombination" allows introduction in a genome of a selected nucleic acid at a defined selected position. Homologous recombination is a standard technology used routinely in biological sciences for lower organisms such as yeast or the moss *Physcomitrella.* Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; Iida and Terada (2004) Curr Opin Biotech 15(2): 132-8), and approaches exist that are generally applicable regardless of the target organism (Miller et al, Nature Biotechnol. 25, 778-785, 2007).

Yield related Trait(s)

A "Yield related trait" is a trait or feature which is related to plant yield. Yield-related traits may comprise one or more of the following non-limitative list of features: early flowering time, yield, biomass, seed yield, early vigour, greenness index, growth rate, agronomic traits, such as e.g. tolerance to submergence (which leads to yield in rice), Water Use Efficiency (WUE), Nitrogen Use Efficiency (NUE), etc.

Reference herein to enhanced yield-related traits, relative to of control plants is taken to mean one or more of an increase in early vigour and/or in biomass (weight) of one or more parts of a plant, which may include (i) aboveground parts and preferably aboveground harvestable parts and/or (ii) parts below ground and preferably harvestable below ground. In particular, such harvestable parts are seeds.

Yield

The term "yield" in general means a measurable produce of economic value, typically related to a specified crop, to an area, and to a period of time. Individual plant parts directly contribute to yield based on their number, size and/or weight, or the actual yield is the yield per square meter for a crop and year, which is determined by dividing total production (includes both harvested and appraised production) by planted square meters.

The terms "yield" of a plant and "plant yield" are used interchangeably herein and are meant to refer to vegetative biomass such as root and/or shoot biomass, to reproductive organs, and/or to propagules such as seeds of that plant.

Flowers in maize are unisexual; male inflorescences (tassels) originate from the apical stem and female inflorescences (ears) arise from axillary bud apices. The female inflorescence produces pairs of spikelets on the surface of a central axis (cob). Each of the female spikelets encloses two fertile florets, one of them will usually mature into a maize kernel once fertilized. Hence a yield increase in maize may be manifested as one or more of the following: increase in the number of plants established per square meter, an increase in the number of ears per plant, an increase in the number of rows, number of kernels per row, kernel weight, thousand kernel weight, ear length/diameter, increase in the seed filling rate, which is the number of filled florets (i.e. florets containing seed) divided by the total number of florets and multiplied by 100), among others.

Inflorescences in rice plants are named panicles. The panicle bears spikelets, which are the basic units of the panicles, and which consist of a pedicel and a floret. The floret is borne on the pedicel and includes a flower that is covered by two protective glumes: a larger glume (the lemma) and a shorter glume (the palea). Hence, taking rice as an example, a yield increase may manifest itself as an increase in one or more of the following: number of plants per square meter, number of panicles per plant, panicle length, number of spikelets per panicle, number of flowers (or florets) per panicle; an increase in the seed filling rate which is the number of filled florets (i.e. florets containing seeds) divided by the total number of florets and multiplied by 100; an increase in thousand kernel weight, among others.

Early flowering time

Plants having an "early flowering time" as used herein are plants which start to flower earlier than control plants. Hence this term refers to plants that show an earlier start of flowering. Flowering time of plants can be assessed by counting the number of days ("time to flower") between sowing and the emergence of a first inflorescence. The "flowering time" of a plant can for instance be determined using the method as described in WO 2007/093444.

Early vigour

"Early vigour" refers to active healthy well-balanced growth especially during early stages of plant growth, and may result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

Increased growth rate

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a mature seed up to the stage where the plant has produced mature seeds, similar to the starting material. This life cycle may be influenced by factors such as speed of germination, early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per square meter (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

Stress resistance

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35%, 30% or 25%, more preferably less than 20% or 15% in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Abiotic stresses may be due to drought or excess water, anaerobic stress, salt stress, chemical toxicity, oxidative stress and hot, cold or freezing temperatures.
"Biotic stresses" are typically those stresses caused by pathogens, such as bacteria, viruses, fungi, nematodes and insects.
The "abiotic stress" may be an osmotic stress caused by a water stress, e.g. due to drought, salt stress, or freezing stress. Abiotic stress may also be an oxidative stress or a cold stress. "Freezing stress" is intended to refer to stress due to freezing temperatures, i.e. temperatures at which available water molecules freeze and turn into ice. "Cold stress", also called "chilling stress", is intended to refer to cold temperatures, e.g. temperatures below 10°, or preferably below 5°C, but at which water molecules do not freeze. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location. Plants with optimal growth conditions, (grown under non-stress conditions) typically yield in increasing order of preference at least 97%, 95%, 92%, 90%, 87%, 85%, 83%, 80%, 77% or 75% of the average production of such plant in a given environment. Average production may be calculated on harvest and/or season basis. Persons skilled in the art are aware of average yield productions of a crop.

In particular, the methods of the present invention may be performed under non-stress conditions. In an example, the methods of the present invention may be performed under non-stress conditions such as mild drought to give plants having increased yield relative to control plants.

In another embodiment, the methods of the present invention may be performed under stress conditions.

In an example, the methods of the present invention may be performed under stress conditions such as drought to give plants having increased yield relative to control plants.

In another example, the methods of the present invention may be performed under stress conditions such as nutrient deficiency to give plants having increased yield relative to control plants.

Nutrient deficiency may result from a lack of nutrients such as nitrogen, phosphates and other phosphorous-containing compounds, potassium, calcium, magnesium, manganese, iron and boron, amongst others.

In yet another example, the methods of the present invention may be performed under stress conditions such as salt stress to give plants having increased yield relative to control plants. The term salt stress is not restricted to common salt (NaCl), but may be any one or more of: NaCl, KCl, LiCl, $MgCl_2$, $CaCl_2$, amongst others.

In yet another example, the methods of the present invention may be performed under stress conditions such as cold stress or freezing stress to give plants having increased yield relative to control plants.

Increase/Improve/Enhance

The terms "increase", "improve" or "enhance" are interchangeable and shall mean in the sense of the application at least a 3%, 4%, 5%, 6%, 7%, 8%, 9% or 10%, preferably at least 15% or 20%, more preferably 25%, 30%, 35% or 40% more yield and/or growth in comparison to control plants as defined herein.

Seed yield

Increased seed yield may manifest itself as one or more of the following:

a) an increase in seed biomass (total seed weight) which may be on an individual seed basis and/or per plant and/or per square meter;
b) increased number of flowers per plant;
c) increased number of seeds;
d) increased seed filling rate (which is expressed as the ratio between the number of filled florets divided by the total number of florets);
e) increased harvest index, which is expressed as a ratio of the yield of harvestable parts, such as seeds, divided by the biomass of aboveground plant parts; and
f) increased thousand kernel weight (TKW), which is extrapolated from the number of seeds counted and their total weight. An increased TKW may result from an increased seed size and/or seed weight, and may also result from an increase in embryo and/or endosperm size.

The terms "filled florets" and "filled seeds" may be considered synonyms.

An increase in seed yield may also be manifested as an increase in seed size and/or seed volume. Furthermore, an increase in seed yield may also manifest itself as an increase in seed area and/or seed length and/or seed width and/or seed perimeter.

Greenness Index

The "greenness index" as used herein is calculated from digital images of plants. For each pixel belonging to the plant object on the image, the ratio of the green value versus the red value (in the RGB model for encoding color) is calculated. The greenness index is expressed as the percentage of pixels for which the green-to-red ratio exceeds a given threshold. Under normal growth conditions, under salt stress growth conditions, and under reduced nutrient availability growth conditions, the greenness index of plants is measured in the last imaging before flowering. In contrast, under drought stress growth conditions, the greenness index of plants is measured in the first imaging after drought.

Biomass

The term "biomass" as used herein is intended to refer to the total weight of a plant. Within the definition of biomass, a distinction may be made between the biomass of one or more parts of a plant, which may include any one or more of the following:

- aboveground parts such as but not limited to shoot biomass, seed biomass, leaf biomass, etc.;
- aboveground harvestable parts such as but not limited to shoot biomass, seed biomass, leaf biomass, etc.;
- parts below ground, such as but not limited to root biomass, tubers, bulbs, etc.;
- harvestable parts below ground, such as but not limited to root biomass, tubers, bulbs, etc.;
- harvestable parts partially below ground such as but not limited to beets and other hypocotyl areas of a plant, rhizomes, stolons or creeping rootstalks;
- vegetative biomass such as root biomass, shoot biomass, etc.;
- reproductive organs; and
- propagules such as seed.

Marker assisted breeding

Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Use as probes in (gene mapping)

Use of nucleic acids encoding the protein of interest for genetically and physically mapping the genes requires only a nucleic acid sequence of at least 15 nucleotides in length. These nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the nucleic acids encoding the protein of interest. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the nucleic acid encoding the protein of interest in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

Plant

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acer* spp., *Actinidia* spp., *Abelmoschus* spp., *Agave sisalana*, *Agropyron* spp., *Agrostis stolonifera*, *Allium* spp., *Amaranthus* spp., *Ammophila arenaria*, *Ananas comosus*, *Annona* spp., *Apium graveolens*, *Arachis* spp, *Artocarpus* spp., *Asparagus officinalis*, *Avena* spp. *(e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica* spp. *(e.g. Brassica napus, Brassica rapa* ssp. [canola, oilseed rape, turnip rape]), *Cadaba farinosa, Camellia sinensis, Canna indica, Cannabis sativa, Capsicum* spp., *Carex elata, Carica papaya, Carissa macrocarpa, Carya* spp., *Carthamus tinctorius, Castanea* spp., *Ceiba pentandra, Cichorium endivia, Cinnamomum* spp., *Citrullus lanatus, Citrus* spp., *Cocos* spp., *Coffea* spp., *Colocasia esculenta, Cola* spp., *Corchorus sp., Coriandrum sativum, Corylus* spp., *Crataegus* spp., *Crocus sativus, Cucurbita* spp., *Cucumis* spp., *Cynara* spp., *Daucus carota, Desmodium* spp., *Dimocarpus longan, Dioscorea* spp., *Diospyros* spp., *Echinochloa* spp., *Elaeis (e.g. Elaeis guineensis, Elaeis oleifera), Eleusine coracana, Eragrostis tef, Erianthus sp., Eriobotrya japonica, Eucalyptus sp., Eugenia uniflora, Fagopyrum* spp., *Fagus* spp., *Festuca arundinacea, Ficus carica, Fortunella* spp., *Fragaria* spp., *Ginkgo biloba, Glycine* spp. *(e.g. Glycine max, Soja hispida or Soja max), Gossypium hirsutum, Helianthus* spp. *(e.g. Helianthus annuus), Hemerocallis fulva, Hibiscus* spp., *Hordeum* spp. *(e.g. Hordeum vulgare), Ipomoea batatas, Juglans* spp., *Lactuca sativa, Lathyrus* spp., *Lens culinaris, Linum usitatissimum, Litchi chinensis, Lotus* spp., *Luffa acutangula, Lupinus* spp., *Luzula sylvatica, Lycopersicon* spp. *(e.g. Lycopersicon esculentum, Lycopersicon lycopersicum, Lycopersicon pyriforme), Macrotyloma* spp., *Malus* spp., *Malpighia emarginata, Mammea americana, Mangifera indica, Manihot* spp., *Manilkara zapota, Medicago sativa, Melilotus* spp., *Mentha* spp., *Miscanthus sinensis, Momordica* spp., *Morus nigra, Musa* spp., *Nicotiana* spp., *Olea* spp., *Opuntia* spp., *Ornithopus* spp., *Oryza* spp. *(e.g. Oryza sativa, Oryza latifolia), Panicum miliaceum, Panicum virgatum, Passiflora edulis, Pastinaca sativa, Pennisetum sp., Persea* spp., *Petroselinum crispum, Phalaris arundinacea, Phaseolus* spp., *Phleum pratense, Phoenix* spp., *Phragmites australis, Physalis* spp., *Pinus* spp., *Pistacia vera, Pisum* spp., *Poa* spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Ricinus communis, Rubus* spp., *Saccharum* spp., *Salix sp., Sambucus* spp., *Secale cereale, Sesamum* spp., *Sinapis sp., Solanum* spp. *(e.g. Solanum tuberosum, Solanum integrifolium or Solanum lycopersicum), Sorghum bicolor, Spinacia* spp., *Syzygium* spp., *Tagetes* spp., *Tamarindus indica, Theobroma cacao, Trifolium* spp., *Tripsacum dactyloides, Triticosecale rimpaui, Triticum* spp. *(e.g. Triticum aestivum, Triticum durum, Triticum turgidum, Triticum hybernum, Triticum macha, Triticum sativum, Triticum monococcum or Triticum vulgare), Tropaeolum minus, Tropaeolum majus, Vaccinium* spp., *Vicia* spp., *Vigna* spp., *Viola odorata, Vitis* spp., *Zea mays, Zizania palustris, Ziziphus* spp., amongst others.

Control plant(s)

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes (or null control plants) are individuals missing the transgene by segregation. Further, control plants are grown under equal growing conditions to the growing conditions of the plants of the invention, i.e. in the vicinity of, and simultaneously with, the plants of the invention. A "control plant" as used herein refers not only to whole plants, but also to plant parts, including seeds and seed parts.

**Description of figures**

The present invention will now be described with reference to the following figures in which:

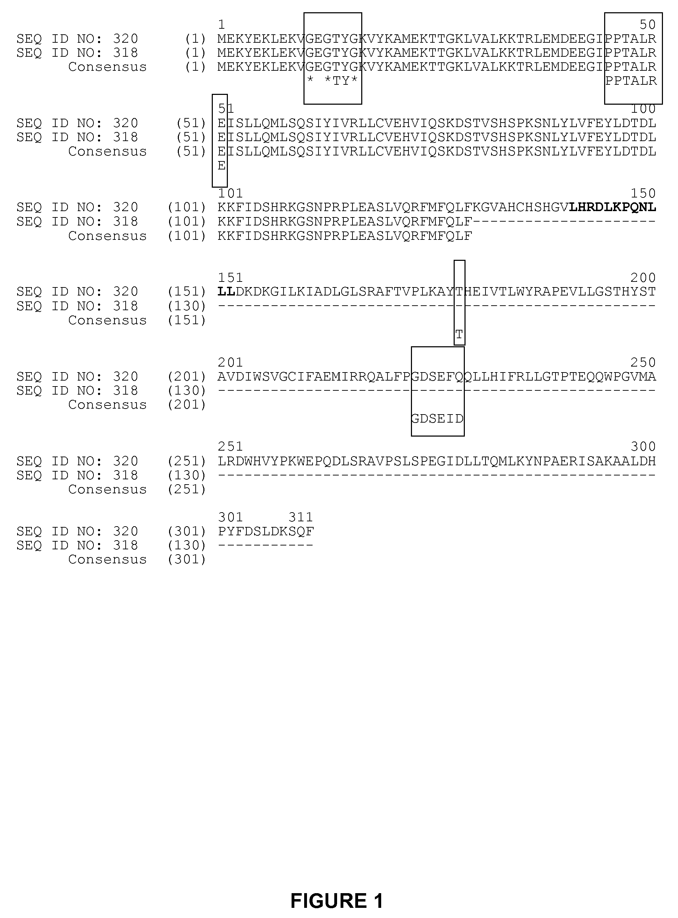

**Figure 1** represents an alignment of a CDKB-RKA polypeptide represented by SEQ ID NO: 318 with a CDKB polypeptide (CDKB1,2) from *Arabidospsis thaliana* (AT2G38620.2; SEQ ID NO: 320) with indication of conserved domains.

**Figure 2** represents a multiple alignment of various full length CDKB polypeptides. The asterisks indicate identical amino acids among the various protein sequences, colons represent highly conserved amino acid substitutions, and the dots represent less conserved amino acid substitution; on other positions there is no sequence conservation. These alignments can be used for defining further motifs or signature sequences, when using conserved amino acids. A conserved cyclin binding domain is represented with a black box.

**Figure 3** represents the binary vector used for increased expression in *Oryza sativa* of a CDKB-RKA-encoding nucleic acid as defined herein under the control of a suitable promoter.

## Examples

The present invention will now be described with reference to the following examples, which are by way of illustration only. The following examples are not intended to limit the scope of the invention. Unless otherwise indicated, the present invention employs conventional techniques and methods of plant biology, molecular biology, bioinformatics and plant breedings.

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

Example 1: Identification of sequences related to the nucleic acid sequence used in the methods of intervention

The nucleic acid molecule represented by SEQ ID NO: 319 and encoding a B-type CDK (CDKB) polypeptide represented by SEQ ID NO: 320 is an example of a full length CDKB polypeptide that has catalytic kinase activity.

Starting from this sequence, other sequences (full length cDNA, ESTs or genomic) related to SEQ ID NO: 319 and SEQ ID NO: 320 were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid of SEQ ID NO: 319 was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

Table A2 provides a list of nucleic acid sequences related to and including SEQ ID NO: 319 and SEQ ID NO: 320.

**Table A2:** Examples of B-type nucleic acids and polypeptides:

| plant source | Nucleic acid sequence | amino acid sequence |
|---|---|---|
| A.thaliana_AT2G38620.2#1 | 319 | 320 |
| A.thaliana_AT3G54180.1#1 | 321 | 322 |
| A.thaliana_AT2G38620.1#1 | 323 | 324 |
| A.thaliana_AT1G76540.1#1 | 325 | 326 |
| A.thaliana_AT1G20930.1#1 | 327 | 328 |
| G.max_Glyma14g39760.1#1 | 329 | 330 |
| G.max_Glyma07g02400.1#1 | 331 | 332 |
| G.max_Glyma07g07640.1#1 | 333 | 334 |

(continued)

| plant source | Nucleic acid sequence | amino acid sequence |
|---|---|---|
| G.max_Glyma09g08250.1#1 | 335 | 336 |
| G.max_Glyma17g38210.1#1 | 337 | 338 |
| H.annuus_CD855729#1 | 339 | 340 |
| H.vulgare_TC160457#1 | 341 | 342 |
| H.vulgare_TC172640#1 | 343 | 344 |
| M.truncatula_TC115190#1 | 345 | 346 |
| M.truncatula_TC117212#1 | 347 | 348 |
| O.sativa_LOC_Os01g67160.1#1 | 349 | 350 |
| O.sativa_LOC_Os08g40170.1#1 | 351 | 352 |
| P.patens_TC41028#1 | 353 | 354 |
| P.patens_NP13120379#1 | 355 | 356 |
| P.patens_NP13127560#1 | 357 | 358 |
| P.patens_TC43373#1 | 359 | 360 |
| P.patens_TC35664#1 | 361 | 362 |
| P.patens_TC47562#1 | 363 | 364 |
| P.trichocarpa_scaff_28.331#1 | 365 | 366 |
| P.trichocarpa_scaff_XVI.1376#1 | 367 | 368 |
| P.trichocarpa_scaff_II.31#1 | 369 | 370 |
| P.trichocarpa_scaff_3712.1#1 | 371 | 372 |
| S.lycopersicum_TC198540#1 | 373 | 374 |
| S.lycopersicum_TC205060#1 | 375 | 376 |
| T.aestivum_TC283074#1 | 377 | 378 |
| T.aestivum_TC290551#1 | 379 | 380 |
| T.aestivum_TC282781#1 | 381 | 382 |
| Z.mays_c62195783gm030403@12303#1 | 383 | 384 |
| M.sativa_P93321 | 385 | 386 |
| R.communis_XP_002519974.1 | 387 | 388 |
| N.tobacco_AAG01532.1 | 389 | 390 |
| S.esculentum_CAC15503.1 | 391 | 392 |
| Lotus japonicus | 393 | 394 |
| V.vitis_XP002266623.1 | 395 | 396 |
| Anthirinum_CAA66235.1 | 397 | 398 |
| C.rubrum_CAC17703.1 | 399 | 400 |
| S.bicolor_XP_002441781.1 | 401 | 402 |

Polypeptides related to SEQ ID NO: 320, such as those represented in Table A2 above, may be used in the present invention, provided that the polypeptides sequences have reduced or preferably lack catalytic kinase activity. Hence, starting from sequences such as those listed in Table A2, CDKB-RKA polypeptides according to the invention, can be designed, for instance by introducing one or more mutations in said polypeptides, and preferably in the catalytic kinase domain of said polypeptides, or by partially or substantially completely truncating the catalytic kinase domain from the CDKB polypeptide.

Sequences have been tentatively assembled and publicly disclosed by research institutions, such as The Institute for Genomic Research (TIGR; beginning with TA). For instance, the Eukaryotic Gene Orthologs (EGO) database may be used to identify such related sequences, either by keyword search or by using the BLAST algorithm with the nucleic acid sequence or polypeptide sequence of interest. Special nucleic acid sequence databases have been created for particular organisms, e.g. for certain prokaryotic organisms, such as by the Joint Genome Institute. Furthermore, access to proprietary databases, has allowed the identification of novel nucleic acid and polypeptide sequences.

Example 2: Alignment of sequences to the plypeptide sequences used in the methods of the invention

Alignment of CDKB polypeptide sequences as represented in Table A2 was performed using the ClustalW 1.81 algorithm of progressive alignment (Thompson et al. (1997) Nucleic Acids Res 25:4876-4882; Chenna et al. (2003). Nucleic Acids Res 31:3497-3500) with standard setting (slow alignment, similarity matrix: Gonnet, gap opening penalty 10, gap extension penalty: 0.2). Minor manual editing was done to further optimise the alignment. B-type CDK (CDKB) polypeptides are aligned in Figure 2.

Example 3: Calculation of global percentage identity between polypeptide sequences

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pairwise alignments using the Myers and Miller global alignment algorithm (with a gap opening penalty of 12, and a gap extension penalty of 2), calculates similarity and identity using for example Blosum 62 (for polypeptides), and then places the results in a distance matrix.

Results of the analysis show the global similarity and identity over the full length of the polypeptide sequences. Sequence similarity is shown in the bottom half of the dividing line and sequence identity is shown in the top half of the diagonal dividing line. Parameters used in the comparison are: Scoring matrix: Blosum62, First Gap: 12, Extending Gap: 2.

A MATGAT table for local alignment of a specific domain, or data on % identity/similarity between specific domains can also be made.

Example 4: Identification of domains comprised in polypeptide sequences useful in performing the methods of the invention

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Pfam is a large collection of multiple sequence alignments and hidden Markov models covering many common protein domains and families. Pfam is hosted at the Sanger Institute server in the United Kingdom. Interpro is hosted at the European Bioinformatics Institute in the United Kingdom.

The results of the InterPro scan (InterPro database, release 31.0) of the polypeptide sequence as represented by SEQ ID NO: 320 are presented in Table C1.

Table C1: InterPro scan results (major accession numbers) of full length polypeptide sequence as represented by SEQ ID NO: 320.

| Database/ Method | Accession number | Accession name | Name | Amino acid coordinates on SEQ ID NO 320 |
|---|---|---|---|---|
| PROFILE | PS50011 | IPR000719 | Protein kinase catalytic domain | 4-303 |
| SMART | SM00220 | IPR002290 | Serine/threonine- protein kinase domain | 4-303 |
| PROSITE | PS00108 | IPR008271 | Serine/threonine- protein kinase active site | 140-152 |

(continued)

| Database/ Method | Accession number | Accession name | Name | Amino acid coordinates on SEQ ID NO 320 |
|---|---|---|---|---|
| PROSITE | PS00107 | IPR017441 | Protein kinase, ATP binding site | 10-33 |
| PFAM | PF00069 | IPR017442 | Serine/threonine-protein kinase domain | 4-303 |
| SMART | SM00219 | IPR020635 | Tyrosine-protein kinase, catalytic domain | 4-303 |

Example 5: Topology prediction of the polypeptide sequences useful in performing the methods of invention

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark.
For the sequences predicted to contain an N-terminal presequence a potential cleavage site can also be predicted.
A number of parameters are selected, such as organism group (non-plant or plant), cutoff sets (none, predefined set of cutoffs, or user-specified set of cutoffs), and the calculation of prediction of cleavage sites (yes or no).
Many other algorithms can be used to perform such analyses, including:

- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark
- PSORT (URL: psort.org)
- PLOC (Park and Kanehisa, Bioinformatics, 19, 1656-1663, 2003).

Example 6: Functional assay related to the polypeptide sequences useful in performing the methods of the invention

In accordance with the present invention, a CDKB-RKA polypeptide is provided for use in methods according to the invention, wherein said polypeptide has reduced, or preferably lacks catalytic kinase activity as compared to a (unmodified or full length) CDKB polypeptide. There are many ways to measure kinase activity, well known to a person skilled in the art.
In one example, catalytic kinase activity of a mutated or truncated CDBK polypeptide as defined herein can be determined by means of a kinase assay and compared to the kinase activity of a corresponding unmodified CDKB polypeptide.
A kinase assay may, for example, be performed as described in Cockcroft et al., 2000 (Nature, 405 (6786), 575-579). The assay involves grinding plant material in liquid nitrogen and resuspending in an appropriate buffer (such as 1ml of 50mM Tris-HCl pH 7.5, 75mM NaCl, 15mM EGTA, 15mMMgCl2, 1 mM dithiothreitol, 0.1% Tween 20, 1X complete Tm protease inhibitor, 1 mMNaF, 0.2mM NaV, 2mM Na-pyrophosphate, 60mM beta-glycerophosphate). The suspension is then homogenized for 4 x 30sec with 30 sec on ice between homogeneizations. The supernatant is then incubated with 20 microliters of protein A Sepharose (50% suspension) for 30 min at 4°C. The supernatant is incubated with 1 microliter of antiserum (directed against the carboxy terminal peptide of a CDC2b protein, as described in Setiady et al., 1996 (Plant Cell Physiology 37 (3), 369-376) for 2h on ice, then 20 microliters protein A-Sepharose added and the sample rotated at 4°C for an hour. Samples are washed 2 times with kinase buffer (50mM Tris-HCl pH7.5, 100mM NaCl, 5mM EGTA, 1mM DTT), resuspended in 15 microliters assay buffer (50mM Tris-HCl pH7.5, 100mM NaCl, 5mM EGTA, 10mM MgCl2, 1mM DTT, 1mM NaF, 0.2 mM Na-Vanadate, 2 mM Na pyrophosphate, 25mM beta glycerophosphate, 0.5 mM histone H1 as a substrate, 0.5mM PMSF, and 74 kBq [gamma $^{32}$P ATP (>185 TBq mmol-1) per 15 microliters of reaction) and incubated at room temperature for 30 min. The reaction may be stopped by adding gel loading buffer and samples may be analysed using SDS-PAGE and quantified using a phosphoimager (Molecular Dynamics).

Example 7: Cloning of the nucleic acid sequence used in methods of the invention

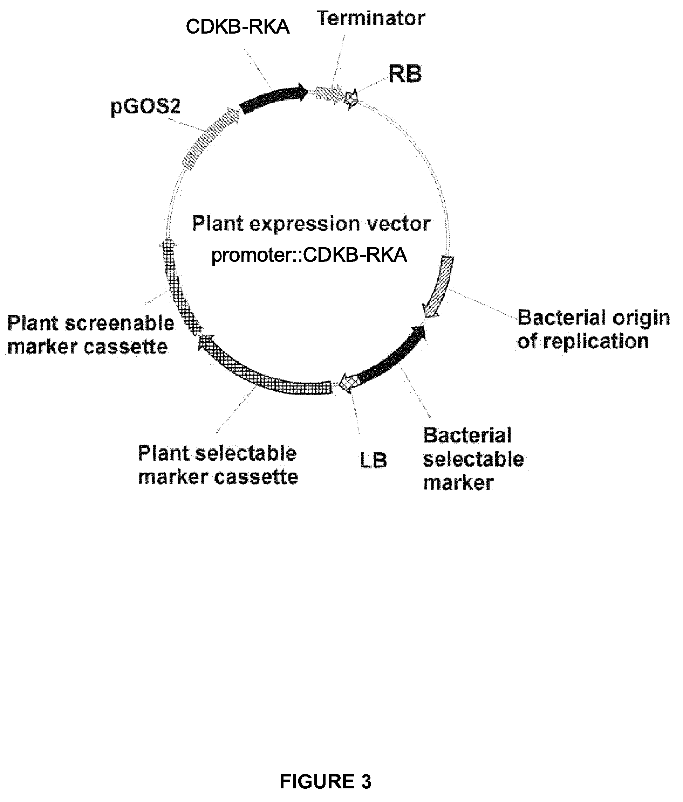

An entry clone comprising SEQ ID NO: 317 was used in an LR reaction with a destination vector used for *Oryza sativa* transformation. This vector contained as functional elements within the T-DNA borders: a plant selectable marker; a screenable marker expression cassette; and a Gateway cassette intended for LR in vivo recombination with the nucleic acid sequence of interest already cloned in the entry clone. A rice High Mobility Group Protein (HMGP) promoter (SEQ ID NO: 405) for constitutive expression was located upstream of this Gateway cassette. After the LR recombination step, the resulting expression vector promoter::CDKB-RKA (Figure 3) was transformed into Agrobacterium strain LBA4044 according to methods well known in the art.

The nucleic acid sequence represented by SEQ ID NO: 317 can be readily obtained by applying common PCR technology, using as template a custom-made *Arabidopsis thaliana* seedlings cDNA library, e.g. *Arabidopsis* ecotype An-1. PCR can be performed using a commercially available proofreading Taq DNA polymerase in standard conditions, using 200 ng of template in a 50 $\mu$l PCR mix.

The CDKB-RKA polypeptide represented by SEQ ID NO: 318, and encoded by SEQ ID NO: 317, is obtained by a nucleotide substitution at position 388 of SEQ ID NO: 320, where an A was replaced by a T (and thus a Lys by a stop codon). Based on this information, a skilled person can design a forward and a reverse primer for use in the PCR mix. The above-indicated point mutation can be included anywhere in the reverse primer, which is designed to have a similar melting temperature as the forward primer. More than one substitution can be made, for example to create another stop codon than TAA (TAG,TGA). Both the forward and the reverse primer include the AttB sites for Gateway recombination. The amplified PCR fragment can be purified also using standard methods. The first step of the Gateway procedure, the BP reaction, is then performed, during which the PCR fragment recombined in vitro with the pDONR201 plasmid to produce, according to the Gateway terminology, an "entry clone", pCDKB-RKA. Plasmid pDONR201 can be purchased from Invitrogen, as part of the Gateway® technology.

Example 8: Plant transformation

*Rice transformation*

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 to 60 minutes, preferably 30 minutes in sodium hypochlorite solution (depending on the grade of contamination), followed by a 3 to 6 times, preferably 4 time wash with sterile distilled water. The sterile seeds were then germinated on a medium containing 2,4-D (callus induction medium). After incubation in light for 6 days scutellum-derived calli is transformed with Agrobacterium as described herein below.

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density ($OD_{600}$) of about 1. The calli were immersed in the suspension for 1 to 15 minutes. The callus tissues were then blotted dry on a filter paper and transferred to solidified, co-cultivation medium and incubated for 3 days in the dark at 25°C. After washing away the *Agrobacterium,* the calli were grown on 2,4-D-containing medium for 10 to 14 days (growth time for indica: 3 weeks) under light at 28°C - 32°C in the presence of a selection agent. During this period, rapidly growing resistant callus developed. After transfer of this material to regeneration media, the embryogenic potential was released and shoots developed in the next four to six weeks. Shoots were excised from the calli and incubated for 2 to 3 weeks on an auxin-containing medium from which they were transferred to soil. Hardened shoots were grown under high humidity and short days in a greenhouse.

Transformation of rice cultivar indica can also be done in a similar way as give above according to techniques well known to a skilled person.

35 to 90 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et al. 1993, Hiei et al. 1994).

Example 9: Transformation of other crops

*Corn transformation*

Transformation of maize (*Zea mays*) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseedlcanola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa transformation*

A regenerating clone of alfalfa (*Medicago sativa*) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 μm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

*Cotton transformation*

Cotton is transformed using *Agrobacterium tumefaciens* according to the method described in US 5,159,135. Cotton seeds are surface sterilised in 3% sodium hypochlorite solution during 20 minutes and washed in distilled water with 500 μ/ml cefotaxime. The seeds are then transferred to SH-medium with 50μ/ml benomyl for germination. Hypocotyls of 4 to 6 days old seedlings are removed, cut into 0.5 cm pieces and are placed on 0.8% agar. An *Agrobacterium* suspension (approx. 108 cells per ml, diluted from an overnight culture transformed with the gene of interest and suitable selection markers) is used for inoculation of the hypocotyl explants. After 3 days at room temperature and lighting, the tissues are transferred to a solid medium (1.6 g/l Gelrite) with Murashige and Skoog salts with B5 vitamins (Gamborg et al., Exp. Cell Res. 50:151-158 (1968)), 0.1 mg/l 2,4-D, 0.1 mg/l 6-furfurylaminopurine and 750 μ/ml MgCL2, and with 50 to 100 μ/ml cefotaxime and 400-500 μ/ml carbenicillin to kill residual bacteria. Individual cell lines are isolated after two to three months (with subcultures every four to six weeks) and are further cultivated on selective medium for tissue amplification (30°C, 16 hr photoperiod). Transformed tissues are subsequently further cultivated on non-selective medium during 2 to 3 months to give rise to somatic embryos. Healthy looking embryos of at least 4 mm length are transferred to tubes with SH medium in fine vermiculite, supplemented with 0.1 mg/l indole acetic acid, 6 furfurylaminopurine and gibberellic acid. The embryos are cultivated at 30°C with a photoperiod of 16 hrs, and plantlets at the 2 to 3 leaf stage are transferred to pots with vermiculite and nutrients. The plants are hardened and subsequently moved to the greenhouse for further cultivation.

*Sugarbeet transformation*

Seeds of sugarbeet (Beta vulgaris L.) are sterilized in 70% ethanol for one minute followed by 20 min. shaking in 20% Hypochlorite bleach e.g. Clorox® regular bleach (commercially available from Clorox, 1221 Broadway, Oakland, CA 94612, USA). Seeds are rinsed with sterile water and air dried followed by plating onto germinating medium (Murashige and Skoog (MS) based medium (Murashige, T., and Skoog, ., 1962. Physiol. Plant, vol. 15, 473-497) including B5 vitamins (Gamborg et al.; Exp. Cell Res., vol. 50, 151-8.) supplemented with 10 g/l sucrose and 0,8% agar). Hypocotyl tissue is used essentially for the initiation of shoot cultures according to Hussey and Hepher (Hussey, G., and Hepher, A., 1978. Annals of Botany, 42, 477-9) and are maintained on MS based medium supplemented with 30g/l sucrose plus 0,25mg/l benzylamino purine and 0,75% agar, pH 5,8 at 23-25°C with a 16-hour photoperiod. Agrobacterium tumefaciens strain carrying a binary plasmid harbouring a selectable marker gene, for example nptII is used in transformation experiments. One day before transformation, a liquid LB culture including antibiotics is grown on a shaker (28°C, 150rpm) until an optical density (O.D.) at 600 nm of ~1 is reached. Overnight-grown bacterial cultures are centrifuged and resuspended in inoculation medium (O.D. ~1) including Acetosyringone, pH 5,5. Shoot base tissue is cut into slices (1.0 cm x 1.0 cm x 2.0 mm approximately). Tissue is immersed for 30s in liquid bacterial inoculation medium. Excess liquid is removed by filter paper blotting. Co-cultivation occurred for 24-72 hours on MS based medium incl. 30g/l sucrose followed by a non-selective period including MS based medium, 30g/l sucrose with 1 mg/l BAP to induce shoot development and cefotaxim for eliminating the Agrobacterium. After 3-10 days explants are transferred to similar selective medium harbouring for example kanamycin or G418 (50-100 mg/l genotype dependent). Tissues are transferred to fresh medium every 2-3 weeks to maintain selection pressure. The very rapid initiation of shoots (after 3-4 days) indicates regeneration of existing meristems rather than organogenesis of newly developed transgenic meristems. Small shoots are transferred after several rounds of subculture to root induction medium containing 5 mg/l NAA and kanamycin or G418. Additional steps are taken to reduce the potential of generating transformed plants that are chimeric (partially transgenic). Tissue samples from regenerated shoots are used for DNA analysis. Other transformation methods for sugarbeet are known in the art, for example those by Linsey & Gallois (Linsey, K., and Gallois, P., 1990. Journal of Experimental Botany; vol. 41, No. 226; 529-36) or the methods published in the international application published as WO9623891A.

*Sugarcane transformation*

Spindles are isolated from 6-month-old field grown sugarcane plants (Arencibia et al., 1998. Transgenic Research, vol. 7, 213-22; Enriquez-Obregon et al., 1998. Planta, vol. 206, 20-27). Material is sterilized by immersion in a 20% Hypochlorite bleach e.g. Clorox® regular bleach (commercially available from Clorox, 1221 Broadway, Oakland, CA 94612, USA) for 20 minutes. Transverse sections around 0,5cm are placed on the medium in the top-up direction. Plant material is cultivated for 4 weeks on MS (Murashige, T., and Skoog, ., 1962. Physiol. Plant, vol. 15, 473-497) based medium incl. B5 vitamins (Gamborg, O., et al., 1968. Exp. Cell Res., vol. 50,151-8) supplemented with 20g/l sucrose, 500 mg/l casein hydrolysate, 0,8% agar and 5mg/l 2,4-D at 23°C in the dark. Cultures are transferred after 4 weeks onto identical fresh medium. Agrobacterium tumefaciens strain carrying a binary plasmid harbouring a selectable marker gene, for example hpt, is used in transformation experiments. One day before transformation, a liquid LB culture including antibiotics is grown on a shaker (28°C, 150rpm) until an optical density (O.D.) at 600 nm of ~0,6 is reached. Overnight-grown bacterial cultures are centrifuged and resuspended in MS based inoculation medium (O.D. ~0,4) including acetosyringone, pH 5,5. Sugarcane embryogenic callus pieces (2-4 mm) are isolated based on morphological characteristics as compact structure and yellow colour and dried for 20 min. in the flow hood followed by immersion in a liquid bacterial inoculation medium for 10-20 minutes. Excess liquid is removed by filter paper blotting. Co-cultivation occurred for 3-5 days in the dark on filter paper which is placed on top of MS based medium incl. B5 vitamins containing 1 mg/l 2,4-D. After co-cultivation calli are washed with sterile water followed by a non-selective cultivation period on similar medium containing 500 mg/l cefotaxime for eliminating remaining Agrobacterium cells. After 3-10 days explants are transferred to MS based selective medium incl. B5 vitamins containing 1 mg/l 2,4-D for another 3 weeks harbouring 25 mg/l of hygromycin (genotype dependent). All treatments are made at 23°C under dark conditions. Resistant calli are further cultivated on medium lacking 2,4-D including 1 mg/l BA and 25 mg/l hygromycin under 16 h light photoperiod resulting in the development of shoot structures. Shoots are isolated and cultivated on selective rooting medium (MS based including, 20g/l sucrose, 20 mg/l hygromycin and 500 mg/l cefotaxime). Tissue samples from regenerated shoots are used for DNA analysis. Other transformation methods for sugarcane are known in the art, for example from the international application published as WO2010/151634A and the granted European patent EP1831378.

Example 10: Phenotypic evaluation procedure

10.1 Evaluation setup

35 to 90 independent T0 rice transformants were generated. The primary transformants were transferred from a tissue culture chamber to a greenhouse for growing and harvest of T1 seed. Six events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%. Plants grown under non-stress conditions were watered at regular intervals to ensure that water and nutrients were not limiting and to satisfy plant needs to complete growth and development, unless they were used in a stress screen.

From the stage of sowing until the stage of maturity the plants were passed several times through a digital imaging cabinet. At each time point digital images (2048x1536 pixels, 16 million colours) were taken of each plant from at least 6 different angles.

T1 events can be further evaluated in the T2 generation following the same evaluation procedure as for the T1 generation, e.g. with less events and/or with more individuals per event.

*Drought screen*

T1 or T2 plants were grown in potting soil under normal conditions until they approached the heading stage. They were then transferred to a "dry" section where irrigation was withheld. Soil moisture probes were inserted in randomly chosen pots to monitor the soil water content (SWC). When SWC went below certain thresholds, the plants were automatically re-watered continuously until a normal level was reached again. The plants were then re-transferred again to normal conditions. The rest of the cultivation (plant maturation, seed harvest) was the same as for plants not grown under abiotic stress conditions. Growth and yield parameters were recorded as detailed for growth under normal conditions.

*Nitrogen use efficiency screen*

T1 or T2 plants were grown in potting soil under normal conditions except for the nutrient solution. The pots were watered from transplantation to maturation with a specific nutrient solution containing reduced N nitrogen (N) content, usually between 7 to 8 times less. The rest of the cultivation (plant maturation, seed harvest) was the same as for plants not grown under abiotic stress. Growth and yield parameters were recorded as detailed for growth under normal conditions.

*Salt stress screen*

T1 or T2 plants are grown on a substrate made of coco fibers and particles of baked clay (Argex) (3 to 1 ratio). A normal nutrient solution is used during the first two weeks after transplanting the plantlets in the greenhouse. After the first two weeks, 25 mM of salt (NaCl) is added to the nutrient solution, until the plants are harvested. Growth and yield parameters are recorded as detailed for growth under normal conditions.

10.2 Statistical analysis: F test

A two factor ANOVA (analysis of variants) was used as a statistical model for the overall evaluation of plant phenotypic characteristics. An F test was carried out on all the parameters measured of all the plants of all the events transformed with the gene of the present invention. The F test was carried out to check for an effect of the gene over all the transformation events and to verify for an overall effect of the gene, also known as a global gene effect. The threshold for significance for a true global gene effect was set at a 5% probability level for the F test. A significant F test value points to a gene effect, meaning that it is not only the mere presence or position of the gene that is causing the differences in phenotype.

10.3 Parameters measured

From the stage of sowing until the stage of maturity the plants were passed several times through a digital imaging cabinet. At each time point digital images (2048x1536 pixels, 16 million colours) were taken of each plant from at least 6 different angles as described in WO 2010/031780. These measurements were used to determine different parameters.

*Biomass-related parameter measurement*

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass.

Increase in root biomass is expressed as an increase in total root biomass (measured as maximum biomass of roots observed during the lifespan of a plant); or as an increase in the root/shoot index, measured as the ratio between root mass and shoot mass in the period of active growth of root and shoot. In other words, the root/shoot index is defined as the ratio of the rapidity of root growth to the rapidity of shoot growth in the period of active growth of root and shoot. Root biomass can be determined using a method as described in WO 2006/029987.

*Parameters related to development time*

The early vigour is the plant aboveground area three weeks post-germination. Early vigour was determined by counting the total number of pixels from aboveground plant parts discriminated from the background. This value was averaged for the pictures taken on the same time point from different angles and was converted to a physical surface value expressed in square mm by calibration.

AreaEmer is an indication of quick early development when this value is decreased compared to control plants. It is the ratio (expressed in %) between the time a plant needs to make 30 % of the final biomass and the time needs to make 90 % of its final biomass.

The "time to flower" or "flowering time" of the plant can be determined using the method as described in WO 2007/093444.

*Seed-related parameter measurements*

The mature primary panicles were harvested, counted, bagged, barcode-labelled and then dried for three days in an oven at 37°C. The panicles were then threshed and all the seeds were collected and counted. The seeds are usually covered by a dry outer covering, the husk. The filled husks (herein also named filled florets) were separated from the empty ones using an air-blowing device. The empty husks were discarded and the remaining fraction was counted again. The filled husks were weighed on an analytical balance.

The total number of seeds was determined by counting the number of filled husks that remained after the separation step. The total seed weight was measured by weighing all filled husks harvested from a plant.

The total number of seeds (or florets) per plant was determined by counting the number of husks (whether filled or not) harvested from a plant.

Thousand Kernel Weight (TKW) is extrapolated from the number of seeds counted and their total weight.

The Harvest Index (HI) in the present invention is defined as the ratio between the total seed weight and the above ground area ($mm^2$), multiplied by a factor $10^6$.

The number of flowers per panicle as defined in the present invention is the ratio between the total number of seeds over the number of mature primary panicles.

The "seed fill rate" or "seed filling rate" as defined in the present invention is the proportion (expressed as a %) of the number of filled seeds (i.e. florets containing seeds) over the total number of seeds (i.e. total number of florets). In other words, the seed filling rate is the percentage of florets that are filled with seed.

Example11: Results of the phenotypic evaluation of the transgenic plants

The results of the evaluation of transgenic rice plants in the T1 generation and expressing a nucleic acid encoding the CDKB-RKA polypeptide of SEQ ID NO: 318 under non-stress conditions are presented below in Table D3.

When grown under non-stress conditions, an increase of at least 5 % was observed for aboveground biomass (AreaMax), root biomass (RootMax and RootThinMax), and for seed yield including total seed weight (totalwgseeds), number of seeds (nrfilledseed). In addition, plants expressing a nucleic acid encoding a CDK polypeptide of SEQ ID NO: 318 showed early vigor (EmerVigor).

**Table D3:** Data summary for transgenic rice plants; for each parameter, the overall percent increase is shown for the confirmation (T2 generation), for each parameter the p-value is <0.05.

| Parameter | Overall increase |
|---|---|
| AreaMax | 10.5 |
| EmerVigor | 24.8 |
| RootMax | 7.2 |
| totalwgseeds | 14.1 |
| nrfilledseed | 13.2 |
| RootThinMax | 5.0 |

**Claims**

1. A method for increasing yield in a plant relative to a control plant, comprising introducing and expressing in said plant a nucleic acid molecule encoding a CDKB-RKA polypeptide, wherein said CDKB-RKA polypeptide is a modified CDKB polypeptide, and wherein said CDKB-RKA polypeptide has reduced catalytic kinase activity, as compared to said CDKB polypeptide.

2. Method according to claim 1, wherein said CDKB-RKA polypeptide lacks catalytic kinase activity.

3. Method according to claim 1 or 2, wherein said CDKB-RKA polypeptide is a mutated CDKB polypeptide.

4.  Method according to claim 1, 2 or 3, wherein said CDKB-RKA polypeptide is a CDKB polypeptide comprising a mutated kinase domain.

5.  Method according to any of claims 1 to 4, wherein said CDKB-RKA polypeptide is a truncated CDKB polypeptide, and wherein said CDKB-RKA polypeptide is a truncated CDKB polypeptide comprising a deletion in the C-terminal half of said polypeptide that reduces, and preferably substantially inactivates, that kinase activity of said CDKB.

6.  Method according to any of claims 1 to 5 wherein said CDKB-RKA polypeptide is a truncated CDKB polypeptide comprising a deletion of the kinase domain of said CDKB polypeptide.

7.  Method according to any of claims 1 to 6 wherein said CDKB polypeptide has cyclin-binding activity, and wherein said CDKB-RKA polypeptide comprises a cyclin binding domain, preferably represented by a PPTALRE motif with no mismatches or with a mismatch at position 2 and/or 4 from left to right, and more preferably represented by SEQ ID NO: 403, and more preferably represented by SEQ ID NO: 404.

8.  Method according to any of claims 1 to 7, wherein said CDKB-RKA polypeptide comprises a protein kinase inhibitory domain.

9.  Method according to any of claims 1 to 8, wherein said CDKB-RKA polypeptide lacks a T-loop activation kinase domain.

10. Method according to any one of claims 1 to 9, wherein said nucleic acid encoding an CDKB-RKA is a nucleic acid that encodes a polypeptide represented by SEQ ID NO: 318 or a homologue, orthologue or paralogue thereof, or a portion of said nucleic acid, or a nucleic acid capable of hybridising with said nucleic acid.

11. Method according to any of claims 1 to 10, wherein said increased yield relative to control plants comprises (i) increased seed yield and/or (ii) increased vegetative biomass, such as increased leaf biomass and/or increased root biomass.

12. Method according to any one of claims 1 to 11 wherein said nucleic acid is operably linked to a constitutive promoter, preferably to a constitutive plant promoter, more preferably to a medium strength constitutive plant promoter.

13. Transgenic plant, transgenic plant part thereof, including seeds, or transgenic plant cell, obtainable by a method according to any one of claims 1 to 12, wherein said plant, plant part or plant cell comprises a recombinant nucleic acid encoding a CDKB-RKA polypeptide as defined in any of claims 1 to 10.

14. Construct comprising:

    (i) nucleic acid encoding a CDKB-RKA polypeptide as defined in any of claims 1 to 10;
    (ii) one or more control sequences capable of driving expression of the nucleic acid sequence of (i); and optionally
    (iii) a transcription termination sequence;

    and wherein one of said control sequences is a constitutive promoter, preferably a constitutive plant promoter, more preferably a medium strength constitutive plant promoter.

15. Transgenic plant, transgenic plant part or transgenic plant cell transformed with a construct according to claim 14.

16. Method for the production of a transgenic plant having enhanced yield-related traits relative to control plants, preferably increased yield relative to control plants, and more preferably increased seed yield and/or increased biomass relative to control plants, comprising:

    (a) introducing and expressing in a plant cell or plant a nucleic acid encoding a CDKB-RKA polypeptide as defined in any of claims 1 to 10; and
    (b) cultivating said plant cell or plant under conditions promoting plant growth and development.

17. Transgenic plant having enhanced yield-related traits relative to control plants, preferably increased yield relative to control plants, and more preferably increased seed yield and/or increased biomass, resulting from the introduction and expression therein of a nucleic acid encoding a CDKB-RKA polypeptide as defined in any of claims 1 to 10, or a transgenic plant cell derived from said transgenic plant.

18. Use of a nucleic acid encoding a CDKB-RKA polypeptide as defined in any of claims 1 to 10, or a construct as defined in claim 14 for enhancing yield-related traits in plants relative to control plants, preferably for increasing yield, and more preferably for increasing seed yield and/or for increasing biomass in plants relative to control plants.

```
                    1                                                50
SEQ ID NO: 320  (1) MEKYEKLEKVGEGTYGKVYKAMEKTTGKLVALKKTRLEMDEEGIPPTALR
SEQ ID NO: 318  (1) MEKYEKLEKVGEGTYGKVYKAMEKTTGKLVALKKTRLEMDEEGIPPTALR
    Consensus   (1) MEKYEKLEKVGEGTYGKVYKAMEKTTGKLVALKKTRLEMDEEGIPPTALR
                             *  *TY*                            PPTALR

                     51                                              100
SEQ ID NO: 320 (51) EISLLQMLSQSIYIVRLLCVEHVIQSKDSTVSHSPKSNLYLVFEYLDTDL
SEQ ID NO: 318 (51) EISLLQMLSQSIYIVRLLCVEHVIQSKDSTVSHSPKSNLYLVFEYLDTDL
    Consensus  (51) EISLLQMLSQSIYIVRLLCVEHVIQSKDSTVSHSPKSNLYLVFEYLDTDL
                    E

                     101                                             150
SEQ ID NO: 320 (101) KKFIDSHRKGSNPRPLEASLVQRFMFQLFKGVAHCHSHGVLHRDLKPQNL
SEQ ID NO: 318 (101) KKFIDSHRKGSNPRPLEASLVQRFMFQLF---------------------
    Consensus  (101) KKFIDSHRKGSNPRPLEASLVQRFMFQLF

                     151                                             200
SEQ ID NO: 320 (151) LLDKDKGILKIADLGLSRAFTVPLKAYTHEIVTLWYRAPEVLLGSTHYST
SEQ ID NO: 318 (130) --------------------------------------------------
    Consensus  (151)
                                               T

                     201                                             250
SEQ ID NO: 320 (201) AVDIWSVGCIFAEMIRRQALFPGDSEFQQLLHIFRLLGTPTEQQWPGVMA
SEQ ID NO: 318 (130) --------------------------------------------------
    Consensus  (201)
                                          GDSEID

                     251                                             300
SEQ ID NO: 320 (251) LRDWHVYPKWEPQDLSRAVPSLSPEGIDLLTQMLKYNPAERISAKAALDH
SEQ ID NO: 318 (130) --------------------------------------------------
    Consensus  (251)

                     301      311
SEQ ID NO: 320 (301) PYFDSLDKSQF
SEQ ID NO: 318 (130) -----------
    Consensus  (301)
```

# FIGURE 1

**FIGURE 2**

```
A.thaliana_AT1G76540.1#1        -----MDEG----------VIAVSAMDAFEKLEKVGEGTYGKVYRAREKAT 36
A.thaliana_AT1G20930.1#1        -----MDNNGV-------KPAVSAMEAFEKLEKVGEGTYGKVYRAREKAT 38
H.annuus_CD855729#1             -----MEST-----------KTAMEAFEKLEKVGEGTYGKVYRARDKTT 33
G.max_Glyma14g39760.1#1         -----MEKTG----------VLSAKEAFEKLEKVGEGTYGKVYRAREKAT 35
G.max_Glyma17g38210.1#1         -----MEKPGG-------GGVLSAKEAFEKLEKVGEGTYGKVYRAREKAT 38
G.max_Glyma07g07640.1#1         -----MEKTGA------GVVVLSAKEAFEKLEKVGEGTYGKVYRAREKAT 39
G.max_Glyma09g08250.1#1         ---MEMEKTGA------GVAVLSAKEAFEKLEKVGEGTYGKVYRAREKAT 41
M.truncatula_TC117212#1         -----MEKPGET-----IAVVLSAKEAFEKLEKVGEGTYGKVYRAREKAT 40
P.trichocarpa_scaff_II.31#1     -----MEKAGGVVAAAPTNTVVSAMDAFEKLEKVGEGTYGKVYRARERAT 45
S.lycopersicum_TC205060#1       -----MEAAKN------AGKSVSAMEAFEKLEKVGEGTYGKVYRARDRIT 39
H.vulgare_TC172640#1            MATLQHQNQGAAAPMTTTGGGLRAMDLYEKLEKVGEGTYGKVYKAREKAT 50
T.aestivum_TC282781#1           MATLQHQNQGAAAPMTTTGGGLRAMDLYEKLEKVGEGTYGKVYKAREKAT 50
O.sativa_LOC_Os08g40170.1#1     MAALHHQAAAAPVTTTTDGGELRAMDLYEKLEKVGEGTYGKVYKAREKAT 50
sorghumXP_002441781.1           -------------------MEINIVDKYEKLEKVGEGTYGKVYKAQDKAT 31
Z.mays_c62195783gm030403@12303  -------------------MEINIVDKYEKLEKVGEGTYGKVYKAQDKAT 31
O.sativa_LOC_Os01g67160.1#1     -------------------------MEKYEKLEKVGEGTYGKVYKAQDRAT 26
H.vulgare_TC160457#1            -------------------------MEKYEKLEKVGEGTYGKVYKAMDKAT 26
T.aestivum_TC283074#1           -------------------------MEKYEKLEKVGEGTYGKVYKAQDKAT 26
T.aestivum_TC290551#1           -------------------------MEKYEKLEKVGEGTYGKVYKARVAAT 26
SEQ ID NO:318                   -------------------------MEKYEKLEKVGEGTYGKVYKAMEKTT 26
A.thaliana_AT2G38620.1#1        -------------------------MEKYEKLEKVGEGTYGKVYKAMEKTT 26
A.thaliana_AT2G38620.2#1        -------------------------MEKYEKLEKVGEGTYGKVYKAMEKTT 26
```

50

EP 3 091 079 A1

```
A.thaliana_AT3G54180.1#1          ---------------------------MEKYEKLEKVGEGTYGKVYKAMEKGT 26
ChenopodiumCAC17703.1             ---------------------------MEKYEKLEKVGEGTYGKVYKAKEKTT 26
P.trichocarpa_scaff_XVI.1376#1    ---------------------------MEKYEKLEKVGEGTYGKVYKAKDKLT 26
P.trichocarpa_scaff_3712.1#1      ---------------------------MEKYEKLEKVGEGTYGKVYKAKDKLT 26
P.trichocarpa_scaff_28.331#1      ---------------------------MEKYEKLEKVGEGTYGKVYKAKDKVT 26
ricinusXP_002519974.1             ---------------------------MEKYEKLEKVGEGTYGKVYKAKDRVT 26
vitisXP_002266623.1               ---------------------------MDKYEKLEKVGEGTYGKVYKAKDKTS 26
S.lycopersicum_TC198540#1         ---------------------------MEKYEKLEKVGEGTYGKVYKAKDKAT 26
tomatoCAC15503.1                  ---------------------------MEKYEKLEKVGEGTYGKVYKAKDKAT 26
tobaccoAAG01532.1                 ---------------------------MEKYEKLEKVAEGTYGKVYKAKDKAT 26
anthirinumCAA66235.1              ---------------------------MEKYEKIEKVGEGTYGKVYKALEKST 26
M.truncatula_TC115190#1           ---------------------------MEKYEKLEKVGEGTYGKVYKAKEIST 26
medicagoP93321                    ---------------------------MEKYEKLEKVGEGTYGKVYKAKELST 26
lotus                             ---------------------------MENYEKLEKVGEGTYGKVYKAKEKST 26
G.max_Glyma07g02400.1#1           ---------------------------MEKYEKLEKVGEGTYGKVYKAREKAS 26
P.patens_NP13120379#1             ---------------------------MENYEKLEKIGEGMYGKVYKARDRRT 26
P.patens_TC47562#1                ---------------------------MENYEKIEKIGEGMYGKVYKAWDKRT 26
P.patens_NP13127560#1             ---------------------------MDEYEKLEKIGEGTYGKVYKARNKST 26
P.patens_TC43373#1                ---------------------------MDGYEKLEKVGEGTYGKVYKARDKRT 26
P.patens_TC41028#1                ----------------------MSVSGMDNYEKLEKVGEGTYGKVYKARDKRS 31
P.patens_TC35664#1                ---------------------------MDKYEKLEKVGEGTYGKVYKARDKLT 26
                                                             : :**:**:*** *****:*       :
```

**FIGURE 2 (continued)**

**FIGURE 2 (continued)**

```
A.thaliana_AT1G76540.1#1             GKIVALKKTRLHEDEEGVPSTTLREISILRMLARDPHVVR-LMDVKQGLS 85
A.thaliana_AT1G20930.1#1             GMIVALKKTRLHEDEEGVPPTTLREISILRMLARDPHIVR-LMDVKQGIN 87
H.annuus_CD855729#1                  GKIVALKKTRLHEDEEGVPPTTLREISILRMLSIDPHVVK-LMDVKQGVN 82
G.max_Glyma14g39760.1#1              GKIVALKKTRLHEDEEGVPPTTLREVSILRMLSRDPHVVR-LMDVKQGQN 84
G.max_Glyma17g38210.1#1              GKIVALKKTRLHEDEEGVPPTTLREVSILRMLSRDPHVVR-LMDVKQGQN 87
G.max_Glyma07g07640.1#1              GKIVALKKTRLHEDQDGVPPTTLREVSILRMLSRDPHVVS-LMDVKQGQN 88
G.max_Glyma09g08250.1#1              GKIVALKKTRLHEDQEGVPPTTLREVSILRMLSRDPHVVR-LMDVKQGQN 90
M.truncatula_TC117212#1              GKIVALKKTRLHEDDEGVPPTTLREVSILRMLSRDPHVVR-LLDVKQGQN 89
P.trichocarpa_scaff_II.31#1          GKIVALKKTRLHEDDEGVPTTTLREVSILRMLSRDPHVVR-LMDVKQGQN 94
S.lycopersicum_TC205060#1            GKIVALKKTRLHEDEEGVPPTTLREISLLKMLSRDPHIVR-LMDVKQGQN 88
H.vulgare_TC172640#1                 GRIVALKKTRLPEDDEGVPPTALREVSLLRMLSQDPHVVR-LLDLKQGQN 99
T.aestivum_TC282781#1                GRIVALKKTRLPEDDEGVPPTALREVSLLRMLSQDPHVVR-LLDLKQGQN 99
O.sativa_LOC_Os08g40170.1#1          GRIVALKKTRLPEDDEGVPPTALREVSLLRMLSQDSHVVR-LLDLKQGQN 99
sorghumXP_002441781.1                GQLVALKKTRLEMDEEGIPPTALREISLLNLLSHSIYVVR-LLAVEQAAK 80
Z.mays_c62195783gm030403@12303       GQLVALKKTRLEMDEEGIPPTALREISLLNLLSHSIYIVR-LLAVEQAAK 80
O.sativa_LOC_Os01g67160.1#1          GQLVALKKTRLEMDEEGIPPTALREISILRLLSQSLYVVR-LLSVEQATK 75
H.vulgare_TC160457#1                 GQVVALKKTRLEMDDEGIPPTALREISLLRLLSSSLYVVR-LLAVEQTTK 75
T.aestivum_TC283074#1                GQVVALKKTRLEMDDEGIPPTALREISLLRLLSSSLYVVR-LLAVEQATK 75
T.aestivum_TC290551#1                GQLVALKKTRLEMDEEGIPPTALREISLLRLLSASLYVVR-LLSVEQVTK 75
SEQ ID NO: 318                       GKLVALKKTRLEMDEEGIPPTALREISLLQMLSQSIYIVR-LLCVEHVIQ 75
A.thaliana_AT2G38620.1#1             GKLVALKKTRLEMDEEGIPPTALREISLLQMLSQSIYIVR-LLCVEHVIQ 75
A.thaliana_AT2G38620.2#1             GKLVALKKTRLEMDEEGIPPTALREISLLQMLSQSIYIVR-LLCVEHVIQ 75
```

**FIGURE 2 (continued)**

```
A.thaliana_AT3G54180.1#1        GKLVALKKTRLEMDEEGIPPTALREISLLQMLSTSIYVVR-LLCVEHVHQ 75
ChenopodiumCAC17703.1           GQLVALKKTRLEMDEEGVPPTALREVSLLQMLSQSLYVVRLLLCVEHVDK 76
P.trichocarpa_scaff_XVI.1376#1  GQLVALKKTRLQMDEEGVPPTALREVSLLQMLSQSLYVVR-LLSVEHLD- 74
P.trichocarpa_scaff_3712.1#1    GQLVALKKTRLQMDEEGVPPTALREVSLLQMLSQSLYVVR-LLSVEHID- 74
P.trichocarpa_scaff_28.331#1    GQIVALKKTRLEMDEEGVPPTALREVSLLQMLSQSLYVVR-LLSVEHVD- 74
ricinusXP_002519974.1           GQIVALKKTRLEMDEEGIPPTALREVSLLQMLSQSLYVVR-LLSVEHVD- 74
vitisXP_002266623.1             GQVVALKKTRLEMDEEGVPPTALREVSLLQMLSQSLYVVR-LLCVEHLD- 74
S.lycopersicum_TC198540#1       GQLVALKKTRLEMDEEGIPPTALREISLLQMLSHSLYIVR-LLCVEHID- 74
tomatoCAC15503.1                GQLVALKKTRLEMDEEGIPPTALREISLLQMLSHSLYIVR-LLCVEHID- 74
tobaccoAAG01532.1               GQLVALKKTRLEMDEEGIPPTALREISLLQMLSNSLYIVR-LLCVEQID- 74
anthirinumCAA66235.1            GQVVALKKTRLEMDEEGVPPTALREVSLLQMLSQSLYVVR-LLSVEHVDC 75
M.truncatula_TC115190#1         GQIVALKKTRLEMDEEGVPPTALREVSLLQMLSQSLYIVR-LLNVEHIDK 75
medicagoP93321                  GQIVALKKTRLEMDEEGVPPTALREVSLLQMLSQSLYIVR-LLNVEHIDK 75
lotus                           GQVVALKKTRLEMDEEGVPPTALREVSLLQMLSQSIYIVR-LLKVEHVDK 75
G.max_Glyma07g02400.1#1         GSLVALKKTRLEMDEEGVPPTALREVSLLQLLSQSIYIVR-LLSVEHVDK 75
P.patens_NP13120379#1           GALVALKKIKIENEEEGIPATTLREVSLLQMLSKCIYFVR-LLGVEHLHK 75
P.patens_TC47562#1              GAMVALKKNKIENEKEGIPATTLREVSLLQVLSTCIYFVR-LLSVENSHK 75
P.patens_NP13127560#1           GRLVALKKTRLEMEDEGVPSTALREVSLLQMLSHSIYIVR-LLSVESVDE 75
P.patens_TC43373#1              GRFVALKKTRLENEDEGVPSTTLREVSLLQMLSHSIYIVR-LLCVESVDE 75
P.patens_TC41028#1              GQLVALKKTRLEMEEEGVPSTALREVSLLQMLSHSMYIVR-LLCVEHVEK 80
P.patens_TC35664#1              GQLVALKKTRLEMEEEGVPSTALREVSLLQMLSHSIYIVR-LLCVEHVEK 75
                                * .***** ::   :.:*:*.*:***.:*.**..*:   :.*   *: ::
```

EP 3 091 079 A1

**FIGURE 2 (continued)**

```
A.thaliana_AT1G76540.1#1            KEG----------------------KTVLYLVFEYMDTDVKKFIRSFRS--T 113
A.thaliana_AT1G20930.1#1            KEG----------------------KTVLYLVFEYVDTDLKKFIRSFRQ--A 115
H.annuus_CD855729#1                 KNG----------------------NTVLYLVFEYMETDLKKFIRSYRQ--T 110
G.max_Glyma14g39760.1#1             KEG----------------------KTVLYLVFEYMDTDLKKFIRSFRQ--S 112
G.max_Glyma17g38210.1#1             KEG----------------------KTVLYLVFEYMDTDLKKFIRSFRQ--T 115
G.max_Glyma07g07640.1#1             KEG----------------------KTVLYLVFEYMDTDLKKFIRSFDQ--P 116
G.max_Glyma09g08250.1#1             KEG----------------------KTVLYLVFEYMDTDLKKFIRSFRQ--T 118
M.truncatula_TC117212#1             KEG----------------------KTVLYLVFEYMDTDLKKFIRSFRQ--T 117
P.trichocarpa_scaff_II.31#1         KQG----------------------KTVLYLVFEYMDTDRLKKFIRSFLQ--T 122
S.lycopersicum_TC205060#1           KEG----------------------KTVLYLVFEYMDTDVKKFIRTFRA--K 116
H.vulgare_TC172640#1                KEG----------------------QTILYLVFEYMDTDLKKFIRGHRQ--N 127
T.aestivum_TC282781#1               KEG----------------------QTILYLVFEYMDTDLKKFIRGHRQ--N 127
O.sativa_LOC_Os08g40170.1#1         KEG----------------------QTILYLVFEYMDTDLKKFIRAHRQ--N 127
sorghumXP_002441781.1               N------------------------GKPVLYLVFEFLDTDLKKYLDVYRRGPA 109
Z.mays_c62195783gm030403@12303      N------------------------GKPVLYLVFEFLDTDLKKYLDVYRRGPS 109
O.sativa_LOC_Os01g67160.1#1         N------------------------GKPVLYLVFEFLDTDLKKFVDAYRKGPN 104
H.vulgare_TC160457#1                GGGAGG----------------GGKPVLYLVFEFLDTDLKKFVDAYRRGPA 110
T.aestivum_TC283074#1               GGGAGS----------------GGKPVLYLVFEFLDTDLKKFVDAYRRGPA 110
T.aestivum_TC290551#1               P------------------------CGKTVLYLVFEFLDTDLKKFVDGFRKGPS 105
SED ID NO: 318                      SKDSTVSHSP-------------KSNLYLVFEYLDTDLKKFIDSHRKGSN 112
A.thaliana_AT2G38620.1#1            SKDSTVSHSP-------------KSNLYLVFEYLDTDLKKFIDSHRKGSN 112
A.thaliana_AT2G38620.2#1            SKDSTVSHSP-------------KSNLYLVFEYLDTDLKKFIDSHRKGSN 112
```

**FIGURE 2 (continued)**

```
A.thaliana_AT3G54180.1#1          P--STKSQST-------------KSNLYLVFEYLDTDLKKFIDSYRKGPN 110
ChenopodiumCAC17703.1             K--NTTTGIS-------------KPILYLVFEYLDTDLKKFIDSHRKGSN 111
P.trichocarpa_scaff_XVI.1376#1    -ANNSD---DDS-----------KSNLYLVFEFLDADLKKFIDSHRKGPN 109
P.trichocarpa_scaff_3712.1#1      -ANNSD---DDS-----------KSNLYLVFEFLDADLKKFIDSHRKGPN 109
P.trichocarpa_scaff_28.331#1      -ANSTNSNLDDSNPSKRNHNNNNKSNLYLVFEYLDTDLKKFIDSHRKGAN 123
ricinusXP_002519974.1             -VNSAD---DNN---INKENAISKSNLYLVFEYLDTDLKKFIDSHRKGPK 117
vitisXP_002266623.1               -KNGKP-----------------FLYLVFEYLDTDLKKFIDSHRKPPN 104
S.lycopersicum_TC198540#1         -KNGKP-----------------ILYLVFEYLDTDLKKFIDSHRKGPN 104
tomatoCAC15503.1                  -KNGKP-----------------ILYLVFEYLDTDLKKFIDSHRKGPN 104
tobaccoAAG01532.1                 -KNGKP-----------------LLYLVFEYLDTDLKKFVDSHRKGPN 104
anthirinumCAA66235.1              AKNGKP-----------------LLYLVFEYLDTDLKKFIDSHRKGPN 106
M.truncatula_TC115190#1           PPKNATHTP------------AKPLLYLVFEYLDTDLKKFIDTFRKGVN 112
medicagoP93321                    PPKNATHTP------------SKPLLYLVFEYLDTDLKKFIDTFRKGTN 112
lotus                             TPKNNNHHPSAA---ADHPPPPSKPLLYLVFEYLDTDLKKYIDTYRKGPN 122
G.max_Glyma07g02400.1#1           VPKSQKSSSN---------PLTKPILYLVFEYLDTDLKKFIDSHRKGPN 115
P.patens_NP13120379#1             NG--------------------KPALYLVFEYIDTDLRRFLDLSWPG-L 103
P.patens_TC47562#1                NG--------------------KTSLFMVFEYIDTDLRRFMDLSWPG-P 103
P.patens_NP13127560#1             NG--------------------KPQLYLVFEYLDSDLKKFIDSYGRGSG 104
P.patens_TC43373#1                NG--------------------KPQLYLVFEYLDSDLKKYIDLHGRGPG 104
P.patens_TC41028#1                GG--------------------KPMLYLVFEYMDTDLKKYIDLHGRGPS 109
P.patens_TC35664#1                GG--------------------KPMLYLVFEYMDTDLKKYIDLHGRGPS 104
                                                         *::***::::*:::::
```

**FIGURE 2 (continued)**

```
A.thaliana_AT1G76540.1#1          GKNIPTQTIKSLMYQLCKGMAFCHGHGILHRDLKPHNLLMDPKTMRLKIA 163
A.thaliana_AT1G20930.1#1          GQNIPQNTVKCLMYQLCKGMAFCHGHGVLHRDLKPHNLLMDRKTMTLKIA 165
H.annuus_CD855729#1               GDNIPPQIVKSLMYQLCKGIAFCHAHGILHRDLKPHNLLMDRKTLMLKIA 160
G.max_Glyma14g39760.1#1           GETIPPHIIKSLMYQLCKGVAFCHGHGILHRDLKPHNLLMDRKTMMLKIA 162
G.max_Glyma17g38210.1#1           GQTVPPQTIKSLMYQLCKGVAFCHGHGILHRDLKPHNLLMDPKTMMLKIA 165
G.max_Glyma07g07640.1#1           GQNIPPETIKSLMYQLCKGIAFCHGHGILHRDLKPHNLLMDRKTMMLKIA 166
G.max_Glyma09g08250.1#1           GQSIPPQTIKSLMYQLCKGIAFCHGHGILHRDLKPHNLLMDRKTMMLKIA 168
M.truncatula_TC117212#1           GQNIPPPTIKGLMYQLCKGVAFCHGHGILHRDLKPHNLLMDRKTMMLKIA 167
P.trichocarpa_scaff_II.31#1       GENIPVKNVKSLMYQLCKGVAFCHGHGVLHRDLKPHNLLMDRKTMMLKIA 172
S.lycopersicum_TC205060#1         GETMPLKIVKSLMYQLCKGVAFCHGHGVLHRDLKPHNLLMDRKTNVLKLA 166
H.vulgare_TC172640#1              HQKIPAHTVKILMYQLCKGVAFCHGRGVLHRDLKPHNLLMDRKTMALKIA 177
T.aestivum_TC282781#1             HEKIPAHTVKILMYQLCKGVAFCHGRGVLHRDLKPHNLLMDRKTMALKIA 177
O.sativa_LOC_Os08g40170.1#1       LQKIPVPTVKILMYQLCKGVAFCHGRGVLHRDLKPHNLLMDRKTMALKIA 177
sorghumXP_002441781.1             ARPLPATLIKNFLYQLCKGVAHCHGHGVLHRDLKPQNLLVDKDKGILKIA 159
Z.mays_c62195783gm030403@12303    ARPLPATLIKNFLYQLCKGVAHCHSHGVLHRDLKPQNLLVDKEKGILKIA 159
O.sativa_LOC_Os01g67160.1#1       PRPLPTNVIKSFLYQLCKGVAHCHGHGVLHRDLKPQNLLVDKEKGILKIA 154
H.vulgare_TC160457#1              PKPLPTHVVKSFLYQLCKGIAHCHGHGVLHRDLKPQNLLVDKEKMILKIA 160
T.aestivum_TC283074#1             PKPLPTQVVKSFLYQLCKGIAHCHGHGVLHRDLKPQNLLVDKEKMILKIA 160
T.aestivum_TC290551#1             PRPVPTQVVKSFLYQLCKGTAHCHGHGVLHRDLKPQNLLIDKEKGVLKIA 155
SEQ ID NO: 318                    PRPLEASLVQRFMFQLF--------------------------------- 129
A.thaliana_AT2G38620.1#1          PRPLEASLVQRFMFQLFKGVAHCHSHGVLHRDLKPQNLLLDKDKGILKIA 162
A.thaliana_AT2G38620.2#1          PRPLEASLVQRFMFQLFKGVAHCHSHGVLHRDLKPQNLLLDKDKGILKIA 162
```

EP 3 091 079 A1

**FIGURE 2 (continued)**

```
A.thaliana_AT3G54180.1#1         PKPLEPFLIQKLMFQLCKGVAHCHSHGVLHRDLKPQNLLLVKDKELLKIA 160
ChenopodiumCAC17703.1            PRPLPPCQIQSFLFQLLKGVSHCHSHGVLHRDLKPQNLLVDKDKGILKIA 161
P.trichocarpa_scaff_XVI.1376#1   PRPLSPSLIQSFLFQLCKGVAHCHSHGVLHRDLKPQNLLLDQERGILKIA 159
P.trichocarpa_scaff_3712.1#1     PRPLSPSLIQSFLFQLCKGVAHCHSHGVLHRDLKPQNLLLDQERGILKIA 159
P.trichocarpa_scaff_28.331#1     PRPLSPSLIQSFLFQLCKGVAHCHSHGVLHRDLKPQNLLLDQEKGILKIA 173
ricinusXP_002519974.1            PTPLAPGLIQSFLFQLCKGVAHCHSHGVLHRDLKPQNLLLDQEKGILKIA 167
vitisXP_002266623.1              PRPMPPALIQSFLYQLCKGVAHCHSHGVLHRDLKPQNLLVDKDKGILKIA 154
S.lycopersicum_TC198540#1        ARALPTALIQSFLFQLCKGVAHCHSHGVLHRDLKPQNLLVDKEKGILKIA 154
tomatoCAC15503.1                 ARALPTALIQSFLFQLCKGVAHCHSHGVLHRDLKPQNLLVDKEKGILKIA 154
tobaccoAAG01532.1                PRPLPPSLIQSFLYQLCKGVAHCHSHGVLHRDLKPQNLLVDKEKGILKIA 154
anthirinumCAA66235.1             PRPLPPQQIQSFLFQLCKGVSHCHAHGVLHRDLKPQNLLLDKDKGVLKIA 156
M.truncatula_TC115190#1          PRPLPNTLVQSFLFQLCKGVAHCHSHGVLHRDLKPQNLLLDQAKGILKIA 162
medicagoP93321                   PRPLPNTLVQSFLFQLCKGVAHCHSHGVLHRDLKPQNLLLDQAKGILKIA 162
lotus                            PRPLPPTLIQSFLFQLCMGVAHCHSHGVLHRDLKPQNLLLDQQKGILKIA 172
G.max_Glyma07g02400.1#1          PRPLPPPLIQSFLFQLCKGVAHCHSHGVLHRDLKPQNLLLDQHKGILKIA 165
P.patens_NP13120379#1            DNPLPQNTIKSFVYQLLKGVAHCHSHGVMHRDLKPQNLLVDWSKGLLKIA 153
P.patens_TC47562#1               DNPLPQNTIKSFMFQLLKGVAHCHSHGIMHRDLKPQNLLVDWKKGLLKIA 153
P.patens_NP13127560#1            -NPIPAKTIQSFMYQLLKGVAHCHGHGVMHRDLKPQNLLVDKEKGLLKIA 153
P.patens_TC43373#1               TNPIPAKTVQSFMYQLLKGVAHCHSHGVMHRDLKPQNLLVDKEKGLLKIA 154
P.patens_TC41028#1               GKPLPPKVVQSFMYQLCTGLAHCHGHGVMHRDLKPQNLLVDKQTRRLKIA 159
P.patens_TC35664#1               GKPLPPKVVQSFMYQLCTGLAHCHGHGVMHRDLKPQNLLVDKQTRRLKIA 154
                                 :       ::  :::**
```

**FIGURE 2 (continued)**

EP 3 091 079 A1

```
A.thaliana_AT1G76540.1#1                    DLGLARAFTLPMKKYTHEILTLWYRAPEVLLGATHYSTAVDMWSVGCIFA 213
A.thaliana_AT1G20930.1#1                    DLGLARAFTLPMKKYTHEILTLWYRAPEVLLGATHYSTGVDMWSVGCIFA 215
H.annuus_CD855729#1                         DLGLARAFTLPIKKYTHEILTLWYRAPEVLLGSTHYSTAVDMWSVGCIFA 210
G.max_Glyma14g39760.1#1                     DLGLARAFTVPIKKYTHEILTLWYRAPEVLLGATHYSMAVDMWSVGCIFA 212
G.max_Glyma17g38210.1#1                     DLGLARAFTVPIKKYTHEILTLWYRAPEVLLGATHYSMAVDIWSVGCIFA 215
G.max_Glyma07g07640.1#1                     DLGLARAFTVPIKKYTHEILTLWYRAPEVLLGATHYSMAVDIWSVGCIFA 216
G.max_Glyma09g08250.1#1                     DLGLARAFTVPIKKYTHEILTLWYRAPEVLLGATHYSMAVDIWSVGCIFA 218
M.truncatula_TC117212#1                     DLGLARAFTVPLKKYTHEILTLWYRAPEVLLGATHYSMAVDMWSVACIFA 217
P.trichocarpa_scaff_II.31#1                 DLGLARAFTLPIKKYTHEILTLWYRAPEVLLGATHYSTAVDVWSVGCIFA 222
S.lycopersicum_TC205060#1                   DFGLGRAYTLPIKKYTHEILTLWYRAPEVLLGATHYSTAVDMWSVGCIFA 216
H.vulgare_TC172640#1                        DLGLSRAFTVPLKKYTHEILTLWYRAPEVLLGATHYSTPVDMWSVGCIFA 227
T.aestivum_TC282781#1                       DLGLSRAFTVPLKKYTHEILTLWYRAPEVLLGATHYSTPVDMWSVGCIFA 227
O.sativa_LOC_Os08g40170.1#1                 DLGLSRSFTVPLKKYTHEILTLWYRAPEVLLGAAHYSTPVDIWSVGCIFA 227
sorghumXP_002441781.1                       DLGLGRAFTVPMKSYTHEIVTLWYRAPEVLLGATHYSTGVDMWSVGCIFA 209
Z.mays_c62195783gm030403@12303              DLGLGRAFTVPMKSYTHEIVTLWYRAPEVLLGATHYSTGVDMWSVGCIFA 209
O.sativa_LOC_Os01g67160.1#1                 DLGLGRAFTVPMKSYTHEIVTLWYRAPEVLLGSTHYSTGVDIWSVGCIFA 204
H.vulgare_TC160457#1                        DLGLSRAFTVPMKSYTHEIVTLWYRAPEVLLGATHYSTGVDIWSIGCIFA 210
T.aestivum_TC283074#1                       DLGLSRAFTVPMKSYTHEIVTLWYRAPEVLLGATHYSTSVDIWSIGCIFA 210
T.aestivum_TC290551#1                       DLGLSRAFTVPMKSYTHEIVTLWYRAPEVLLGATHYSTGVDIWSIGCIFA 205
SEQ ID NO: 318                              --------------------------------------------------
A.thaliana_AT2G38620.1#1                    DLGLSRAFTVPLKAYTHEIVTLWYRAPEVLLGSTHYSTAVDIWSVGCIFA 212
A.thaliana_AT2G38620.2#1                    DLGLSRAFTVPLKAYTHEIVTLWYRAPEVLLGSTHYSTAVDIWSVGCIFA 212
```

FIGURE 2 (continued)

EP 3 091 079 A1

```
A.thaliana_AT3G54180.1#1          DLGLGRAFTVPLKSYTHEIVTLWYRAPEVLLGSTHYSTGVDMWSVGCIFA 210
ChenopodiumCAC17703.1             DLGLGRAFTVPMKSYTHEIVTLWYRAPEVLLGSTHYSTGVDIWSVGCIFA 211
P.trichocarpa_scaff_XVI.1376#1    DLGLGRAFTVPLKSYTHEIVTLWYRAPEVLLGSTHYSIAIDMWSVGCIFA 209
P.trichocarpa_scaff_3712.1#1      DLGLGRAFTVPLKSYTHEIVTLWYRAPEVLLGSTHYSIAIDMWSVGCIFA 209
P.trichocarpa_scaff_28.331#1      DLGLGRAFTVPLKSYTHEIVTLWYRAPEVLLGSTHYSTGVDMWSVGCIFA 223
ricinusXP_002519974.1             DLGLGRAFTVPLKSYTHEIVTLWYRAPEVLLGSTHYSTAVDMWSVGCIFA 217
vitisXP_002266623.1               DLGLGRAFTVPLKSYTHEIVTLWYRAPEVLLGSTHYSTGVDMWSVGCIFA 204
S.lycopersicum_TC198540#1         DLGLGRAFTVPMKSYTHEIVTLWYRAPEVLLGSTHYSTAVDMWSVGCIFA 204
tomatoCAC15503.1                  DLGLGRAFTVPMKSYTHEIVTLWYRAPEVLLGSTHYSTAVDMWSVGCIFA 204
tobaccoAAG01532.1                 DLGLGRAFTVPIKSYTHEIVTLWYRAPEVLLGSTHYSTAVDMWSVGCIFA 204
anthirinumCAA66235.1              DLGLARAFTVPLKSYTHEIVTLSYRAPEVLLGSSHYSTAVDMSSVGCIFA 206
M.truncatula_TC115190#1           DLGLGRAFTVPLKSYTHEIVTLWYRAPEVLLGSSTYSTSVDIWSVGCIFA 212
medicagoP93321                    DLGLGRAFTVPLKSYTHEIVTLWYRAPEVLLGSSTYSTGVDIWSVGCIFA 212
lotus                             DLGLGRAFTVPLKSYTHEIVTLWYRAPEVLLGSTHYSTGVDMWSVGCIFA 222
G.max_Glyma07g02400.1#1           DLGLGRAFTVPLKSYTHEIVTLWYRAPEVLLGSTHYSTGVDIWSVGCIFA 215
P.patens_NP13120379#1             DLGLGRAFTVPVKSYTHEVVTLWYRAPEILLGASHYSTPVDMWSVGCIFA 203
P.patens_TC47562#1                DLGLGRAFTVPMKSYTHEVVTLWYRAPEILLGASHYSTPVDMWSVGCVFA 203
P.patens_NP13127560#1             DLGLGRAFTIPLKSYTHEIVTLWYRAPEVLLGSSQYSTSVDMWSVGCIFA 203
P.patens_TC43373#1                DLGLGRAFTVPLKSYTHEIVTLWYRAPEVLLGASHYSTSVDVWSVGCIFA 204
P.patens_TC41028#1                DLGLGRAFTVPMKSYTHEIVTLWYRAPEVLLGATHYSLPVDIWSVGCIFA 209
P.patens_TC35664#1                DLGFGRAFTVPMKSYTHEIVTLWYRAPXVFS------------------E 186
```

**FIGURE 2 (continued)**

```
A.thaliana_AT1G76540.1#1          ELVTNQAIFQGDSELQQLLHIFKLFGTPNEEMWPGVSTLKNWHEYPQWKP 263
A.thaliana_AT1G20930.1#1          ELVTKQAIFAGDSELQQLLRIFRLLGTPNEEVWPGVSKLKDWHEYPQWKP 265
H.annuus_CD855729#1               ELVTNTAHFC-----------WRF-------------------------- 223
G.max_Glyma14g39760.1#1           ELVTKQALFPGDSELQQLLHIFRLLGTPNEDVWPGVSKLMNWHEYPQWNP 262
G.max_Glyma17g38210.1#1           ELVTKQALFPGDSELQQLLHIFRLLGTPNEDVWPGVSKLMNWHEYPQWNP 265
G.max_Glyma07g07640.1#1           ELVTRRALFPGDSELQQLLHIFRLLGTPNEEVWPGVSKLKDWHEYPQWNS 266
G.max_Glyma09g08250.1#1           ELVTKQALFAGDSELQQLLHIFRLLGTPNEEVWPGVSKLKDWHEYPQWNP 268
M.truncatula_TC117212#1           ELVTKTALFPGDSELQQLLHIFRLLGTPNEDVWPGVSKLMNWHEYPQWGP 267
P.trichocarpa_scaff_II.31#1       ELATKQPLFPGDSELQQLLHIFRLLGTPNEEMWPGVSNLMNWHEYPQWKP 272
S.lycopersicum_TC205060#1         ELVTNQALFPGDSELQQLLHIFRLLGTPNEELWPGVSKLVNWHEYPQWKP 266
H.vulgare_TC172640#1              ELITTTALFPGDSEVQQLLHIFQLLGTPNEEVWPGVGKLPNWHEYPQWNV 277
T.aestivum_TC282781#1             ELITTTALFPGDSEVQQLLHIFKLLGTPNEDVWPGVGKLPNWHEYPQWNV 277
O.sativa_LOC_Os08g40170.1#1       ELATNQPLFAGDSEVQQLLHIFKLLGTPNEQVWPGVSKLPNWHEYPQWNP 277
sorghumXP_002441781.1             EMARRQALFPGDSELQQLLHIFRLLGTPSEEQWPGVSELRDWHEFPQWKP 259
Z.mays_c62195783gm030403@12303    EMARRQALFPGDSELQQLLHIFRLLGTPTEEQWPGVSDLRDWHEFPQWKP 259
O.sativa_LOC_Os01g67160.1#1       EMVRRQALFPGDSELQQLLHIFRLLGTPTEEQWPGVTDLRDWHEFPQWKP 254
H.vulgare_TC160457#1              EMVRKQALFPGDSELQQLLHIFRLMGTPTEEDWPGVTALRDWHEFPQWKA 260
T.aestivum_TC283074#1             EMVXKQALFPGDSELQQLLHIFKLLGN----SYRGGLAWSNCSEG----L 252
T.aestivum_TC290551#1             EMVRRQALFPGDSELQQLLHIFRLLGTPNEEKWPGVCALRDWHDYPQWKP 255
SEQ ID NO: 318                    --------------------------------------------------
A.thaliana_AT2G38620.1#1          EMIRRQALFPGDSEFQQLLHIFRYL-----------LLVFDANVYVYRNQ 251
A.thaliana_AT2G38620.2#1          EMIRRQALFPGDSEFQQLLHIFRLLGTPTEQQWPGVMALRDWHVYPKWEP 262
```

EP 3 091 079 A1

EP 3 091 079 A1

**FIGURE 2 (continued)**

```
A.thaliana_AT3G54180.1#1        EMVRRQALFPGDSEFQQLLHIFRLLGTPTEQQWPGVSTLRDWHVYPKWEP 260
ChenopodiumCAC17703.1           EMVRRQALFPGDSEFQQLLHIFRLLGTPTDEQWPGVTSLRTGMCIRNGSL 261
P.trichocarpa_scaff_XVI.1376#1  EMSRRQALFPGDSELQQLL------GTPTEEQWPGVTSLRDWHVYPKWEP 253
P.trichocarpa_scaff_3712.1#1    EMSRRQALFPGDSELQQLLHIFRLLGTPTEEQWPGVTSLRDWHVYPKWEP 259
P.trichocarpa_scaff_28.331#1    EMSRRQALFPGDSEFQQLLHIFRLLGTPTEEQWPGVTALRDWHVYPKWEP 273
ricinusXP_002519974.1           EMARRQALFPGDSEFQQLLHIFRLLGTPTEKQWPGVTSFRDWHVYPQWEP 267
vitisXP_002266623.1             EMVRRQALFPGDSEFQQLLHIFRLLGTPTEKQWPGVSSLRDWHVYPQWEP 254
S.lycopersicum_TC198540#1       EMVRRQALFPGDSEFQQLLHIFRLLGTPTDKQWPGVSSLRDWHVYPQWEP 254
tomatoCAC15503.1                EMVRRQALFPGDSEFQQLLHIFRLLGTPTDKQWPGVSSLRDWHVYPQWEP 254
tobaccoAAG01532.1               EMVRRQALFPGDSEFQQLLHIFRLLGTPTEKQWPGVSSLRDWHVYPKWEP 254
anthirinumCAA66235.1            EMVRRQALFPGDSEFQQLLHIFRLLGTPSDEQWPGVSSLRDWHVYPQWEP 256
M.truncatula_TC115190#1         EMVRRQALFPGDSEFQQLLNIFKLLGTPTDQQWPGVSSLRDWHVYPRWEP 262
medicagoP93321                  EMVRRQALFPGDSEFQQLLNIFKLLGTPTEQQWPGVSSLRDWHVYPRWEP 262
lotus                           EMARRQALFPGDSEFQQLLNIFKVLGTPTEEQWPGVTSLRDWHVYPRWEP 272
G.max_Glyma07g02400.1#1         EMVRRQALFPGDSEFQQLIHIFKMLGTPTEENWPGVTSLRDWHVYPRWEP 265
P.patens_NP13120379#1           ELCRKTPLFPGNSELQQLLYIFRLLGTPNEQVWPGVTTLRDWHAYPQWRA 253
P.patens_TC47562#1              ELCRKSPLFPGNSELQQLLFIFRLLGTPNEQIWPGVTTLRNWHSYPQWKP 253
P.patens_NP13127560#1           ELSRKAPLFPGDSELQQLLHIFRLLGTPTEESWPGVKKLRDWHEYPQWQP 253
P.patens_TC43373#1              ELSRKAPLFPGDSELQQLLHIFRMLGTPKEECWPGVNKLRDWHEYPQWPA 254
P.patens_TC41028#1              ELVRKMPLFTGDSELQQLLHIFRLLGTPNETIWPGVSQHRDWHEFPQWRP 259
P.patens_TC35664#1              QPTTPFLLIFG------LLDVFSLSSFAKCRSSPGIQNFNSSTSSGYLA 230
```

**FIGURE 2 (continued)**

```
A.thaliana_AT1G76540.1#1              STLSSAVP------NLDEAGVDLLSKMLQYEPAKRISAKMAMEHPYFDDL 307
A.thaliana_AT1G20930.1#1              LSLSTAVP------NLDEAGLDLLSKMLEYEPAKRISAKKAMEHPYFDDL 309
H.annuus_CD855729#1                   --------------------------------------------------
G.max_Glyma14g39760.1#1               QSLSTAVP------SLDELGLDLLSQMLKYEPSKRISAKKAMEHVYFDDL 306
G.max_Glyma17g38210.1#1               QSLSTAVP------SLDELGLDLLSQMLKYEPSKRISAKKAMEHAYFDDL 309
G.max_Glyma07g07640.1#1               QSLSTAVP------GLEELGLDLLSQMLEYEPSKRISAKKAMEHAYFDDL 310
G.max_Glyma09g08250.1#1               KSLSTAVP------GLDELGLDLLSQMLEYEPSKRISAKKAMEHAYFNDL 312
M.truncatula_TC117212#1               QSLSKAVP------GLEEAGVDLLSQMLQYEPSKRLSAKKAMEHPYFDDL 311
P.trichocarpa_scaff_II.31#1           QSLSSSVT------NLDKDGLDLLSQMLQYDPSKRISAKKAMEHPYFDEL 316
S.lycopersicum_TC205060#1             QPLSTVVP------GLDEDGIHLLSEMLHYEPSRRISAKKAMEHPYFDDL 310
H.vulgare_TC172640#1                  S----------------------------KAVFCNSQSRCRWY--- 292
T.aestivum_TC282781#1                 SKLSSVIP------SLDADGIDLLEKMLQYEPAKRISAKKAMEHPYFDDV 321
O.sativa_LOC_Os08g40170.1#1           SKVSDLVH------GLDADALDLLEKMLQYEPSKRISAKKAMEHPYFNDV 321
sorghumXP_002441781.1                 QSLARVVP------TLEPEGVDLLSKMLQLDPSNRISAIAAMEHPYFDSL 303
Z.mays_c62195783gm030403@12303        QGLARVVP------TLEPEGVDLLSKMLQLDPSNRISALAAMEHPYFNSL 303
O.sativa_LOC_Os01g67160.1#1           QILERQVP------SLEPEGVDLLSKMLQYNPANRISAKAAMEHPYFDSL 298
H.vulgare_TC160457#1                  QRMTRAVP------TLETEGIDLLSKMLQFDPANRISAKAALEHPYFNSL 304
T.aestivum_TC283074#1                 ARVS-SVE------STEPRRNDLLSEMFQFDPGKRISAKVGLKIPFSTAS 295
T.aestivum_TC290551#1                 QHLSRAVP------TLEPEGLDLLAKMLQFDPANRISAKAAMDHPYFDSL 299
SEQ ID NO: 318                        --------------------------------------------------
A.thaliana_AT2G38620.1#1              FRTLAH-------------------------------------------- 257
A.thaliana_AT2G38620.2#1              QDLSRAVP------SLSPEGIDLLTQMLKYNPAERISAKAALDHPYFDSL 306
```

62

**FIGURE 2 (continued)**

```
A.thaliana_AT3G54180.1#1        QDLTLAVP------SLSPQGVDLLTKMLKYNPAERISAKTALDHPYFDSL 304
ChenopodiumCAC17703.1           RILLVQFHRWDLMVLISYLKCSNMIQLKGSRPRQLWIIPSLIILISLNSK 311
P.trichocarpa_scaff_XVI.1376#1  QNLARAVP------SLGPQGVDLLSKMLKYDPAERISAKAAMDHPYFDSL 297
P.trichocarpa_scaff_3712.1#1    QNLARAVP------SLGPQGVDLLSKMLKYDPAERISAKAAMDHPYFDSL 303
P.trichocarpa_scaff_28.331#1    QNLARVVQ------SLGPEGVDLLSKMLKYDPAERISAKAAMDHPYFDSL 317
ricinusXP_002519974.1           QNLARAVS------SLGPDGVDLLSEMLKYDPAERISAKAAMDHPYFDSL 311
vitisXP_002266623.1             QNLARAVP------SLGPDGVDLLSKMLKYDPSERISAKAALDHPYFDSL 298
S.lycopersicum_TC198540#1       QNLASAVP------ALGPDGVDLLTKMLKFDPSDRISAKAALDHPYFDSL 298
tomatoCAC15503.1                QNLASAVP------ALGPDGVDLLTKMLKFDPSDRISAKAALDHPYFDSL 298
tobaccoAAG01532.1               QNLASAVP------ALGPDGVDLLTKMLQYDPADRISAKAALDHPYFDSL 298
anthirinumCAA66235.1            QNSAPAVP------SLGPDGLDLLTKTLKYDPADRISAKAALDHPYFDTL 300
M.truncatula_TC115190#1         QNLARAVP------SLSPDGVDLLTKMLKYNPAERISAKAALDHPYFDSL 306
medicagoP93321                  QNLARAVP------SLSPDGVDLLTKMLKYNPAERISAKAALDHPYFDSL 306
lotus                           QTLARAVP------SLGPEGVDLLSKMLKYNPADRISAKAALDHPYFDTL 316
G.max_Glyma07g02400.1#1         QSLAKNVP------SLGPDGVDLLSKMLKYNPSERISAKAALDHPYFDSL 309
P.patens_NP13120379#1           HDIAQAVP------GIEPSGVDLLDRMLQYNPANRISAKEALIHPYFDNL 297
P.patens_TC47562#1              HEIAQAVP------RVERSGVDLLDRLLQYNPANRISAKEALVHPYFDSL 297
P.patens_NP13127560#1           QNLSRAVP------DMGPEALDLLTRMLMFDPAKRVSAKAALNHPFFDDL 297
P.patens_TC43373#1              KDLSLAVP------DMSPDALDLLSRMLVFDPAKRISAKAALHHPFFDDL 298
P.patens_TC41028#1              QDLSLAVP------GLSAVGLDLLAKMLVFEPSKRISAKAALSHTYFADV 303
P.patens_TC35664#1              HRMKRFGP------GLASI---------VIGTSSRSGGPKSYLWRFLVYA 265
```

63

## FIGURE 2 (continued)

| | |
|---|---|
| A.thaliana_AT1G76540.1#1 | PEKSSL------------------------------------------------ 313 |
| A.thaliana_AT1G20930.1#1 | PDKSSL------------------------------------------------ 315 |
| H.annuus_CD855729#1 | ------------------------------------------------------ |
| G.max_Glyma14g39760.1#1 | DKRHL------------------------------------------------- 311 |
| G.max_Glyma17g38210.1#1 | DKRHL------------------------------------------------- 314 |
| G.max_Glyma07g07640.1#1 | DKRNL------------------------------------------------- 315 |
| G.max_Glyma09g08250.1#1 | DKGHL------------------------------------------------- 317 |
| M.truncatula_TC117212#1 | DKTHL------------------------------------------------- 316 |
| P.trichocarpa_scaff_II.31#1 | EKECL------------------------------------------------- 321 |
| S.lycopersicum_TC205060#1 | DKTPL------------------------------------------------- 315 |
| H.vulgare_TC172640#1 | ------------------------------------------------------ |
| T.aestivum_TC282781#1 | DKALY------------------------------------------------- 326 |
| O.sativa_LOC_Os08g40170.1#1 | NKELY------------------------------------------------- 326 |
| sorghumXP_002441781.1 | DKSQF------------------------------------------------- 308 |
| Z.mays_c6219578③gm030④04③@12303 | DKSQF------------------------------------------------- 308 |
| O.sativa_LOC_Os01g67160.1#1 | DKSQF------------------------------------------------- 303 |
| H.vulgare_TC160457#1 | DKSQF------------------------------------------------- 309 |
| T.aestivum_TC283074#1 | TVQSWRSTSGGFSHMGGWLGPHFSAWEYV ------------------------- 324 |
| T.aestivum_TC290551#1 | DKSQF------------------------------------------------- 304 |
| SEQ ID NO: 318 | ------------------------------------------------------ |
| A.thaliana_AT2G38620.1#1 | ------------------------------------------------------ |
| A.thaliana_AT2G38620.2#1 | DKSQF------------------------------------------------- 311 |

**FIGURE 2 (continued)**

```
A.thaliana_AT3G54180.1#1           DKSQF----------------------- 309
ChenopodiumCAC17703.1             NAIVSY---------------------- 317
P.trichocarpa_scaff_XVI.1376#1    DKSQF----------------------- 302
P.trichocarpa_scaff_3712.1#1      DKSQF----------------------- 308
P.trichocarpa_scaff_28.331#1      DKSQF----------------------- 322
ricinusXP_002519974.1             DKSQF----------------------- 316
vitisXP_002266623.1               DKSQF----------------------- 303
S.lycopersicum_TC198540#1         DKSQF----------------------- 303
tomatoCAC15503.1                  DKSQF----------------------- 303
tobaccoAAG01532.1                 DKSQF----------------------- 303
anthirinumCAA66235.1              DKSQF----------------------- 305
M.truncatula_TC115190#1           DKSQY----------------------- 311
medicagoP93321                    DKSQY----------------------- 311
lotus                             DKSQY----------------------- 321
G.max_Glyma07g02400.1#1           DKSQF----------------------- 314
P.patens_NP13120379#1             DKSQY----------------------- 302
P.patens_TC47562#1                DKSQF----------------------- 302
P.patens_NP13127560#1             DKSQF----------------------- 302
P.patens_TC43373#1                DKSQF----------------------- 303
P.patens_TC41028#1                DKTAT----------------------- 308
P.patens_TC35664#1                Q--------------------------- 266
```

CDKB-RKA Terminator

RB

pGOS2

Plant expression vector
promoter::CDKB-RKA

Bacterial origin
of replication

Plant screenable
marker cassette

Plant selectable
marker cassette

LB

Bacterial
selectable
marker

FIGURE 3

Europäisches
Patentamt

European
Patent Office

Office européen
des brevets

# EUROPEAN SEARCH REPORT

**Application Number**

EP 16 16 8936

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/024029 A2 (CROPDESIGN NV [BE]; BROEKAERT WILLEM [BE]; FRANKARD VALERIE [BE]; HATZ) 17 March 2005 (2005-03-17) * the whole document * | 1-18 | INV. C12N15/82 C07K14/415 |
| X | HEMERLY A ET AL: "DOMINANT NEGATIVE MUTANTS OF THE CDC2 KINASE UNCOUPLE CELL DIVISIONFROM ITERATIVE PLANT DEVELOPMENT", EMBO JOURNAL, OXFORD UNIVERSITY PRESS, SURREY, GB, vol. 14, no. 16, 1 January 1995 (1995-01-01), pages 3925-3936, XP002045514, ISSN: 0261-4189 * abstract * * page 3932, column 2, paragraph 4 - page 3933, column 1, paragraph 2 * | 1-18 | |
| A | EP 1 586 645 A2 (CERES INC [US]) 19 October 2005 (2005-10-19) * the whole document * | 1-18 | |
| A | YOSHIZUMI TAKESHI ET AL: "An Arabidopsis cell cycle-dependent kinase-related gene, CDC2b, plays a role in regulating seedling growth in darkness", THE PLANT CELL, AMERICAN SOCIETY OF PLANT BIOLOGISTS, US, vol. 11, no. 10, 1 October 1999 (1999-10-01), pages 1883-1895, XP002316407, ISSN: 1040-4651, DOI: 10.1105/TPC.11.10.1883 | 1-18 | TECHNICAL FIELDS SEARCHED (IPC) C12N C07K |

The present search report has been drawn up for all claims

| Place of search | Date of completion of the search | Examiner |
|---|---|---|
| Munich | 23 August 2016 | Keller, Yves |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 16 8936

This annex lists the patent family members relating to the patent documents cited in the above-mentioned European search report.
The members are as contained in the European Patent Office EDP file on
The European Patent Office is in no way liable for these particulars which are merely given for the purpose of information.

23-08-2016

| Patent document cited in search report | | Publication date | Patent family member(s) | | Publication date |
|---|---|---|---|---|---|
| WO 2005024029 | A2 | 17-03-2005 | AT | 448314 T | 15-11-2009 |
| | | | AU | 2004270888 A1 | 17-03-2005 |
| | | | AU | 2008202839 A1 | 24-07-2008 |
| | | | BR | PI0413329 A | 10-10-2006 |
| | | | CA | 2536650 A1 | 17-03-2005 |
| | | | CN | 1845997 A | 11-10-2006 |
| | | | CN | 102174545 A | 07-09-2011 |
| | | | EP | 1664309 A2 | 07-06-2006 |
| | | | ES | 2335588 T3 | 30-03-2010 |
| | | | US | 2007199085 A1 | 23-08-2007 |
| | | | WO | 2005024029 A2 | 17-03-2005 |
| | | | ZA | 200602763 B | 25-07-2007 |
| EP 1586645 | A2 | 19-10-2005 | EP | 1586645 A2 | 19-10-2005 |
| | | | US | 2006084796 A1 | 20-04-2006 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070214517 A
- WO 2005024029 A
- US 5811238 A
- US 6395547 B
- WO 2004070039 A
- WO 2004065596 A
- US 4962028 A
- WO 0114572 A
- WO 9514098 A
- WO 9412015 A
- US 5401836 A
- US 20050044585 A
- EP 99106056 A
- US 5565350 A
- WO 0015815 A
- WO 9322443 A, Zarling
- WO 9853083 A, Grierson
- WO 9953050 A, Waterhouse

- US 4987071 A, Cech
- US 5116742 A, Cech
- WO 9400012 A, Atkins
- WO 9503404 A, Lenne
- WO 0000619 A, Lutziger
- WO 9713865 A, Prinsen
- WO 9738116 A, Scott
- WO 9836083 A
- WO 9915682 A
- EP 1198985 A1
- WO 2007093444 A
- US 5164310 A
- US 5159135 A
- WO 9623891 A
- WO 2010151634 A
- EP 1831378 A
- WO 2010031780 A
- WO 2006029987 A

**Non-patent literature cited in the description**

- **WANG et al.** *Planta,* 2003, vol. 218, 1-14
- **LA THANGUE.** *Curr. Opin. Cell Biol.,* 1994, vol. 6 (3), 443-450
- **MULLER et al.** *Genes Dev.,* 2001, vol. 15 (3), 267-285
- **DE VEYLDER et al.** *EMBO J.,* 2002, vol. 21 (6), 1360-1368
- **MIRONOV et al.** *Plant Cell,* 1999, vol. 11 (4), 509-522
- **REED.** *Progression Cell cycle Research,* 1996, vol. 2, 15-27
- **HEMERLY et al.** *EMBO J.,* 1995, vol. 14 (16), 3925-3936
- **MAGYAR et al.** *Plant Cell,* 1997, vol. 9 (2), 223-235
- **PORCEDDU et al.** *J. Biol. Chem.,* 2001, vol. 276 (39), 36354-36360
- **UMEDA et al.** *Proc Natl Acad Sci U S A.,* 1998, vol. 95 (9), 5021-5026
- **JOUBÈS et al.** *Plant Physiol.,* 2001, vol. 126 (4), 1403-1415
- **VANDEPOELE et al.** *Plant Cell,* 2002, vol. 14 (4), 903-916
- **VAN LEENE et al.** *Mol Syst Biol,* 2010, vol. 6, 397

- **FRANCIS.** *New Phytol,* 2007, vol. 174 (2), 261-78
- **DOONAN ; KITSIOS.** *Mol Biotechnol,* 2009, vol. 42 (1), 14-29
- **MAGYAR et al.** *plant cell,* 1997, vol. 9 (2), 223-235
- Current Protocols in Molecular Biology
- **CREIGHTON.** Proteins. 1984
- Current Protocols in Molecular Biology. John Wiley & Sons, 1989
- **TERPE.** *Appl. Microbiol. Biotechnol,* 2003, vol. 60, 523-533
- **SCHULTZ et al.** *Proc. Natl. Acad. Sci. USA,* 1998, vol. 95, 5857-5864
- **LETUNIC et al.** *Nucleic Acids Res,* 2002, vol. 30, 242-244
- **MULDER et al.** *Nucl. Acids. Res.,* 2003, vol. 31, 315-318
- A generalized profile syntax for biomolecular sequences motifs and its function in automatic sequence interpretation. **BUCHER ; BAIROCH.** In) IS-MB-94; Proceedings 2nd International Conference on Intelligent Systems for Molecular Biology. AAAI Press, 1994, 53-61
- **HULO et al.** *Nucl. Acids. Res.,* 2004, vol. 32, D134-D137

- **BATEMAN et al.** *Nucleic Acids Research,* 2002, vol. 30 (1), 276-280
- **GASTEIGER et al.** ExPASy: the proteomics server for in-depth protein knowledge and analysis. *Nucleic Acids Res.,* 2003, vol. 31, 3784-3788
- **NEEDLEMAN ; WUNSCH.** *J Mol Biol,* 1970, vol. 48, 443-453
- **ALTSCHUL et al.** *J Mol Biol,* 1990, vol. 215, 403-10
- **CAMPANELLA et al.** *BMC Bioinformatics,* 10 July 2003, vol. 4, 29
- **SMITH TF ; WATERMAN MS.** *J. Mol. Biol,* 1981, vol. 147 (1), 195-7
- **MEINKOTH ; WAHL.** *Anal. Biochem.,* 1984, vol. 138, 267-284
- **SAMBROOK et al.** Molecular Cloning: a laboratory manual. Cold Spring Harbor Laboratory Press, CSH, 2001
- **FOISSAC ; SCHIEX.** *BMC Bioinformatics,* 2005, vol. 6, 25
- **CASTLE et al.** *Science,* 2004, vol. 304 (5674), 1151-4
- **HEID et al.** *Genome Methods,* 1996, vol. 6, 986-994
- **MCELROY et al.** *Plant Cell,* 1990, vol. 2, 163-171
- **ODELL et al.** *Nature,* 1985, vol. 313, 810-812
- **NILSSON et al.** *Physiol. Plant.,* 1997, vol. 100, 456-462
- **DE PATER et al.** *Plant J,* November 1992, vol. 2 (6), 837-44
- **CHRISTENSEN et al.** *Plant Mol. Biol.,* 1992, vol. 18, 675-689
- **BUCHHOLZ et al.** *Plant Mol Biol.,* 1994, vol. 25 (5), 837-43
- **LEPETIT et al.** *Mol. Gen. Genet.,* 1992, vol. 231, 276-285
- **WU et al.** *Plant Mol. Biol.,* 1988, vol. 11, 641-649
- **AN et al.** *Plant J.,* 1996, vol. 10 (1), 107-121
- **SANGER et al.** *Plant. Mol. Biol.,* 1990, vol. 14, 433-443
- **LEISNER.** *Proc Natl Acad Sci USA,* 1988, vol. 85 (5), 2553
- **JAIN et al.** *Crop Science,* 1999, vol. 39 (6), 1696
- **SHAW et al.** *Nucleic Acids Res.,* 1984, vol. 12 (20), 7831-7846
- **GATZ.** *Annu. Rev. Plant Physiol. Plant Mol. Biol.,* 1997, vol. 48, 89-108
- *Plant Mol Biol.,* January 1995, vol. 27 (2), 237-48
- **KOYAMA et al.** *J Biosci Bioeng.,* January 2005, vol. 99 (1), 38-42
- **MUDGE et al.** *Plant J.,* 2002, vol. 31, 341
- **XIAO et al.** *Plant Biol (Stuttg),* July 2006, vol. 8 (4), 439-49
- **NITZ et al.** *Plant Sci,* 2001, vol. 161 (2), 337-346
- **TINGEY et al.** *EMBO J.,* 1987, vol. 6, 1
- **VAN DER ZAAL et al.** *Plant Mol. Biol.,* 1991, vol. 16, 983
- **OPPENHEIMER et al.** *Gene,* 1988, vol. 63, 87
- **CONKLING et al.** *Plant Physiol.,* 1990, vol. 93, 1203
- **SUZUKI et al.** *Plant Mol. Biol.,* 1993, vol. 21, 109-119
- **BAUMBERGER et al.** *Genes & Dev.,* 2001, vol. 15, 1128
- **LAUTER et al.** *PNAS,* 1996, vol. 3, 8139
- **LIU et al.** *Plant Mol. Biol.,* vol. 17 (6), 1139-1154
- **DOWNEY et al.** *J. Biol. Chem.,* 2000, vol. 275, 39420
- **W SONG.** PhD Thesis. North Carolina State University, 1997
- **WANG et al.** *Plant Sci.,* 2002, vol. 163, 273
- **DIENER et al.** *Plant Cell,* 2001, vol. 13, 1625
- **QUESADA et al.** *Plant Mol. Biol.,* 1997, vol. 34, 265
- **QING QU ; TAKAIWA.** *Plant Biotechnol. J.,* 2004, vol. 2, 113-125
- **SIMON et al.** *Plant Mol. Biol.,* 1985, vol. 5, 191
- **SCOFIELD et al.** *J. Biol. Chem.,* 1987, vol. 262, 12202
- **BASZCZYNSKI et al.** *Plant Mol. Biol.,* 1990, vol. 14, 633
- **PEARSON et al.** *Plant Mol. Biol.,* 1992, vol. 18, 235-245
- **ELLIS et al.** *Plant Mol. Biol.,* 1988, vol. 10, 203-214
- **TAKAIWA et al.** *Mol. Gen. Genet.,* 1986, vol. 208, 15-22
- **TAKAIWA et al.** *FEBS Letts.,* 1987, vol. 221, 43-47
- **MATZKE et al.** *Plant Mol Biol,* 1990, vol. 14 (3), 323-32
- **STALBERG et al.** *Planta,* 1996, vol. 199, 515-519
- *Mol Gen Genet,* 1989, vol. 216, 81-90
- *NAR,* 1989, vol. 17, 461-2
- **ALBANI et al.** *Plant Cell,* 1997, vol. 9, 171-184
- *EMBO J.,* 1984, vol. 3, 1409-15
- **DIAZ et al.** *Mol Gen Genet,* 1995, vol. 248 (5), 592-8
- *Theor Appl Gen,* 1999, vol. 98, 1253-62
- *Plant J,* 1993, vol. 4, 343-55
- *Mol Gen Genet,* 1996, vol. 250, 750-60
- **MENA et al.** *The Plant Journal,* 1998, vol. 116 (1), 53-62
- **VICENTE-CARBAJOSA et al.** *Plant J.,* 1998, vol. 13, 629-640
- **WU et al.** *Plant Cell Physiology,* 1998, vol. 39 (8), 885-889

- **SATO et al.** *Proc. Natl. Acad. Sci. USA,* 1996, vol. 93, 8117-8122
- **NAKASE et al.** *Plant Mol. Biol.,* 1997, vol. 33, 513-522
- *Trans Res,* 1997, vol. 6, 157-68
- *Plant J,* 1997, vol. 12, 235-46
- **DEROSE et al.** *Plant Mol. Biol,* 1996, vol. 32, 1029-35
- **POSTMA-HAARSMA et al.** *Plant Mol. Biol.,* 1999, vol. 39, 257-71
- **WU et al.** *J. Biochem.,* 1998, vol. 123, 386
- **CUMMINS et al.** *Plant Mol. Biol.,* 1992, vol. 19, 873-876
- **LANAHAN et al.** *Plant Cell,* 1992, vol. 4, 203-211
- **SKRIVER et al.** *Proc Natl Acad Sci USA,* 1991, vol. 88, 7266-7270
- **CEJUDO et al.** *Plant Mol Biol,* 1992, vol. 20, 849-856
- **KALLA et al.** *Plant J.,* 1994, vol. 6, 849-60
- **LEAH et al.** *Plant J.,* 1994, vol. 4, 579-89
- **SELINGER et al.** *Genetics,* 1998, vol. 149, 1125-38
- **TAKAIWA et al.** *Mol Gen Genet,* 1986, vol. 208, 15-22
- **COLOT et al.** *Mol Gen Genet,* 1989, vol. 216, 81-90
- **ANDERSON et al.** *NAR,* 1989, vol. 17, 461-2
- **RAFALSKI et al.** *EMBO,* 1984, vol. 3, 1409-15
- **CHO et al.** *Theor Appl Genet,* 1999, vol. 98, 1253-62
- **MULLER et al.** *Plant J,* 1993, vol. 4, 343-55
- **SORENSON et al.** *Mol Gen Genet,* 1996, vol. 250, 750-60
- **MENA et al.** *Plant J,* 1998, vol. 116 (1), 53-62
- **ONATE et al.** *J Biol Chem,* 1999, vol. 274 (14), 9175-82
- **VICENTE-CARBAJOSA et al.** *Plant J,* 1998, vol. 13, 629-640
- **WU et al.** *Plant Cell Physiol,* 1998, vol. 39 (8), 885-889
- **NAKASE et al.** *Plant Molec Biol,* 1997, vol. 33, 513-522
- **RUSSELL et al.** *Trans Res,* 1997, vol. 6, 157-68
- **OPSAHL-FERSTAD et al.** *Plant J,* 1997, vol. 12, 235-46
- **DEROSE et al.** *Plant Mol Biol,* 1996, vol. 32, 1029-35
- **FUKAVAMA et al.** *Plant Physiol.,* November 2001, vol. 127 (3), 1136-46
- **KAUSCH et al.** *Plant Mol Biol.,* January 2001, vol. 45 (1), 1-15
- **LIN et al.** *DNA,* August 2004, vol. 15 (4), 269-76
- **NOMURA et al.** *Plant Mol Biol.,* September 2000, vol. 44 (1), 99-106
- **PANGULURI et al.** *Indian J Exp Biol.,* April 2005, vol. 43 (4), 369-72
- **WAGNER ; KOHORN.** *Plant Cell,* 2001, vol. 13 (2), 303-318
- **TRIBBLE et al.** *J. Biol. Chem.,* 2000, vol. 275, 22255-22267
- **VELMURUGAN et al.** *J. Cell Biol.,* 2000, vol. 149, 553-566
- **BUCHMAN ; BERG.** *Mol. Cell biol.,* 1988, vol. 8, 4395-4405
- **CALLIS et al.** *Genes Dev,* 1987, vol. 1, 1183-1200
- The Maize Handbook. Springer, 1994
- **GAULTIER et al.** *Nucl Ac Res,* 1987, vol. 15, 6625-6641
- **INOUE et al.** *Nucl Ac Res,* 1987, vol. 15, 6131-6148
- **INOUE et al.** *FEBS Lett.,* 1987, vol. 215, 327-330
- **HASELHOFF ; GERLACH.** *Nature,* 1988, vol. 334, 585-591
- **BARTEL ; SZOSTAK.** *Science,* 1993, vol. 261, 1411-1418
- **ANGELL ; BAULCOMBE.** *Plant J,* 1999, vol. 20 (3), 357-62
- **HELENE, C.** *Anticancer Drug Res.,* 1991, vol. 6, 569-84
- **HELENE et al.** *Ann. N.Y. Acad. Sci.,* 1992, vol. 660, 27-36
- **MAHER, L.J.** *Bioassays,* 1992, vol. 14, 807-15
- **SCHWAB et al.** *Dev. Cell,* 2005, vol. 8, 517-527
- **SCHWAB et al.** *Plant Cell,* 2006, vol. 18, 1121-1133
- **KRENS, F.A. et al.** *Nature,* 1982, vol. 296, 72-74
- **NEGRUTIU I et al.** *Plant Mol Biol,* 1987, vol. 8, 363-373
- **SHILLITO R.D. et al.** *Bio/Technol,* 1985, vol. 3, 1099-1102
- **CROSSWAY A et al.** *Mol. Gen Genet,* 1986, vol. 202, 179-185
- **KLEIN TM et al.** *Nature,* 1987, vol. 327, 70
- **CLOUGH ; BENT.** *Plant J.,* 1998, vol. 16, 735-743
- **ALDEMITA ; HODGES.** *Planta,* 1996, vol. 199, 612-617
- **CHAN et al.** *Plant Mol Biol,* 1993, vol. 22 (3), 491-506
- **HIEI et al.** *Plant J,* 1994, vol. 6 (2), 271-282
- **ISHIDA et al.** *Nat. Biotechnol,* 1996, vol. 14 (6), 745-50
- **FRAME et al.** *Plant Physiol,* 2002, vol. 129 (1), 13-22
- Techniques for Gene Transfer, in: Transgenic Plants. **B. JENES et al.** Engineering and Utilization. Academic Press, 1993, vol. 1, 128-143

- Potrykus Annu. Rev. Plant Physiol. *Plant Molec. Biol.,* 1991, vol. 42, 205-225
- **BEVAN et al.** *Nucl. Acids Res.,* 1984, vol. 12, 8711
- **HÖFGEN ; WILLMITZER.** *Nucl. Acid Res.,* 1988, vol. 16, 9877
- Vectors for Gene Transfer in Higher Plants; in Transgenic Plants. **F.F. WHITE.** Engineering and Utilization. Academic Press, 1993, vol. 1, 15-38
- **FELDMAN, KA ; MARKS MD.** *Mol Gen Genet,* 1987, vol. 208, 1-9
- Methods in Arabidopsis Research. **FELDMANN K.** Word Scientific. 1992, 274-289
- **CHANG.** *Plant J.,* 1994, vol. 5, 551-558
- **KATAVIC.** *Mol Gen Genet,* 1994, vol. 245, 363-370
- **BECHTHOLD, N.** *C R Acad Sci Paris Life Sci,* 1993, vol. 316, 1194-1199
- **CLOUGH, SJ ; BENT AF.** *The Plant J.,* 1998, vol. 16, 735-743
- **KLAUS et al.** *Nature Biotechnology,* 2004, vol. 22 (2), 225-229
- **BOCK.** Transgenic plastids in basic research and plant biotechnology. *J Mol Biol.,* 21 September 2001, vol. 312 (3), 425-38
- **MALIGA, P.** Progress towards commercialization of plastid transformation technology. *Trends Biotechnol.,* 2003, vol. 21, 20-28
- **HAYASHI et al.** *Science,* 1992, 1350-1353
- **REDEI GP ; KONCZ C.** In Methods in Arabidopsis Research. World Scientific Publishing Co, 1992, 16-82
- **FELDMANN et al.** Arabidopsis. Cold Spring Harbor Laboratory Press, 1994, 137-172
- **LIGHTNER J ; CASPAR T.** Methods on Molecular Biology. Humana Press, 1998, vol. 82, 91-104
- **MCCALLUM et al.** *Nat Biotechnol,* 2000, vol. 18, 455-457
- **STEMPLE.** *Nat Rev Genet,* 2004, vol. 5 (2), 145-50
- **OFFRINGA et al.** *EMBO J,* 1990, vol. 9 (10), 3077-84
- **TERADA et al.** *Nat Biotech,* 2002, vol. 20 (10), 1030-4
- **LIDA ; TERADA.** *Curr Opin Biotech,* 2004, vol. 15 (2), 132-8
- **MILLER et al.** *Nature Biotechnol.,* 2007, vol. 25, 778-785
- **RABBANI et al.** *Plant Physiol,* 2003, vol. 133, 1755-1767
- **SAMBROOK J ; FRITSCH EF ; MANIATIS T.** Molecular Cloning, A Laboratory Manual. 1989
- **LANDER et al.** *Genomics,* 1987, vol. 1, 174-181
- **BOTSTEIN et al.** *Am. J. Hum. Genet.,* 1980, vol. 32, 314-331
- **BERNATZKY ; TANKSLEY.** *Plant Mol. Biol. Reporter,* 1986, vol. 4, 37-41
- **HOHEISEL et al.** In: Non-mammalian Genomic Analysis: A Practical Guide. Academic press, 1996, 319-346
- **TRASK.** *Trends Genet.,* 1991, vol. 7, 149-154
- **LAAN et al.** *Genome Res.,* 1995, vol. 5, 13-20
- **KAZAZIAN.** *J. Lab. Clin. Med,* 1989, vol. 11, 95-96
- **SHEFFIELD et al.** *Genomics,* 1993, vol. 16, 325-332
- **LANDEGREN et al.** *Science,* 1988, vol. 241, 1077-1080
- **SOKOLOV.** *Nucleic Acid Res.,* 1990, vol. 18, 3671
- **WALTER et al.** *Nat. Genet.,* 1997, vol. 7, 22-28
- **DEAR ; COOK.** *Nucleic Acid Res.,* 1989, vol. 17, 6795-6807
- **SAMBROOK.** Molecular Cloning: a laboratory manual. Cold Spring Harbor Laboratory Press, 2001
- Current Protocols in Molecular Biology. **AUSUBEL et al.** Current Protocols. 1994, vol. 1 and 2
- **R.D.D. CROY.** Plant Molecular Biology Labfax. BIOS Scientific Publications Ltd (UK) and Blackwell Scientific Publications (UK), 1993
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **THOMPSON et al.** *Nucleic Acids Res,* 1997, vol. 25, 4876-4882
- **CHENNA et al.** *Nucleic Acids Res,* 2003, vol. 31, 3497-3500
- *BMC Bioinformatics,* 2003, vol. 4, 29
- **PARK ; KANEHISA.** *Bioinformatics,* 2003, vol. 19, 1656-1663
- **COCKCROFT et al.** *Nature,* 2000, vol. 405 (6786), 575-579
- **SETIADY et al.** *Plant Cell Physiology,* 1996, vol. 37 (3), 369-376
- **ISHIDA et al.** *Nature Biotech,* 1996, vol. 14 (6), 745-50
- **BABIC et al.** *Plant Cell Rep,* 1998, vol. 17, 183-188
- **MCKERSIE et al.** *Plant Physiol,* 1999, vol. 119, 839-847
- **BROWN DCW ; A ATANASSOV.** *Plant Cell Tissue Organ Culture,* 1985, vol. 4, 111-112
- **WALKER et al.** *Am J Bot,* 1978, vol. 65, 654-659
- **GAMBORG et al.** *Exp. Cell Res.,* 1968, vol. 50, 151-158
- **MURASHIGE, T. ; SKOOG.** *Physiol. Plant,* 1962, vol. 15, 473-497
- **GAMBORG et al.** *Exp. Cell Res.,* vol. 50, 151-8
- **HUSSEY, G. ; HEPHER, A.** *Annals of Botany,* 1978, vol. 42, 477-9

- **LINSEY, K. ; GALLOIS, P.** *Journal of Experimental Botany,* 1990, vol. 41 (226), 529-36
- **ARENCIBIA et al.** *Transgenic Research,* 1998, vol. 7, 213-22
- **ENRIQUEZ-OBREGON et al.** *Planta,* 1998, vol. 206, 20-27
- **GAMBORG, O. et al.** *Exp. Cell Res.,* 1968, vol. 50, 151-8